# EUROPEAN PATENT APPLICATION

(11) **EP 3 828 267 A2**
(43) Date of publication of application: **02.06.2021**
(21) Application number: 20195244.7
(22) Date of filing: 11.08.2015
(51) Int. Cl.: C12N 5/22, C12N 5/0783, A61K 39/00, C07K 14/725, A61P 35/00, A61P 17/00, A61P 1/00

(54) **CAR-T LYMPHOCYTES ENGINEERED TO HOME TO LYMPH NODE B CELL ZONE, SKIN, OR GASTROINTESTINAL TRACT**

(30) Priority: 12.08.2014 US 201462036447 P
(62) Divisional of application: 15831772.7
(71) Applicant: Celgene Corporation, Summit, NJ 07901 (US)
(72) Inventor: LIANG, Bitao, Closter, New Jersey 07624 (US); LIU, Wei, Bridgewater, New Jersey 08807 (US); LI, Tianjian, Belle Mead, New Jersey 08502 (US)
(74) Representative: Jones Day

(57) **Abstract**

Provided herein is a T lymphocyte comprising: (i) a skin homing receptor, a gastrointestinal homing receptor or a B cell zone homing receptor; and (ii) a chimeric antigen receptor (CAR). Also provided herein is such a T lymphocyte for use a method of treating a skin or a gastrointestinal tumor or cancer. Also provided herein is a method of generating a population of T lymphocytes, wherein a plurality of said T lymphocytes home to skin tissue or skin cells or gastrointestinal tract; and wherein said method comprises engineering a population of T lymphocytes to express a skin or a gastrointestinal homing receptor, respectively.

## Description

This application claims benefit of U.S. Provisional Patent Application No. 62/036,447, filed August 12, 2014, the disclosure of which is incorporated by reference herein in its entirety.

### 1. FIELD

The disclosure herein relates to the field of immunology, and more specifically, to the modification of T lymphocytes or other immune cells.

### 2. BACKGROUND

T lymphocytes recognize and interact with specific antigens, including tumor-associated or tumor-specific antigens. Because T lymphocytes are able to kill tumor cells, the last 25 years has seen a great deal of interest in targeting tumor cells with T lymphocytes, either antigen-specific T lymphocytes, or T lymphocytes genetically modified to express one or more chimeric antigen receptors (CARs; *see, e.g.,* Eshhar, U.S. Patent No. 7,741,465; Eshhar, U.S. Patent Application Publication No. 2012/0093842). However, given the difficulty of targeting CAR T cells to the necessary areas of the body, it is desirable to find new methods to enhance the homing of CAR T cells.

### 3. SUMMARY

Provided herein are genetically modified cells, for example immune cells, such as T lymphocytes, e.g., human T lymphocytes, that comprise a receptor that causes a cell expressing said receptor to home to a particular anatomical zone, a particular tissue, or a particular type of cell, e.g., the B cell zone of the lymph nodes, gastrointestinal tract, or skin. In a specific embodiment, the genetically modified cells provided herein are T lymphocytes, for example, primary T lymphocytes. In certain embodiments, the genetically modified cells express a chimeric antigen receptor (CAR). Without wishing to be bound by any particular mechanism or theory, it is thought that when the genetically modified cells herein express receptors that cause a cell expressing said receptors to home to a particular zone, they are more likely to be exposed to native antigen, where the cells, for example, cells expressing a CAR, are capable of being activated.

In a first aspect, provided herein is a cell, e.g., a T lymphocyte, for example, a primary T lymphocyte, or a population of such cells, that comprises, e.g., has been engineered to express, a homing receptor and a CAR. In certain embodiments, the homing receptor is not normally or endogenously expressed in the T lymphocyte. In certain embodiments, the homing receptor is a B cell zone homing receptor. In a specific embodiment, the B cell zone homing receptor is CXCR5. In a more specific embodiment, the cell, or population of cells comprising the homing receptor, e.g., the B cell zone homing receptor, additionally lacks expression of a T cell zone homing receptor, e.g., CXCR7, or has reduced expression of said T cell zone homing receptor as compared to normal T cells, e.g., the cell, or population of cells, is engineered to express a T cell homing receptor at a lower level than normal T cells.

In certain embodiments, the homing receptor is a gastrointestinal homing receptor. In a specific embodiment, the gastrointestinal homing receptor is integrin α4β7 (also known as lymphocyte Peyer patch adhesion molecule). In other specific embodiments, the gastrointestinal homing receptor is CCR9 (C-C chemokines receptor type 9). In certain embodiments, the cell, e.g., a T lymphocyte, for example, a primary T lymphocyte, or population of such cells, comprises, e.g., has been engineered to express more than one, e.g., a second, gastrointestinal homing receptor, e.g., the primary T lymphocyte comprises CCR9 and integrin α4β7.

In one embodiment, the cell, e.g., the T lymphocyte, or a population of such cells, that comprises, e.g., has been engineered to express, a gastrointestinal homing receptor has been activated, expanded, or both activated and expanded in the presence of a Vitamin A metabolite. In specific embodiments, the Vitamin A metabolite is retinoic acid.

In certain embodiments, the homing receptor is a skin homing receptor. In a specific embodiment, the skin homing receptor is CLA (cutaneous lymphocyte-associated antigen receptor). In other specific embodiments, the skin homing receptor is CCR4, CCR8, or CCR10. In certain embodiments, the cell, e.g., a T lymphocyte, for example, a primary T lymphocyte, or population of such cells comprises, e.g., has been engineered to express, second skin homing receptor. In yet other embodiments, the cell, e.g., a T lymphocyte, for example, a primary T lymphocyte, or population of such cells, comprises, e.g., expresses, a third skin homing receptor. In yet other embodiments, the cell, e.g., a T lymphocyte, for example, a primary T lymphocyte, or population of such cells, comprises, e.g., expresses, a fourth skin homing receptor. In other embodiments, the cell or population of cells comprises, e.g., expresses, two or more skin homing receptors, e.g., two or more of CLA, CCR4, CCR8, and/or CCR10.

In one embodiment, the cell, e.g., the T lymphocyte, for example, the primary T lymphocyte, or population of such cells comprising, e.g., expressing, a skin-homing receptor has been activated, expanded, or both activated and expanded in the presence of a Vitamin D metabolite. In specific embodiments, the Vitamin D metabolite is 1,26-dihydroxycholecalciferol (1,25(OH)₂D₃). In some embodiments, the cell, e.g., the T lymphocyte, for example, the primary T lymphocyte, or population of such cells has been activated, expanded, or both activated and expanded in the presence of IL-12. In specific embodiments, the cell, e.g., the T lymphocyte, for example, the primary T lymphocyte, or population of such cells, have been activated, expanded, or activated and expanded in the presence of both IL-12 and a vitamin D metabolite.

In some embodiments, the cell, e.g., the T lymphocyte, for example, the primary T lymphocyte, or population of such cells, comprises, e.g., has been engineered to express, either a gastrointestinal homing receptor or a skin homing receptor, and additionally comprises a B cell homing receptor. In specific embodiments, the B cell homing receptor is CXCR5.

In another aspect, provided herein are methods of generating a cell, e.g., a T lymphocyte, for example, a primary T lymphocyte, or population of such cells, which expresses a homing receptor. In certain embodiments, provided herein is a method of generating a cell, e.g., a T lymphocyte, for example, a primary T lymphocyte, or population of such cells, that homes to the B cell zone of a lymph node, comprising engineering the cell or population of cells to express a B cell zone homing receptor in an amount or at a level sufficient to cause the cell or population of cells to home to the B cell zone of the lymph node. In a specific embodiment, the B cell zone homing receptor is CXCR5.

In certain embodiments, provided herein is a method of generating a cell, e.g., a T lymphocyte, for example, a primary T lymphocyte, or population of such cells, that homes to the gastrointestinal tract, comprising engineering the cell or population of cells to express a gastrointestinal homing receptor in an amount or at a level sufficient to cause the cell or population of cells to home to the gastrointestinal tract. In a specific embodiment, the gastrointestinal homing receptor is integrin α4β7. In other specific embodiments, the gastrointestinal homing receptor is CCR9. In certain embodiments, provided herein is a method of generating a cell, e.g., a T lymphocyte, for example, a primary T lymphocyte, or population of such cells, comprising engineering the cell or population of cells to express an additional gastrointestinal homing receptor, e.g., additionally express a second gastrointestinal homing receptor. In certain embodiments, the methods provided herein additionally comprise a step wherein the cell or population of cells is activated, expanded, or both activated and expanded in the presence of a Vitamin A metabolite. In specific embodiments, the Vitamin A metabolite is retinoic acid.

In certain embodiments, provided herein is a method of generating a cell, e.g., a T lymphocyte, for example, a primary T lymphocyte, or population of such cells, that homes to the skin, comprising engineering the cell or population of cells to express a skin homing receptor in an amount or at a level sufficient to cause the cell or population of cells to home to the skin. In a specific embodiment, the skin homing receptor is CLA. In other specific embodiments, the skin homing receptor is CCR4, CCR8, or CCR10. In certain embodiments, provided herein is a method of generating a cell, e.g., a T lymphocyte, for example, a primary T lymphocyte, or population of such cells, comprising engineering the cell or population of cells to express an additional skin homing receptor, e.g., additionally express a second skin homing receptor. In certain embodiments, provided herein is a method of generating a cell, e.g., a T lymphocyte, for example, a primary T lymphocyte, or population of such cells, comprising engineering the cell or population of cells to additionally express a third skin homing receptor. In certain embodiments, provided herein is a method of generating a cell, e.g., a T lymphocyte, for example, a primary T lymphocyte, or a population of such cells, comprising engineering the cell or population of cells to additionally express a fourth skin homing receptor. In certain embodiments, the methods provided herein additionally comprise a step wherein the cell or population of cells is activated, expanded, or both activated and expanded in the presence of a Vitamin D metabolite. In specific embodiments, the Vitamin D metabolite is 1,26-dihydroxycholecalciferol (1,25(OH)₂D₃). In certain embodiments, the methods provided herein additionally comprise a step wherein the cell or population of cells is activated, expanded, or both activated and expanded in the presence of IL-12.

In certain embodiments, provided herein is a method of generating a cell, e.g., a T lymphocyte, for example, a primary T lymphocyte, or a population of such cells, that is engineered to express a gastrointestinal homing receptor or a skin homing receptor, and additionally is engineered to express a B cell zone homing receptor. In specific embodiments, provided herein is a method of generating a cell, e.g., a T lymphocyte, for example, a primary T lymphocyte, or a population of such cells, that is engineered to express a gastrointestinal homing receptor or a skin homing receptor, and additionally is engineered to express CXCR5.

In certain embodiments, provided herein is a method of generating a cell, e.g., a T lymphocyte, for example, a primary T lymphocyte, or a population of such cells, that is engineered to express a gastrointestinal homing receptor or a skin homing receptor, which method comprises a step of administering to the T lymphocyte a lentiviral vector encoding a chimeric antigen receptor (CAR).

In another aspect, provided herein are methods of treating a cancer or tumor in an individual, comprising administering to an individual in need thereof a T lymphocyte, or a population of T lymphocytes, comprising (i) a B cell zone homing receptor; and (ii) a CAR. In certain embodiments, the B cell zone homing receptor is CXCR5.

In certain embodiments, provided herein are methods of treating a gastrointestinal cancer or tumor in an individual, comprising administering to an individual in need thereof a T lymphocyte, or a population of T lymphocytes, comprising, e.g., expressing, (i) a gastrointestinal homing receptor; and (ii) a CAR. In specific embodiments, the gastrointestinal homing receptor is integrin α4β7. In other specific embodiments, the gastrointestinal homing receptor is CCR9. In certain embodiments, provided herein are methods of treating a gastrointestinal cancer or tumor in an individual, comprising administering to an individual in need thereof a T lymphocyte, or a population of Ty lymphocytes, additionally comprising, e.g., expressing, a second gastrointestinal homing receptor.

In certain embodiments, provided herein are methods of treating a skin cancer or tumor in an individual, comprising administering to an individual in need thereof a T lymphocyte, or a population of T lymphocytes, comprising (i) a skin homing receptor; and (ii) a CAR. In specific embodiments, the skin homing receptor is CLA. In other specific embodiments, the skin homing receptor is CCR4, CCR8, or CCR10. In certain embodiments, provided herein are methods of treating a skin cancer or tumor in an individual, comprising administering to an individual in need thereof a T lymphocyte, or a population of T lymphocytes, additionally comprising a second skin homing receptor. In certain embodiments, provided herein are methods of treating a skin cancer or tumor in an individual, comprising administering to an individual in need thereof a T lymphocyte, or a population of T lymphocytes, additionally comprising a third skin homing receptor. In certain embodiments, provided herein are methods of treating a skin cancer or tumor in an individual, comprising administering to an individual in need thereof a T lymphocyte, or a population of T lymphocytes, additionally comprising a fourth skin homing receptor.

In certain embodiments, provided herein are methods of treating a gastrointestinal cancer or tumor in an individual, comprising administering to an individual in need thereof a T lymphocyte, or a population of T lymphocytes, comprising (i) a gastrointestinal homing receptor; and (ii) a CAR, wherein the T lymphocyte, or a population of T lymphocytes, further comprises a B cell zone homing receptor. In specific embodiments, the B cell zone homing receptor is CXCR5. In certain embodiments, the T lymphocytes provided herein are for use in methods of treating a gastrointestinal cancer or tumor.

In certain embodiments, provided herein are methods of treating a skin cancer or tumor in an individual, comprising administering to an individual in need thereof a T lymphocyte, or a population of T lymphocytes, comprising (i) a skin homing receptor; and (ii) a CAR, wherein the T lymphocyte, or a population of T lymphocytes, further comprises a B cell zone homing receptor. In specific embodiments, the B cell zone homing receptor is CXCR5. In certain embodiments, the T lymphocytes provided herein are for use in methods of treating a skin cancer or tumor.

In specific embodiments, provided herein are methods of treating a cancer or tumor in an individual, comprising administering to an individual in need thereof a T lymphocyte, or a population of T lymphocytes, comprising (i) a homing receptor; and (ii) a CAR, wherein the extracellular domain of the CAR binds an antigen selected from the group consisting of Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD19, CD34, CD45, CD99, CD117, chromogranin, cytokeratin, desmin, glial fibrillary acidic protein (GFAP), gross cystic disease fluid protein (GCDFP-15), HMB-45 antigen, high molecular weight melanoma-associated antigen (HMW-MAA), protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, an abnormal p53 protein, fuc-GM1, GM2 (oncofetal antigen-immunogenic-1; OFA-I-1); GD2 (OFA-I-2), GM3, GD3, alpha-actinin-4, Bage-1, BCR-ABL, Bcr-Abl fusion protein, beta-catenin, CA 15-3 (CA 27.29\BCAA), CA 195, CA 242, CA-50, CAM43, Casp-8, cdc27, cdk4, cdkn2a, coa-1, dek-can fusion protein, EBNA, EF2, Epstein Barr virus antigens, ETV6-AML1 fusion protein, HLA-A2, HLA-A11, hsp70-2, KIAAO205, Mart2, Mum-1, 2, and 3, neo-PAP, myosin class I, OS-9, pml-RARa fusion protein, PTPRK, triosephosphate isomerase, Gage 3,4,5,6,7, GnTV, Herv-K-mel, Lage-1, NA-88, NY-Eso-1/Lage-2, SP17, SSX-2, TRP2-Int2, , gp100 (Pmel 17), tyrosinase, TRP-1, TRP-2, MAGE-1, MAGE-3, RAGE, GAGE-1, GAGE-2, p15(58), RAGE,, SCP-1, Hom/Mel-40, PRAME, HER-2/neu, E2A-PRL, H4-RET, IGH-IGK, MYL-RAR, human papillomavirus (HPV) antigens E6 and E7, TSP-180, p185erbB2, p180erbB-3, c-met, nm-23H1, PSA, TAG-72-4, CA 19-9, CA 72-4, CAM 17.1, NuMa, 13-Catenin, Mum-1, p16, TAGE, PSMA, CT7, telomerase, 43-9F, 5T4, 791Tgp72, 13HCG, BCA225, BTAA, , CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90, TAAL6, TAG72, TLP, TPS, CD19, CD22, CD27, CD30, CD70, EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), integrin αvβ3 (CD61), galactin, and Ral-B. In specific embodiments, provided herein are methods of treating a gastrointestinal cancer or tumor in an individual, comprising administering to an individual in need thereof a T lymphocyte, or a population of T lymphocytes, comprising (i) a gastrointestinal homing receptor; and (ii) a CAR, wherein the extracellular domain of the CAR binds an antigen associated with, e.g., expressed by, a gastrointestinal tumor or cancer. In specific embodiments, the antigen associated with, e.g., expressed by, a gastrointestinal tumor or cancer is CEA, Her2, CA242, MUC1, CA125, or CA19-9. In specific embodiments, the T lymphocytes provided herein are for use in methods of treating a cancer or tumor.

In other specific embodiments, provided herein are methods of treating a skin cancer or tumor in an individual, comprising administering to an individual in need thereof a T lymphocyte, or a population of T lymphocytes, comprising (i) a skin homing receptor; and (ii) a CAR, wherein the extracellular domain of the CAR binds an antigen associated with a skin tumor or cancer. In specific embodiments, the antigen associated with, e.g., expressed by, a skin tumor or cancer is HMW-MAA, Her2, GD2, GD3, CEA, or SPAG9. In other specific embodiments, the T lymphocytes provided herein are for use in methods of treating a skin cancer or tumor.

### 4. DETAILED DESCRIPTION

Adaptive immune responses are initiated in secondary lymphoid organs, including the lymph nodes. B cells and T cells are sequestered in distinct regions of the lymph nodes, termed the "B cell zone," located in the outer cortex of the lymph node, or follicles, and the "T cell zone," which is more diffusely distributed in the area surrounding the follicles (also known as the paracortex) respectively. B cells and T cells express receptors that allow them to home to these respective zones so that they can be exposed to antigen. Intact antigens are present in the B cell zone, whereas in the T cell zone, antigens are presented by antigen-presenting cells, such as dendritic cells. Intact antigens, such as tumor antigens, are also present at the site of the tumor.

Provided herein are genetically modified cells, for example immune cells, such as T lymphocytes, e.g., human T lymphocytes, that comprise a receptor that causes a cell expressing said receptor to home to a particular anatomical zone, a particular tissue, or a particular type of cell, e.g., B cell zone of the lymph nodes, gastrointestinal tract, or skin. In a specific embodiment, the genetically modified cells provided herein are T lymphocytes. In certain embodiments, the genetically modified cells express a chimeric antigen receptor (CAR), as described in Section 4.4. In certain embodiments, the genetically modified cells express a CAR and endogenously express one or more homing receptors, e.g., receptors that that cause a cell expressing the receptors to home to the B cell zone of the lymph nodes, gastrointestinal tract, or skin. Without wishing to be bound by any particular mechanism or theory, it is thought that when the genetically modified cells herein express receptors that cause a cell expressing said receptors to home to a particular zone, they are more likely to be exposed to native antigen, where the cells, for example, cells expressing a CAR, are capable of being activated.

### 4.1 Cells Comprising B-cell Zone Homing Receptors, Methods of Making, and Uses Thereof

In one embodiment, provided herein are genetically modified cells, for example immune cells, such as T lymphocytes, e.g., human T lymphocytes, that comprise, e.g., have been engineered to express, a receptor that causes the cells to home to the B cell zone of the lymph nodes, e.g., the follicles of the lymph node. Such a receptor is referred to herein as a "B cell zone homing receptor." In specific embodiments, the B cell zone homing receptor is CXCR5, for example, human CXCR5. GenBank™ accession numbers NM_001716.4 and NM_032966.2 provide exemplary nucleotide sequences for human CXCR5. GenBank™ accession numbers NP_116743.1 and NP_001707.1 provide exemplary amino acid sequences for human CXCR5. Exemplary nucleotide and amino acid sequences for human homing receptors can be found in Table 1. In a specific embodiment, the genetically modified cells provided herein are T lymphocytes. In certain embodiments, the genetically modified cells express a B cell zone homing receptor and also express a CAR, as described in Section 4.4. In certain embodiments, the genetically modified cells express a CAR and endogenously express one or more B cell zone homing receptors.

Also provided herein is a method of generating genetically engineered T lymphocytes that home to the B cell zone of a lymph node, e.g., the follicles of a lymph node, comprising a step of engineering a T lymphocyte to express a B cell zone homing receptor, e.g., CXCR5, wherein said B cell zone homing receptor is expressed by the cell at a sufficient level or sufficient amount to cause the cell to home to the B cell zone of the lymph node. In some embodiments, the step of engineering a T cell to express a B cell zone homing receptor comprises a step of introducing to the cells one or more vectors comprising the receptor nucleic acid sequence(s), i.e., the nucleic acid sequence (s) encoding the receptor(s). In specific embodiments, the vector comprises the nucleic acid sequence for human CXCR5. In a certain embodiment, the step of engineering a T cell to express a B cell zone homing receptor is performed by any method known to one of skill in the art.

Exemplary nucleic acids useful for engineering a T lymphocyte to express a B cell zone homing receptor are disclosed, for example, in Section 4.5.

Also provided herein are methods of treating a cancer or tumor in an individual comprising administering to the individual a therapeutically effective amount of genetically modified cells, e.g. human T lymphocytes, that comprise (i) a receptor that causes a cell expressing said receptor to home to a B cell zone of the lymph nodes, e.g., the follicles of the lymph node, and (ii) a CAR. In specific embodiments, the B cell zone homing receptor is CXCR5. In certain embodiments, the genetically modified cells, e.g. human T lymphocytes, that comprise (i) a receptor that causes a cell expressing said receptor to home to a B cell zone of the lymph nodes, e.g., the follicles of the lymph node, and (ii) a CAR, suppress the proliferation of tumor cells. In certain embodiments, the genetically modified cells, e.g. human T lymphocytes, that comprise (i) a receptor that causes a cell expressing said receptor to home to a B cell zone of the lymph nodes, e.g., the follicles of the lymph node, and (ii) a CAR, inhibit growth of the tumor. In certain embodiments, the genetically modified cells, e.g. human T lymphocytes, that comprise (i) a receptor that causes a cell expressing said receptor to home to a B cell zone of the lymph nodes, e.g., the follicles of the lymph node, and (ii) a CAR, kill tumor cells. In specific embodiments, the extracellular domain of the CAR binds an antigen binds an antigen selected from the group consisting of Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD19, CD34, CD45, CD99, CD117, chromogranin, cytokeratin, desmin, glial fibrillary acidic protein (GFAP), gross cystic disease fluid protein (GCDFP-15), HMB-45 antigen, high molecular weight melanoma-associated antigen (HMW-MAA), protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, an abnormal p53 protein, fuc-GM1, GM2 (oncofetal antigen-immunogenic-1; OFA-I-1); GD2 (OFA-I-2), GM3, GD3, alpha-actinin-4, Bage-1, BCR-ABL, Bcr-Abl fusion protein, beta-catenin, CA 15-3 (CA 27.29\BCAA), CA 195, CA 242, CA-50, CAM43, Casp-8, cdc27, cdk4, cdkn2a, coa-1, dek-can fusion protein, EBNA, EF2, Epstein Barr virus antigens, ETV6-AML1 fusion protein, HLA-A2, HLA-A11, hsp70-2, KIAAO205, Mart2, Mum-1, 2, and 3, neo-PAP, myosin class I, OS-9, pml-RARα fusion protein, PTPRK, triosephosphate isomerase, Gage 3,4,5,6,7, GnTV, Herv-K-mel, Lage-1, NA-88, NY-Eso-1/Lage-2, SP17, SSX-2, TRP2-Int2, , gp100 (Pmel 17), tyrosinase, TRP-1, TRP-2, MAGE-1, MAGE-3, RAGE, GAGE-1, GAGE-2, p15(58), RAGE,, SCP-1, Hom/Mel-40, PRAME, HER-2/neu, E2A-PRL, H4-RET, IGH-IGK, MYL-RAR, human papillomavirus (HPV) antigens E6 and E7, TSP-180, p185erbB2, p180erbB-3, c-met, nm-23H1, PSA, TAG-72-4, CA 19-9, CA 72-4, CAM 17.1, NuMa, 13-Catenin, Mum-1, p16, TAGE, PSMA, CT7, telomerase, 43-9F, 5T4, 791Tgp72, 13HCG, BCA225, BTAA, , CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90, TAAL6, TAG72, TLP, TPS, CD19, CD22, CD27, CD30, CD70, EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), integrin αvβ3 (CD61), galactin, and Ral-B. Also provided herein are genetically modified cells for use in methods of treating a cancer or tumor.

### 4.2. Cells Comprising Gastrointestinal Homing Receptors, Methods of Making, and Uses Thereof

In one embodiment, provided herein are genetically modified cells, for example immune cells, such as T lymphocytes, e.g., human T lymphocytes, that comprise, e.g., have been engineered to express, a receptor that causes a cell expressing said receptor to home to the gastrointestinal tract, e.g., gastrointestinal organs, tissues, or cells. Such a receptor that causes a cell to home to the gastrointestinal tract is referred to herein as a "gastrointestinal homing receptor." In a specific embodiment, the genetically modified cells provided herein are T lymphocytes. In certain embodiments, the gastrointestinal homing receptor is CCR9 or integrin α4β7, for example, human CCR9 or human integrin α4β7. GenBank™ accession numbers NM_031200.2 and NM001256369.1 provide exemplary nucleotide sequences for human CCR9. GenBank™ accession numbers NP_112477.1 and NP_001243298.1 provide exemplary amino acid sequences for human CCR9. GenBank™ accession numbers NM_000885.4 and NM_000889.2 provide exemplary nucleotide sequences for human α4 and human β7, respectively. GenBank™ accession numbers NP_000876.3 and NP_000880.1 provide exemplary amino acid sequences for human α4 and human β7, respectively. Exemplary nucleotide and amino acid sequences for human homing receptors can be found in Table 1. In some embodiments, the genetically modified cells further comprise a second gastrointestinal homing receptor. In some embodiments, the genetically modified cells comprise a first gastrointestinal homing receptor, wherein the first gastrointestinal homing receptor is CCR9, and further comprise a second gastrointestinal homing receptor, wherein the second gastrointestinal homing receptor is integrin α4β7. In other specific embodiments, the genetically modified cells comprise the gastrointestinal-homing receptor CXCR3.

In certain embodiments, the genetically modified cells containing one or more gastrointestinal homing receptors are expanded, activated, or both expanded and activated in the presence of a Vitamin A metabolite. In specific embodiments, the expansion, activation, or both expansion and activation occurs *in vivo, in vitro,* or *ex vivo.* In specific embodiments, the Vitamin A metabolite is retinoic acid. In certain embodiments, the genetically modified cells containing one or more gastrointestinal homing receptors additionally comprise a B cell zone homing receptor. In specific embodiments, the B cell zone homing receptor is CXCR5. In certain embodiments, the genetically modified cells express a gastrointestinal homing receptor and also express a CAR, as described in Section 4.4. In certain embodiments, the genetically modified cells express a CAR and endogenously express one or more gastrointestinal homing receptors.

Also provided herein are methods of generating genetically modified cells, e.g. human T lymphocytes, which comprise one or more receptors that cause a cell expressing the one or more receptors to home to the gastrointestinal tract, e.g., gastrointestinal organs, skin, or tissue. In certain embodiments, T lymphocytes comprising one or more receptors that that cause a cell expressing the one or more receptors to home to the gastrointestinal tract, e.g., CCR9 or integrin α4β7, are generated by a method comprising a step of engineering a T lymphocyte to express one or more gastrointestinal homing receptors. In some embodiments, the step of engineering a T cell to express one or more gastrointestinal homing receptors comprises introducing to the cells one or more vectors comprising a nucleic acid sequence encoding the receptor. In specific embodiments, the vector comprises the nucleic acid sequence for human CCR9, the nucleic acid sequence for human integrin α4β7, or both.

In certain embodiments, T lymphocytes that home to the gastrointestinal tract are generated by a method comprising a step of treating the cells with a molecule that induces the expression of one or more gastrointestinal homing receptors, e.g., CCR9 or α4β7. In specific embodiments, the molecule is Vitamin A.

In certain embodiments, the method for generating the genetically modified T lymphocytes that comprise one or more receptors that that cause a cell expressing the one or more receptors to home to the gastrointestinal tract comprises a step of expanding the cells, which step is carried out in the presence of a vitamin A metabolite. In certain embodiments, the method for generating the genetically modified T lymphocytes that comprise one or more receptors homing to the gastrointestinal tract comprises a step of activating the cells, which step is carried out in the presence of a vitamin A metabolite. In certain embodiments, both the expanding and activating steps are carried out in the presence of a vitamin A metabolite. In certain embodiments the vitamin A metabolite is retinoic acid. In a certain embodiment, the step of engineering a T cell to express a gastrointestinal homing receptor is performed by any method known to one of skill in the art.

Exemplary nucleic acids useful for engineering a T lymphocyte to express a gastrointestinal homing receptor are discussed, for example, in Section 4.5.

Also provided herein are methods of treating a gastrointestinal cancer or tumor in an individual comprising administering to the individual a therapeutically effective amount of genetically modified cells, e.g. human T lymphocytes that comprise (i) one or more receptors that cause a cell expressing the one or more receptors to home to the gastrointestinal tract, and (ii) a CAR. In certain embodiments, the gastrointestinal homing receptor is CCR9 or α4β7. In some embodiments, the genetically modified cells further comprise a second gastrointestinal homing receptor. In some embodiments, the genetically modified cells comprise a first gastrointestinal homing receptor, wherein the first gastrointestinal homing receptor is CCR9, and further comprise a second gastrointestinal homing receptor, wherein the second gastrointestinal homing receptor is α4β7. In certain embodiments, the genetically modified cells, e.g. human T lymphocytes, that comprise (i) one or more receptors that cause a cell expressing the one or more receptors to home to the gastrointestinal tract, and (ii) a CAR, suppress the proliferation of tumor cells. In certain embodiments, the genetically modified cells, e.g. human T lymphocytes, that comprise (i) one or more receptors that cause a cell expressing the one or more receptors to home to the gastrointestinal tract, and (ii) a CAR, inhibit growth of the tumor. In certain embodiments, the genetically modified cells, e.g. human T lymphocytes, that comprise (i) one or more receptors that cause a cell expressing the one or more receptors to home to the gastrointestinal tract, and (ii) a CAR, kill tumor cells. In specific embodiments, the gastrointestinal cancer or tumor is liver cancer, stomach cancer, esophageal cancer, gallbladder cancer, colorectal cancer, anal cancer, or pancreatic cancer. In certain embodiments, the extracellular domain of the CAR binds an antigen associated with a gastrointestinal cancer or tumor. Also provided herein are genetically modified cells for use in methods of treating a gastrointestinal cancer or tumor.

### 4.3. Cells Comprising Skin Homing Receptors, Methods of Making, and Uses Thereof

In one embodiment, provided herein are genetically modified cells, for example immune cells, such as T lymphocytes, e.g., human T lymphocytes, that comprise a receptor that causes a cell expressing said receptor to home to the skin, e.g., skin tissue, or skin cells. In a specific embodiment, the genetically modified cells provided herein are T lymphocytes. In certain embodiments, the skin homing receptor is CCR10, CCR8, CCR4, or CLA, for example, human CCR10, human CCR8, human CCR4, or human CLA. GenBank™ accession numbers NM_016602.2 and AF215981.1 provide exemplary nucleotide sequences for human CCR10. GenBank™ accession numbers NP_057686.2 and P46092.3 provide exemplary amino acid sequences for human CCR10. GenBank™ accession numbers NM_005201.3 and BC107159.1 provide exemplary nucleotide sequences for human CCR8. GenBank™ accession numbers NP_005192.1 and AAI07160.1 provide exemplary amino acid sequences for human CCR8. GenBank™ accession number NM_005508.4 provides an exemplary nucleotide sequence for human CCR4. GenBank™ accession number P51679.1 provides an exemplary amino acid sequence for human CCR4. GenBank™ accession numbers NM_001206609.1 and NM_003006.4 provide exemplary nucleotide sequences for human CLA. GenBank™ accession numbers NP_001193538.1 and NP_002997.2 provide exemplary amino acid sequences for human CLA. Exemplary nucleotide and amino acid sequences for human homing receptors can be found in Table 1. In some embodiments, the genetically modified cells further comprise a second skin homing receptor. In some embodiments, the genetically modified cells comprise a first skin homing receptor, wherein the first skin homing receptor is CCR10, and further comprise a second skin homing receptor, wherein the second skin homing receptor is CLA. In some embodiments, the genetically modified cells comprise a first skin homing receptor, wherein the first skin homing receptor is CCR10, and further comprise a second skin homing receptor, wherein the second skin homing receptor is CCR4. In some embodiments, the genetically modified cells comprise a first skin homing receptor, wherein the first skin homing receptor is CCR4, and further comprise a second skin homing receptor, wherein the second skin homing receptor is CLA. In some embodiments, the genetically modified cells further comprise a third skin homing receptor. In some embodiments, the genetically modified cells comprise a first skin homing receptor, wherein the first skin homing receptor is CCR10, further comprise a second skin homing receptor, wherein the second skin homing receptor is CCR4, and further comprise a third skin homing receptor, wherein the third skin homing receptor is CLA. In some embodiments, the genetically modified cells comprise a first skin homing receptor, wherein the first skin homing receptor is CCR8, and further comprise a second skin homing receptor, wherein the second skin homing receptor is CLA, CCR4, or CCR10. In some embodiments, the genetically modified cells comprise a first skin homing receptor, wherein the first skin homing receptor is CCR8, further comprise a second skin homing receptor, wherein the second skin homing receptor is CLA, CCR4, or CCR10, and further comprise a third skin homing receptor, wherein the third skin homing receptor is distinct from the second skin homing receptor, and is selected from the group consisting of CLA, CCR4, and CCR10. In some embodiments, the genetically modified cells further comprise a third skin homing receptor. In some embodiments, the genetically modified cells comprise a first skin homing receptor, wherein the first skin homing receptor is CCR10, further comprise a second skin homing receptor, wherein the second skin homing receptor is CCR4, further comprise a third skin homing receptor, wherein the third skin homing receptor is CLA, and further comprise a fourth skin homing receptor, wherein the fourth skin homing receptor is CCR8. In certain embodiments, the genetically modified cells comprise one or more skin homing receptors. In other specific embodiments, the genetically modified cells comprise the skin-homing receptor CCR6.

In certain embodiments, the genetically modified cells containing one or more skin homing receptors are expanded, activated, or both expanded and activated in the presence of a Vitamin D metabolite. In specific embodiments, the expansion, activation, or both expansion and activation occurs *in vivo, in vitro,* or *ex vivo.* In specific embodiments, the Vitamin D metabolite is 1,25-dihydroxycholecalciferol (1,25(OH)₂D₃). In certain embodiments, the genetically modified cells containing one or more skin homing receptors are expanded, activated, or both expanded and activated in the presence of IL-12. In specific embodiments, the expansion, activation, or both expansion and activation occurs *in vivo, in vitro,* or *ex vivo.* In more specific embodiments, the genetically modified cells containing one or more skin homing receptors are expanded, activated, or both expanded and activated in the presence of a Vitamin D metabolite and IL-12. In specific embodiments, the expansion, activation, or both expansion and activation occurs *in vivo, in vitro,* or *ex vivo.* In certain embodiments, the genetically modified cells containing one or more skin homing receptors additionally comprise a B cell zone homing receptor. In specific embodiments, the B cell zone homing receptor is CXCR5. In certain embodiments, the genetically modified cells express a skin homing receptor and also express a CAR, as described in Section 4.4. In certain embodiments, the genetically modified cells express a CAR and endogenously express one or more skin homing receptors.

Also provided herein are methods of generating genetically modified cells, e.g. human T lymphocytes, that comprise one or more receptors homing to the skin, e.g., skin tissue or cells. In certain embodiments, T lymphocytes that home to the skin are generated by a method comprising a step of engineering the T lymphocytes to express a skin homing receptor, e.g., CCR4, CCR8, CCR10, or CLA. In some embodiments, the step of engineering the T lymphocytes to express a skin homing receptor comprises introducing into the cells one or more vectors comprising the receptor nucleic acid sequence(s), i.e., the nucleic acid sequence(s) encoding the receptor(s). In specific embodiments, the vector comprises the nucleic acid sequence for human CCR10, the nucleic acid sequence for human CLA, or both. In specific embodiments, the vector comprises the nucleic acid sequence for human CCR4, and optionally the nucleic acid sequence for human CLA. In specific embodiments, the vector comprises the nucleic acid sequence for human CCR4 and the nucleic acid sequence for human CCR10. In specific embodiments, the vector comprises the nucleic acid sequence for human CCR10, the nucleic acid sequence for human CCR4, and the nucleic acid sequence for human CLA. In specific embodiments, the vector comprises the nucleic acid sequence for human CCR8. In specific embodiments, the vector comprises the nucleic acid sequence for human CCR8, and optionally the nucleic acid sequence for human CLA. In specific embodiments, the vector comprises the nucleic acid sequence for human CCR8 and the nucleic acid sequence for human CCR10. In specific embodiments, the vector comprises the nucleic acid sequence for human CCR8, the nucleic acid sequence for human CCR4, and the nucleic acid sequence for human CLA. In specific embodiments, the vector comprises the nucleic acid sequence for human CCR8, the nucleic acid sequence for human CCR10, and the nucleic acid sequence for human CLA. In specific embodiments, the vector comprises the nucleic acid sequence for human CCR8, the nucleic acid sequence for human CCR4, and the nucleic acid sequence for human CCR10. In specific embodiments, the vector comprises the nucleic acid sequence for human CCR8, the nucleic acid sequence for human CCR4, the nucleic acid for CCR10, and the nucleic acid sequence for human CLA.

In certain embodiments, cells, e.g., T lymphocytes, that home to the skin are generated by a method comprising a step of treating the cells, e.g., T lymphocytes, with a molecule that induces, e.g., increases, the expression of one or more skin homing receptors, e.g., CCR4, CCR10, CCR8, or CLA. In specific embodiments, the molecule is Vitamin D. In certain embodiments, the induction of expression of skin homing receptors is aided by treating the cells, e.g., T lymphocytes, with IL-12, e.g., contacting the cells with IL-12 in an amount and for a time sufficient to increase expression of one or more of CCR4, CCR8, CCR10, or CLA by said cells.

In certain embodiments, the method for generating the genetically modified T lymphocytes that comprise one or more receptors that cause a cell expressing the one or more receptors to home to the skin, comprises a step of expanding the cells, which step is carried out in the presence of a vitamin D metabolite and, optionally, IL-12. In certain embodiments, the method for generating the genetically modified T lymphocytes that comprise one or more receptors that that cause a cell expressing the one or more receptors to home to the gastrointestinal tract, comprises a step of activating the cells, which step is carried out in the presence of a vitamin D metabolite, and, optionally, IL-12. In certain embodiments, both the expanding and activating steps are carried out in the presence of a vitamin D metabolite, and, optionally, IL-12. In certain embodiments the vitamin D metabolite is 1,25(OH)₂D₃. In a certain embodiment, the step of engineering a T cell to express a skin homing receptor is performed by any method known to one of skill in the art.

Exemplary nucleic acids useful for engineering a T lymphocyte to express a skin homing receptor are discussed, for example, in Section 4.5.

Also provided herein are methods of treating a skin cancer or tumor in an individual comprising administering to the individual a therapeutically effective amount of genetically modified cells, e.g. human T lymphocytes that comprise (i) one or more receptors that that cause a cell expressing the one or more receptors to home to the skin, and (ii) a CAR. In certain embodiments, the skin homing receptor is CCR10, CCR8, CCR4, or CLA. In some embodiments, the genetically modified cells further comprise a second skin homing receptor. In some embodiments, the genetically modified cells comprise a first skin homing receptor, wherein the first skin homing receptor is CCR10, and further comprise a second skin homing receptor, wherein the second skin homing receptor is CLA. In some embodiments, the genetically modified cells comprise a first skin homing receptor, wherein the first skin homing receptor is CCR10, and further comprise a second skin homing receptor, wherein the second skin homing receptor is CCR4. In some embodiments, the genetically modified cells comprise a first skin homing receptor, wherein the first skin homing receptor is CCR10, and further comprise a second skin homing receptor, wherein the second skin homing receptor is CCR8. In some embodiments, the genetically modified cells comprise a first skin homing receptor, wherein the first skin homing receptor is CCR4, and further comprise a second skin homing receptor, wherein the second skin homing receptor is CLA. In some embodiments, the genetically modified cells comprise a first skin homing receptor, wherein the first skin homing receptor is CCR4, and further comprise a second skin homing receptor, wherein the second skin homing receptor is CCR8. In some embodiments, the genetically modified cells comprise a first skin homing receptor, wherein the first skin homing receptor is CLA, and further comprise a second skin homing receptor, wherein the second skin homing receptor is CCR8. In some embodiments, the genetically modified cells further comprise a third skin homing receptor. In some embodiments, the genetically modified cells comprise a first skin homing receptor, wherein the first skin homing receptor is CCR10, further comprise a second skin homing receptor, wherein the second skin homing receptor is CCR4, and further comprise a third skin homing receptor, wherein the third skin homing receptor is CLA. In some embodiments, the genetically modified cells comprise a first skin homing receptor, wherein the first skin homing receptor is CCR10, further comprise a second skin homing receptor, wherein the second skin homing receptor is CCR4, and further comprise a third skin homing receptor, wherein the third skin homing receptor is CCR8. In some embodiments, the genetically modified cells comprise a first skin homing receptor, wherein the first skin homing receptor is CCR8, further comprise a second skin homing receptor, wherein the second skin homing receptor is CCR4, and further comprise a third skin homing receptor, wherein the third skin homing receptor is CLA. In some embodiments, the genetically modified cells comprise a first skin homing receptor, wherein the first skin homing receptor is CCR10, further comprise a second skin homing receptor, wherein the second skin homing receptor is CCR8, and further comprise a third skin homing receptor, wherein the third skin homing receptor is CLA. In some embodiments, the genetically modified cells further comprise a fourth skin homing receptor. In some embodiments, the genetically modified cells comprise a first skin homing receptor, wherein the first skin homing receptor is CCR10, further comprise a second skin homing receptor, wherein the second skin homing receptor is CCR4, further comprise a third skin homing receptor, wherein the third skin homing receptor is CLA, and further comprise a fourth skin homing receptor, wherein the fourth skin homing receptor is CCR8. In certain embodiments, the genetically modified cells, e.g. human T lymphocytes, that comprise (i) one or more receptors that cause a cell expressing the one or more receptors to home to the skin, and (ii) a CAR, suppress the proliferation of tumor cells. In certain embodiments, the genetically modified cells, e.g. human T lymphocytes, that comprise (i) one or more receptors that cause a cell expressing the one or more receptors to home to the skin, and (ii) a CAR, inhibit growth of the tumor. In certain embodiments, the genetically modified cells, e.g. human T lymphocytes, that comprise (i) one or more receptors that cause a cell expressing the one or more receptors to home to the skin, and (ii) a CAR, kill tumor cells. In specific embodiments, the skin cancer or tumor is melanoma, squamous cell carcinoma, or basal cell carcinoma. In certain embodiments, the extracellular domain of the CAR binds an antigen associated with a skin cancer or tumor. Also provided herein are genetically modified cells for use in methods of treating a skin cancer or tumor.

**Table 1. Exemplary nucleotide and amino acid sequences for human homing receptors.**

| **SEQ ID NO:** | **GenBank Accession Number and Description** | **Sequence** |
|---|---|---|
| 1 | NM_001716.4 Exemplary nucleic acid sequence encoding human CXCR5 | 1 aaaaaaaaaa agtgatgagt tgtgaggcag gtcgcggccc tactgcctca ggagacgatg 61 cgcagctcat ttgcttaaat ttgcagctga cggctgccac ctctctagag gcacctggcg 121 gggagcctct caacataaga cagtgaccag tctggtgact cacagccggc acagccatga 181 actacccgct aacgctggaa atggacctcg agaacctgga ggacctgttc tgggaactgg 241 acagattgga caactataac gacacctccc tggtggaaaa tcatctctgc cctgccacag 301 aggggcccct catggcctcc ttcaaggccg tgttcgtgcc cgtggcctac agcctcatct 361 tcctcctggg cgtgatcggc aacgtcctgg tgctggtgat cctggagcgg caccggcaga 421 cacgcagttc cacggagacc ttcctgttcc acctggccgt ggccgacctc ctgctggtct |
| | | 481 tcatcttgcc ctttgccgtg gccgaggcct ctgtgggctg ggtcctgggg accttcctct 541 gcaaaactgt gattgccctg cacaaagtca acttctactg cagcagcctg ctcctggcct 601 gcatcgccgt ggaccgctac ctggccattg tccacgccgt ccatgcctac cgccaccgcc 661 gcctcctctc catccacatc acctgtggga ccatctggct ggtgggcttc ctccttgcct 721 tgccagagat tctcttcgcc aaagtcagcc aaggccatca caacaactcc ctgccacgtt 781 gcaccttctc ccaagagaac caagcagaaa cgcatgcctg gttcacctcc cgattcctct 841 accatgtggc gggattcctg ctgcccatgc tggtgatggg ctggtgctac gtgggggtag 901 tgcacaggtt gcgccaggcc cagcggcgcc ctcagcggca gaaggcagtc agggtggcca 961 tcctggtgac aagcatcttc ttcctctgct ggtcacccta ccacatcgtc atcttcctgg 1021 acaccctggc gaggctgaag gccgtggaca atacctgcaa gctgaatggc tctctccccg 1081 tggccatcac catgtgtgag ttcctgggcc tggcccactg ctgcctcaac cccatgctct 1141 acactttcgc cggcgtgaag ttccgcagtg acctgtcgcg gctcctgacg aagctgggct 1201 gtaccggccc tgcctccctg tgccagctct tccctagctg gcgcaggagc agtctctctg 1261 agtcagagaa tgccacctct ctcaccacgt tctaggtccc agtgtcccct tttattgctg 1321 cttttccttg gggcaggcag tgatgctgga tgctccttcc aacaggagct gggatcctaa 1381 gggctcaccg tggctaagag tgtcctagga gtatcctcat ttgggtagct tagaggaacc 1441 aacccccatt tctagaacat ccctgccagc tcttctgccg gccctggggc taggctggag 1501 cccagggagc ggaaagcagc tcaaaggcac agtgaaggct gtccttaccc atctgcaccc 1561 ccctgggctg agagaacctc acgcacctcc catcctaatc atccaatgct caagaaacaa 1621 cttctacttc tgcccttgcc aacggagagc gcctgcccct cccagaacac actccatcag 1681 cttaggggct gctgacctcc acagcttccc ctctctcctc ctgcccacct gtcaaacaaa 1741 gccagaagct gagcaccagg ggatgagtgg aggttaaggc tgaggaaagg ccagctggca 1801 gcagagtgtg gccttcggac aactcagtcc ctaaaaacac agacattctg ccaggccccc 1861 aagcctgcag tcatcttgac caagcaggaa gctcagactg gttgagttca ggtagctgcc 1921 cctggctctg accgaaacag cgctgggtcc accccatgtc accggatcct gggtggtctg 1981 caggcagggc tgactctagg tgcccttgga ggccagccag tgacctgagg aagcgtgaag 2041 gccgagaagc aagaaagaaa cccgacagag ggaagaaaag agctttcttc ccgaacccca 2101 aggagggaga tggatcaatc aaacccggcg gtcccctccg ccaggcgaga tggggtgggg 2161 tggagaactc ctagggtggc tgggtccagg ggatgggagg ttgtgggcat tgatggggaa 2221 ggaggctggc ttgtcccctc ctcactccct tcccataagc tatagacccg aggaaactca 2281 gagtcggaac ggagaaaggt ggactggaag gggcccgtgg gagtcatctc aaccatcccc 2341 tccgtggcat caccttaggc agggaagtgt aagaaacaca ctgaggcagg gaagtcccca 2401 ggccccagga agccgtgccc tgcccccgtg aggatgtcac tcagatggaa ccgcaggaag 2461 ctgctccgtg cttgtttgct cacctggggt gtgggaggcc cgtccggcag ttctgggtgc 2521 tccctaccac ctccccagcc tttgatcagg tggggagtca gggacccctg cccttgtccc 2581 actcaagcca agcagccaag ctccttggga ggccccactg gggaaataac agctgtggct 2641 cacgtgagag tgtcttcacg gcaggacaac gaggaagccc taagacgtcc cttttttctc 2701 tgagtatctc ctcgcaagct gggtaatcga tgggggagtc tgaagcagat gcaaagaggc 2761 aagaggctgg attttgaatt ttctttttaa taaaaaggca cctataaaac aggtcaatac 2821 agtacaggca gcacagagac ccccggaaca agcctaaaaa ttgtttcaaa ataaaaacca 2881 agaagatgtc ttcacatatt gtaaaaaaaa aaaaaaaaa |
| 2 | NM_032966.2 Exemplary nucleic acid sequence encoding human | 1 ccactctaag gaatgcggtc cctttgacag gcgaaaaact gaagttggaa aagacaaagt 61 gatttgttca aaattgaaat ttgaaacttg acatttggtc agtgggccct atgtaggaaa 121 aaacctccaa gagagctagg gttcctctca gagaggaaag acaggtcctt aggtcctcac 181 cctcccgtct ccttgccctt gcagttctgg gaactggaca gattggacaa ctataacgac 241 acctccctgg tggaaaatca tctctgccct gccacagagg ggcccctcat ggcctccttc |
| | CXCR5 | 301 aaggccgtgt tcgtgcccgt ggcctacagc ctcatcttcc tcctgggcgt gatcggcaac 361 gtcctggtgc tggtgatcct ggagcggcac cggcagacac gcagttccac ggagaccttc 421 ctgttccacc tggccgtggc cgacctcctg ctggtcttca tcttgccctt tgccgtggcc 481 gagggctctg tgggctgggt cctggggacc ttcctctgca aaactgtgat tgccctgcac 541 aaagtcaact tctactgcag cagcctgctc ctggcctgca tcgccgtgga ccgctacctg 601 gccattgtcc acgccgtcca tgcctaccgc caccgccgcc tcctctccat ccacatcacc 661 tgtgggacca tctggctggt gggcttcctc cttgccttgc cagagattct cttcgccaaa 721 gtcagccaag gccatcacaa caactccctg ccacgttgca ccttctccca agagaaccaa 781 gcagaaacgc atgcctggtt cacctcccga ttcctctacc atgtggcggg attcctgctg 841 cccatgctgg tgatgggctg gtgctacgtg ggggtagtgc acaggttgcg ccaggcccag 901 cggcgccctc agcggcagaa ggcagtcagg gtggccatcc tggtgacaag catcttcttc 961 ctctgctggt caccctacca catcgtcatc ttcctggaca ccctggcgag gctgaaggcc 1021 gtggacaata cctgcaagct gaatggctct ctccccgtgg ccatcaccat gtgtgagttc 1081 ctgggcctgg cccactgctg cctcaacccc atgctctaca ctttcgccgg cgtgaagttc 1141 cgcagtgacc tgtcgcggct cctgacgaag ctgggctgta ccggccctgc ctccctgtgc 1201 cagctcttcc ctagctggcg caggagcagt ctctctgagt cagagaatgc cacctctctc 1261 accacgttct aggtcccagt gtcccctttt attgctgctt ttccttgggg caggcagtga 1321 tgctggatgc tccttccaac aggagctggg atcctaaggg ctcaccgtgg ctaagagtgt 1381 cctaggagta tcctcatttg gggtagctag aggaaccaac ccccatttct agaacatccc 1441 tgccagctct tctgccggcc ctggggctag gctggagccc agggagcgga aagcagctca 1501 aaggcacagt gaaggctgtc cttacccatc tgcacccccc tgggctgaga gaacctcacg 1561 cacctcccat cctaatcatc caatgctcaa gaaacaactt ctacttctgc ccttgccaac 1621 ggagagcgcc tgcccctccc agaacacact ccatcagctt aggggctgct gacctccaca 1681 gcttcccctc tctcctcctg cccacctgtc aaacaaagcc agaagctgag caccagggga 1741 tgagtggagg ttaaggctga ggaaaggcca gctggcagca gagtgtggcc ttcggacaac 1801 tcagtcccta aaaacacaga cattctgcca ggcccccaag cctgcagtca tcttgaccaa 1861 gcaggaagct cagactggtt gagttcaggt agctgcccct ggctctgacc gaaacagcgc 1921 tgggtccacc ccatgtcacc ggatcctggg tggtctgcag gcagggctga ctctaggtgc 1981 ccttggaggc cagccagtga cctgaggaag cgtgaaggcc gagaagcaag aaagaaaccc 2041 gacagaggga agaaaagagc tttcttcccg aaccccaagg agggagatgg atcaatcaaa 2101 cccggcggtc ccctccgcca ggcgagatgg ggtggggtgg agaactccta gggtggctgg 2161 gtccagggga tgggaggttg tgggcattga tggggaagga ggctggcttg tcccctcctc 2221 actcccttcc cataagctat agacccgagg aaactcagag tcggaacgga gaaaggtgga 2281 ctggaagggg cccgtgggag tcatctcaac catcccctcc gtggcatcac cttaggcagg 2341 gaagtgtaag aaacacactg aggcagggaa gtccccaggc cccaggaagc cgtgccctgc 2401 ccccgtgagg atgtcactca gatggaaccg caggaagctg ctccgtgctt gtttgctcac 2461 ctggggtgtg ggaggcccgt ccggcagttc tgggtgctcc ctaccacctc cccagccttt 2521 gatcaggtgg ggagtcaggg acccctgccc ttgtcccact caagccaagc agccaagctc 2581 cttgggaggc cccactgggg aaataacagc tgtggctcac gtgagagtgt cttcacggca 2641 ggacaacgag gaagccctaa gacgtccctt ttttctctga gtatctcctc gcaagctggg 2701 taatcgatgg gggagtctga agcagatgca aagaggcaag aggctggatt ttgaattttc 2761 tttttaataa aaaggcacct ataaaacagg tcaatacagt acaggcagca cagagacccc 2821 cggaacaagc ctaaaaattg tttcaaaata aaaaccaaga agatgtcttc acatattgta 2881 aaaaaaaaaa aaaaaa |
| 3 | NP_116743.1 | 1 masfkavfvp vayslifllg vignvlvlvi lerhrqtrss tetflfhlav adlllvfilp 61 favaegsvgw vlgtflcktv ialhkvnfyc sslllaciav drylaivhav hayrhrrlls |
| | Exemplary amino acid sequence for human CXCR5 precursor | 121 ihitcgtiwl vgfllalpei lfakvsqghh nnslprctfs qenqaethaw ftsrflyhva 181 gfllpmlvmg wcyvgvvhrl rqaqrrpqrq kavrvailvt sifflcwspy hivifldtla 241 rlkavdntck lngslpvait mceflglahc clnpmlytfa gvkfrsdlsr lltklgctgp 301 aslcqlfpsw rrsslsesen atslttf |
| 4 | NP_001707.1 Exemplary amino acid sequence for human CXCR5 precursor | 1 mnypltlemd lenledlfwe ldrldnyndt slvenhlcpa tegplmasfk avfvpvaysl 61 ifllgvignv lvlvilerhr qtrsstetfl fhlavadlll vfilpfavae gsvgwvlgtf 121 lcktvialhk vnfycsslll aciavdryla ivhavhayrh rrllsihitc gtiwlvgfll 181 alpeilfakv sqghhnnslp rctfsqenqa ethawftsrf lyhvagfllp mlvmgwcyvg 241 vvhrlrqaqr rpqrqkavrv ailvtsiffl cwspyhivif ldtlarlkav dntcklngsl 301 pvaitmcefl glahcclnpm lytfagvkfr sdlsrlltkl gctgpaslcq lfpswrrssl 361 sesenatslt tf |
| 5 | NM_031200.2 Exemplary nucleic acid sequence encoding human CCR9 | 1 gcttcctttc tcgtgttgtt atcgggtagc tgcctgctca gaacccacaa agcctgcccc 61 tcatcccagg cagagagcaa cccagctctt tccccagaca ctgagagctg gtggtgcctg 121 ctgtcccagg gagagttgca tcgccctcca cagagcaggc ttgcatctga ctgacccacc 181 atgacaccca cagacttcac aagccctatt cctaacatgg ctgatgacta tggctctgaa 241 tccacatctt ccatggaaga ctacgttaac ttcaacttca ctgacttcta ctgtgagaaa 301 aacaatgtca ggcagtttgc gagccatttc ctcccaccct tgtactggct cgtgttcatc 361 gtgggtgcct tgggcaacag tcttgttatc cttgtctact ggtactgcac aagagtgaag 421 accatgaccg acatgttcct tttgaatttg gcaattgctg acctcctctt tcttgtcact 481 cttcccttct gggccattgc tgctgctgac cagtggaagt tccagacctt catgtgcaag 541 gtggtcaaca gcatgtacaa gatgaacttc tacagctgtg tgttgctgat catgtgcatc 601 agcgtggaca ggtacattgc cattgcccag gccatgagag cacatacttg gagggagaaa 661 aggcttttgt acagcaaaat ggtttgcttt accatctggg tattggcagc tgctctctgc 721 atcccagaaa tcttatacag ccaaatcaag gaggaatccg gcattgctat ctgcaccatg 781 gtttacccta gcgatgagag caccaaactg aagtcagctg tcttgaccct gaaggtcatt 841 ctggggttct tccttccctt cgtggtcatg gcttgctgct ataccatcat cattcacacc 901 ctgatacaag ccaagaagtc ttccaagcac aaagccctaa aagtgaccat cactgtcctg 961 accgtctttg tcttgtctca gtttccctac aactgcattt tgttggtgca gaccattgac 1021 gcctatgcca tgttcatctc caactgtgcc gtttccacca acattgacat ctgcttccag 1081 gtcacccaga ccatcgcctt cttccacagt tgcctgaacc ctgttctcta tgtttttgtg 1141 ggtgagagat tccgccggga tctcgtgaaa accctgaaga acttgggttg catcagccag 1201 gcccagtggg tttcatttac aaggagagag ggaagcttga agctgtcgtc tatgttgctg 1261 gagacaacct caggagcact ctccctctga ggggtcttct ctgaggtgca tggttctttt 1321 ggaagaaatg agaaatacag aaacagtttc cccactgatg ggaccagaga gagtgaaaga 1381 gaaaagaaaa ctcagaaagg gatgaatctg aactatatga ttacttgtag tcagaatttg 1441 ccaaagcaaa tatttcaaaa tcaactgact agtgcaggag gctgttgatt ggctcttgac 1501 tgtgatgccc gcaattctca aaggaggact aaggaccggc actgtggagc accctggctt 1561 tgccactcgc cggagcatca atgccgctgc ctctggagga gcccttggat tttctccatg 1621 cactgtgaac ttctgtggct tcagttctca tgctgcctct tccaaaaggg gacacagaag 1681 cactggctgc tgctacagac cgcaaaagca gaaagtttcg tgaaaatgtc catctttggg 1741 aaattttcta ccctgctctt gagcctgata acccatgcca ggtcttatag attcctgatc 1801 tagaaccttt ccaggcaatc tcagacctaa tttccttctg ttctccttgt tctgttctgg 1861 gccagtgaag gtccttgttc tgattttgaa acgatctgca ggtcttgcca gtgaacccct 1921 ggacaactga ccacacccac aaggcatcca aagtctgttg gcttccaatc catttctgtg 1981 tcctgctgga ggttttaacc tagacaagga ttccgcttat tccttggtat ggtgacagtg |
| | | 2041 tctctccatg gcctgagcag ggagattata acagctgggt tcgcaggagc cagccttggc 2101 cctgttgtag gcttgttctg ttgagtggca cttgctttgg gtccaccgtc tgtctgctcc 2161 ctagaaaatg ggctggttct tttggccctc ttctttctga ggcccacttt attctgagga 2221 atacagtgag cagatatggg cagcagccag gtagggcaaa ggggtgaagc gcaggccttg 2281 ctggaaggct atttacttcc atgcttctcc ttttcttact ctatagtggc aacattttaa 2341 aagcttttaa cttagagatt aggctgaaaa aaataagtaa tggaattcac ctttgcatct 2401 tttgtgtctt tcttatcatg atttggcaaa atgcatcacc tttgaaaata tttcacatat 2461 tggaaaagtg ctttttaatg tgtatatgaa gcattaatta cttgtcactt tctttaccct 2521 gtctcaatat tttaagtgtg tgcaattaaa gatcaaatag atacatt |
| 6 | NM001256369.1 Exemplary nucleic acid sequence encoding human CCR9 | 1 gcttcctttc tcgtgttgtt atcgggtagc tgcctgctca gaacccacaa agcctgcccc 61 tcatcccagg cagagagcaa cccagctctt tccccagaca ctgagagctg gtggtgcctg 121 ctgtcccagg gagagttgca tcgccctcca cagagcaggc ttgcatctga ctgacccacc 181 atgacaccca cagacttcac atctcctcca ggccccgctc cagatcacct tccctcgctg 241 gcccaggaat ccatctcctt ccaggacctt agcccaggac taacacaagc cctattccta 301 acatggctga tgactatggc tctgaatcca catcttccat ggaagactac gttaacttca 361 acttcactga cttctactgt gagaaaaaca atgtcaggca gtttgcgagc catttcctcc 421 caccettgta ctggctcgtg ttcatcgtgg gtgccttggg caacagtctt gttatccttg 481 tctactggta ctgcacaaga gtgaagacca tgaccgacat gttccttttg aatttggcaa 541 ttgctgacct cctctttctt gtcactcttc ccttctgggc cattgctgct gctgaccagt 601 ggaagttcca gaccttcatg tgcaaggtgg tcaacagcat gtacaagatg aacttctaca 661 gctgtgtgtt gctgatcatg tgcatcagcg tggacaggta cattgccatt gcccaggcca 721 tgagagcaca tacttggagg gagaaaaggc ttttgtacag caaaatggtt tgctttacca 781 tctgggtatt ggcagctgct ctctgcatcc cagaaatctt atacagccaa atcaaggagg 841 aatccggcat tgctatctgc accatggttt accctagcga tgagagcacc aaactgaagt 901 cagctgtctt gaccctgaag gtcattctgg ggttcttcct tcccttcgtg gtcatggctt 961 gctgctatac catcatcatt cacaccctga tacaagccaa gaagtcttcc aagcacaaag 1021 ccctaaaagt gaccatcact gtcctgaccg tctttgtctt gtctcagttt ccctacaact 1081 gcattttgtt ggtgcagacc attgacgcct atgccatgtt catctccaac tgtgccgttt 1141 ccaccaacat tgacatctgc ttccaggtca cccagaccat cgccttcttc cacagttgcc 1201 tgaaccctgt tctctatgtt tttgtgggtg agagattccg ccgggatctc gtgaaaaccc 1261 tgaagaactt gggttgcatc agccaggccc agtgggtttc atttacaagg agagagggaa 1321 gcttgaagct gtcgtctatg ttgctggaga caacctcagg agcactctcc ctctgagggg 1381 tcttctctga ggtgcatggt tcttttggaa gaaatgagaa atacagaaac agtttcccca 1441 ctgatgggac cagagagagt gaaagagaaa agaaaactca gaaagggatg aatctgaact 1501 atatgattac ttgtagtcag aatttgccaa agcaaatatt tcaaaatcaa ctgactagtg 1561 caggaggctg ttgattggct cttgactgtg atgcccgcaa ttctcaaagg aggactaagg 1621 accggcactg tggagcaccc tggctttgcc actcgccgga gcatcaatgc cgctgcctct 1681 ggaggagccc ttggattttc tccatgcact gtgaacttct gtggcttcag ttctcatgct 1741 gcctcttcca aaaggggaca cagaagcact ggctgctgct acagaccgca aaagcagaaa 1801 gtttcgtgaa aatgtccatc tttgggaaat tttctaccct gctcttgagc ctgataaccc 1861 atgccaggtc ttatagattc ctgatctaga acctttccag gcaatctcag acctaatttc 1921 cttctgttct ccttgttctg ttctgggcca gtgaaggtcc ttgttctgat tttgaaacga 1981 tctgcaggtc ttgccagtga acccctggac aactgaccac acccacaagg catccaaagt 2041 ctgttggctt ccaatccatt tctgtgtcct gctggaggtt ttaacctaga caaggattcc 2101 gcttattcct tggtatggtg acagtgtctc tccatggcct gagcagggag attataacag 2161 ctgggttcgc aggagccagc cttggccctg ttgtaggctt gttctgttga gtggcacttg |
| | | 2221 ctttgggtcc accgtctgtc tgctccctag aaaatgggct ggttcttttg gccctcttct 2281 ttctgaggcc cactttattc tgaggaatac agtgagcaga tatgggcagc agccaggtag 2341 ggcaaagggg tgaagcgcag gccttgctgg aaggctattt acttccatgc ttctcctttt 2401 cttactctat agtggcaaca ttttaaaagc ttttaactta gagattaggc tgaaaaaaat 2461 aagtaatgga attcaccttt gcatcttttg tgtctttctt atcatgattt ggcaaaatgc 2521 atcacctttg aaaatatttc acatattgga aaagtgcttt ttaatgtgta tatgaagcat 2581 taattacttg tcactttctt taccctgtct caatatttta agtgtgtgca attaaagatc 2641 aaatagatac att |
| 7 | NP_112477.1 Exemplary amino acid sequence for human CCR9 precursor | 1 mtptdftspi pnmaddygse stssmedyvn fnftdfycek nnvrqfashf lpplywlvfi 61 vgalgnslvi lvywyctrvk tmtdmfllnl aiadllflvt lpfwaiaaad qwkfqtfmck 121 vvnsmykmnf yscvllimci svdryiaiaq amrahtwrek rllyskmvcf tiwvlaaalc 181 ipeilysqik eesgiaictm vypsdestkl ksavltlkvi lgfflpfvvm accytiiiht 241 liqakksskh kalkvtitvl tvfvlsqfpy ncillvqtid ayamfisnca vstnidicfq 301 vtqtiaffhs clnpvlyvfv gerfrrdlvk tlknlgcisq aqwvsftrre gslklssmll 361 ettsgalsl |
| 8 | NP_001243298.1 Exemplary amino acid sequence for human CCR9 precursor | 1 maddygsest ssmedyvnfn ftdfyceknn vrqfashflp plywlvfivg algnslvilv 61 ywyctrvktm tdmfllnlai adllflvtlp fwaiaaadqw kfqtfmckvv nsmykmnfys 121 cvllimcisv dryiaiaqam rahtwrekrl lyskmvcfti wvlaaalcip eilysqikee 181 sgiaictmvy psdestklks avltlkvilg fflpfvvmac cytiiihtli qakksskhka 241 lkvtitvltv fvlsqfpync illvqtiday amfisncavs tnidicfqvt qtiaffhscl 301 npvlyvfvge rfrrdlvktl knlgcisqaq wvsftrregs lklssmllet tsgalsl |
| 9 | NM_000885.4 Exemplary nucleic acid sequence encoding human α4 | 1 ataacgtctt tgtcactaaa atgttcccca ggggccttcg gcgagtcttt ttgtttggtt 61 ttttgttttt aatctgtggc tcttgataat ttatctagtg gttgcctaca cctgaaaaac 121 aagacacagt gtttaactat caacgaaaga actggacggc tccccgccgc agtcccactc 181 cccgagtttg tggctggcat ttgggccacg ccgggctggg cggtcacagc gaggggcgcg 241 cagtttgggg tcacacagct ccgcttctag gccccaacca ccgttaaaag ggaagcccg 301 tgccccatca ggtccgctct tgctgagccc agagccatcc cgcgctctgc gggctgggag 361 gcccgggcca ggacgcgagt cctgcgcagc cgaggttccc cagcgccccc tgcagccgcg 421 cgtaggcaga gacggagccc ggccctgcgc ctccgcacca cgcccgggac cccacccagc 481 ggcccgtacc cggagaagca gcgcgagcac ccgaagctcc cggctggcgg cagaaaccgg 541 gagtggggcc gggcgagtgc gcggcatccc aggccggccc gaacgctccg cccgcggtgg 601 gccgacttcc cctcctcttc cctctctcct tcctttagcc cgctggcgcc ggacacgctg 661 cgcctcatct cttggggcgt tcttccccgt tggccaaccg tcgcatcccg tgcaactttg 721 gggtagtggc cgtttagtgt tgaatgttcc ccaccgagag cgcatggctt gggaagcgag 781 gcgcgaaccc ggcccccgaa gggccgccgt ccgggagacg gtgatgctgt tgctgtgcct 841 gggggtcccg accggccgcc cctacaacgt ggacactgag agcgcgctgc tttaccaggg 901 cccccacaac acgctgttcg gctactcggt cgtgctgcac agccacgggg cgaaccgatg 961 gctcctagtg ggtgcgccca ctgccaactg gctcgccaac gcttcagtga tcaatcccgg 1021 ggcgatttac agatgcagga tcggaaagaa tcccggccag acgtgcgaac agctccagct 1081 gggtagccct aatggagaac cttgtggaaa gacttgtttg gaagagagag acaatcagtg 1141 gttgggggtc acactttcca gacagccagg agaaaatgga tccatcgtga cttgtgggca 1201 tagatggaaa aatatatttt acataaagaa tgaaaataag ctccccactg gtggttgcta 1261 tggagtgccc cctgatttac gaacagaact gagtaaaaga atagctccgt gttatcaaga 1321 ttatgtgaaa aaatttggag aaaattttgc atcatgtcaa gctggaatat ccagttttta |
| | | 1381 cacaaaggat ttaattgtga tgggggcccc aggatcatct tactggactg gctctctttt 1441 tgtctacaat ataactacaa ataaatacaa ggctttttta gacaaacaaa atcaagtaaa 1501 atttggaagt tatttaggat attcagtcgg agctggtcat tttcggagcc agcatactac 1561 cgaagtagtc ggaggagctc ctcaacatga gcagattggt aaggcatata tattcagcat 1621 tgatgaaaaa gaactaaata tcttacatga aatgaaaggt aaaaagcttg gatcgtactt 1681 tggagcttct gtctgtgctg tggacctcaa tgcagatggc ttctcagatc tgctcgtggg 1741 agcacccatg cagagcacca tcagagagga aggaagagtg tttgtgtaca tcaactctgg 1801 ctcgggagca gtaatgaatg caatggaaac aaacctcgtt ggaagtgaca aatatgctgc 1861 aagatttggg gaatctatag ttaatcttgg cgacattgac aatgatggct ttgaagatgt 1921 tgctatcgga gctccacaag aagatgactt gcaaggtgct atttatattt acaatggccg 1981 tgcagatggg atctcgtcaa ccttctcaca gagaattgaa ggacttcaga tcagcaaatc 2041 gttaagtatg tttggacagt ctatatcagg acaaattgat gcagataata atggctatgt 2101 agatgtagca gttggtgctt ttcggtctga ttctgctgtc ttgctaagga caagacctgt 2161 agtaattgtt gacgcttctt taagccaccc tgagtcagta aatagaacga aatttgactg 2221 tgttgaaaat ggatggcctt ctgtgtgcat agatctaaca ctttgtttct catataaggg 2281 caaggaagtt ccaggttaca ttgttttgtt ttataacatg agtttggatg tgaacagaaa 2341 ggcagagtct ccaccaagat tctatttctc ttctaatgga acttctgacg tgattacagg 2401 aagcatacag gtgtccagca gagaagctaa ctgtagaaca catcaagcat ttatgcggaa 2461 agatgtgcgg gacatcctca ccccaattca gattgaagct gcttaccacc ttggtcctca 2521 tgtcatcagt aaacgaagta cagaggaatt cccaccactt cagccaattc ttcagcagaa 2581 gaaagaaaaa gacataatga aaaaaacaat aaactttgca aggttttgtg cccatgaaaa 2641 ttgttctgct gatttacagg tttctgcaaa gattgggttt ttgaagcccc atgaaaataa 2701 aacatatctt gctgttggga gtatgaagac attgatgttg aatgtgtcct tgtttaatgc 2761 tggagatgat gcatatgaaa cgactctaca tgtcaaacta cccgtgggtc tttatttcat 2821 taagatttta gagctggaag agaagcaaat aaactgtgaa gtcacagata actctggcgt 2881 ggtacaactt gactgcagta ttggctatat atatgtagat catctctcaa ggatagatat 2941 tagctttctc ctggatgtga gctcactcag cagagcggaa gaggacctca gtatcacagt 3001 gcatgctacc tgtgaaaatg aagaggaaat ggacaatcta aagcacagca gagtgactgt 3061 agcaatacct ttaaaatatg aggttaagct gactgttcat gggtttgtaa acccaacttc 3121 atttgtgtat ggatcaaatg atgaaaatga gcctgaaacg tgcatggtgg agaaaatgaa 3181 cttaactttc catgttatca acactggcaa tagtatggct cccaatgtta gtgtggaaat 3241 aatggtacca aattctttta gcccccaaac tgataagctg ttcaacattt tggatgtcca 3301 gactactact ggagaatgcc actttgaaaa ttatcaaaga gtgtgtgcat tagagcagca 3361 aaagagtgca atgcagacct tgaaaggcat agtccggttc ttgtccaaga ctgataagag 3421 gctattgtac tgcataaaag ctgatccaca ttgtttaaat ttcttgtgta attttgggaa 3481 aatggaaagt ggaaaagaag ccagtgttca tatccaactg gaaggccggc catccatttt 3541 agaaatggat gagacttcag cactcaagtt tgaaataaga gcaacaggtt ttccagagcc 3601 aaatccaaga gtaattgaac taaacaagga tgagaatgtt gcgcatgttc tactggaagg 3661 actacatcat caaagaccca aacgttattt caccatagtg attatttcaa gtagcttgct 3721 acttggactt attgtacttc tgttgatctc atatgttatg tggaaggctg gcttctttaa 3781 aagacaatac aaatctatcc tacaagaaga aaacagaaga gacagttgga gttatatcaa 3841 cagtaaaagc aatgatgatt aaggacttct ttcaaattga gagaatggaa aacagactca 3901 ggttgtagta aagaaattta aaagacactg tttacaagaa aaaatgaatt ttgtttggac 3961 ttcttttact catgatcttg tgacatatta tgtcttcatg caaggggaaa atctcagcaa 4021 tgattactct ttgagataga agaactgcaa aggtaataat acagccaaag ataatctctc 4081 agcttttaaa tgggtagaga aacactaaag cattcaattt attcaagaaa agtaagccct 4141 tgaagatatc ttgaaatgaa agtataactg agttaaatta tactggagaa gtcttagact |
| | | 4201 tgaaatacta cttaccatat gtgcttgcct cagtaaaatg aaccccactg ggtgggcaga 4261 ggttcatttc aaatacatct ttgatacttg ttcaaaatat gttctttaaa aatataattt 4321 tttagagagc tgttcccaaa ttttctaacg agtggaccat tatcacttta aagcccttta 4381 tttataatac atttcctacg ggctgtgttc caacaaccat tttttttcag cagactatga 4441 atattatagt attataggcc aaactggcaa acttcagact gaacatgtac actggtttga 4501 gcttagtgaa attacttctg gataattatt tttttataat tatggatttc accatctttc 4561 tttctgtata tatacatgtg tttttatgta ggtatatatt taccattctt cctatctatt 4621 cttcctataa cacaccttta tcaagcatac ccaggagtaa tcttcaaatc ttttgttata 4681 ttctgaaaca aaagattgtg agtgttgcac tttacctgat acacgctgat ttagaaaata 4741 cagaaaccat acctcactaa taactttaaa atcaaagctg tgcaaagact agggggccta 4801 tacttcatat gtattatgta ctatgtaaaa tattgactat cacacaacta tttccttgga 4861 tgtaattctt tgttaccctt tacaagtata agtgttacct tacatggaaa cgaagaaaca 4921 aaattcataa atttaaattc ataaatttag ctgaaagata ctgattcaat ttgtatacag 4981 tgaatataaa tgagacgaca gcaaaatttt catgaaatgt aaaatatttt tatagtttgt 5041 tcatactata tgaggttcta ttttaaatga ctttctggat tttaaaaaat ttctttaaat 5101 acaatcattt ttgtaatatt tattttatgc ttatgatcta gataattgca gaatatcatt 5161 ttatctgact ctgccttcat aagagagctg tggccgaatt ttgaacatct gttataggga 5221 gtgatcaaat tagaaggcaa tgtggaaaaa caattctggg aaagatttct ttatatgaag 5281 tccctgccac tagccagcca tcctaattga tgaaagttat ctgttcacag gcctgcagtg 5341 atggtgagga atgttctgag atttgcgaag gcatttgagt agtgaaatgt aagcacaaaa 5401 cctcctgaac ccagagtgtg tatacacagg aataaacttt atgacattta tgtattttta 5461 aaaaactttg tatcgttata aaaaggctag tcattctttc aggagaacat ctaggatcat 5521 agatgaaaaa tcaagccccg atttagaact gtcttctcca ggatggtctc taaggaaatt 5581 tacatttggt tctttcctac tcagaactac tcagaaacaa ctatatattt caggttatct 5641 gagcacagtg aaagcagagt actatggttg tccaacacag gcctctcaga tacaagggga 5701 acacaattac atattgggct agattttgcc cagttcaaaa tagtatttgt tatcaactta 5761 ctttgttact tgtatcatga attttaaaac cctaccactt taagaagaca gggatgggtt 5821 attctttttt ggcaggtagg ctatataact atgtgatttt gaaatttaac tgctctggat 5881 tagggagcag tgaatcaagg cagacttatg aaatctgtat tatatttgta acagaatata 5941 ggaaatttaa cataattgat gagctcaaat cctgaaaaat gaaagaatcc aaattatttc 6001 agaattatct aggttaaata ttgatgtatt atgatggttg caaagttttt ttgtgtgtcc 6061 aataaacaca ttgtaaaaaa aa |
| 10 | NM_000889.2 Exemplary nucleic acid sequence encoding human β7 | 1 aaatcttccc caccctgggg agtgtcactt cctcctctgc cgtctcccag atcagtacac 61 aaaggctgct gctgccgcca gaggaaggac tgctctgcac gcacctatgt ggaaactaaa 121 gcccagagag aaagtctgac ttgccccaca gccagtgagt gactgcagca gcaccagaat 181 ctggtctgtt tcctgtttgg ctcttctacc actacggctt gggatctcgg gcatggtggc 241 tttgccaatg gtccttgttt tgctgctggt cctgagcaga ggtgagagtg aattggacgc 301 caagatccca tccacagggg atgccacaga atggcggaat cctcacctgt ccatgctggg 361 gtcctgccag ccagccccct cctgccagaa gtgcatcctc tcacacccca gctgtgcatg 421 gtgcaagcaa ctgaacttca ccgcgtcggg agaggcggag gcgcggcgct gcgcccgacg 481 agaggagctg ctggctcgag gctgcccgct ggaggagctg gaggagcccc gcggccagca 541 ggaggtgctg caggaccagc cgctcagcca gggcgcccgc ggagagggtg ccacccagct 601 ggcgccgcag cgggtccggg tcacgctgcg gcctggggag ccccagcagc tccaggtccg 661 cttccttcgt gctgagggat acccggtgga cctgtactac cttatggacc tgagctactc 721 catgaaggac gacctggaac gcgtgcgcca gctcgggcac gctctgctgg tccggctgca 781 ggaagtcacc cattctgtgc gcattggttt tggttccttt gtggacaaaa cggtgctgcc |
| | | 841 ctttgtgagc acagtaccct ccaaactgcg ccacccctgc cccacccggc tggagcgctg 901 ccagtcacca ttcagctttc accatgtgct gtccctgacg ggggacgcac aagccttcga 961 gcgggaggtg gggcgccaga gtgtgtccgg caatctggac tcgcctgaag gtggcttcga 1021 tgccattctg caggctgcac tctgccagga gcagattggc tggagaaatg tgtcccggct 1081 gctggtgttc acttcagacg acacattcca tacagctggg gacgggaagt tgggcggcat 1141 tttcatgccc agtgatgggc actgccactt ggacagcaat ggcctctaca gtcgcagcac 1201 agagtttgac tacccttctg tgggtcaggt agcccaggcc ctctctgcag caaatatcca 1261 gcccatcttt gctgtcacca gtgccgcact gcctgtctac caggagctga gtaaactgat 1321 tcctaagtct gcagttgggg agctgagtga ggactccagc aacgtggtac agctcatcat 1381 ggatgcttat aatagcctgt cttccaccgt gacccttgaa cactcttcac tccctcctgg 1441 ggtccacatt tcttacgaat cccagtgtga gggtcctgag aagagggagg gtaaggctga 1501 ggatcgagga cagtgcaacc acgtccgaat caaccagacg gtgactttct gggtttctct 1561 ccaagccacc cactgcctcc cagagcccca tctcctgagg ctccgggccc ttggcttctc 1621 agaggagctg attgtggagt tgcacacgct gtgtgactgt aattgcagtg acacccagcc 1681 ccaggctccc cactgcagtg atggccaggg acacctacaa tgtggtgtat gcagctgtgc 1741 ccctggccgc ctaggtcggc tctgtgagtg ctctgtggca gagctgtcct ccccagacct 1801 ggaatctggg tgccgggctc ccaatggcac agggcccctg tgcagtggaa agggtcactg 1861 tcaatgtgga cgctgcagct gcagtggaca gagctctggg catctgtgcg agtgtgacga 1921 tgccagctgt gagcgacatg agggcatcct ctgcggaggc tttggtcgct gccaatgtgg 1981 agtatgtcac tgtcatgcca accgcacggg cagagcatgc gaatgcagtg gggacatgga 2041 cagttgcatc agtcccgagg gagggctctg cagtgggcat ggacgctgca aatgcaaccg 2101 ctgccagtgc ttggacggct actatggtgc tctatgcgac caatgcccag gctgcaagac 2161 accatgcgag agacaccggg actgtgcaga gtgtggggcc ttcaggactg gcccactggc 2221 caccaactgc agtacagctt gtgcccatac caatgtgacc ctggccttgg cccctatctt 2281 ggatgatggc tggtgcaaag agcggaccct ggacaaccag ctgttcttct tcttggtgga 2341 ggatgacgcc agaggcacgg tcgtgctcag agtgagaccc caagaaaagg gagcagacca 2401 cacgcaggcc attgtgctgg gctgcgtagg gggcatcgtg gcagtggggc tggggctggt 2461 cctggcttac cggctctcgg tggaaatcta tgaccgccgg gaatacagtc gctttgagaa 2521 ggagcagcaa caactcaact ggaagcagga cagtaatcct ctctacaaaa gtgccatcac 2581 gaccaccatc aatcctcgct ttcaagaggc agacagtccc actctctgaa ggagggaggg 2641 acacttaccc aaggctcttc tccttggagg acagtgggaa ctggagggtg agaggaaggg 2701 tgggtctgta agaccttggt aggggactaa ttcactggcg aggtgcggcc accaccctac 2761 ttcattttca gagtgacacc caagagggct gcttcccatg cctgcaacct tgcatccatc 2821 tgggctaccc cacccaagta tacaataaag tcttacctca gaccacaaaa aaaaaaaa |
| 11 | NP_000876.3 Exemplary amino acid sequence for human α4 precursor | 1 mawearrepg prraavretv mlllclgvpt grpynvdtes allyqgphnt lfgysvvlhs 61 hganrwllvg aptanwlana svinpgaiyr crigknpgqt ceqlqlgspn gepcgktcle 121 erdnqwlgvt lsrqpgengs ivtcghrwkn ifyiknenkl ptggcygvpp dlrtelskri 181 apcyqdyvkk fgenfascqa gissfytkdl ivmgapgssy wtgslfvyni ttnkykafld 241 kqnqvkfgsy lgysvgaghf rsqhttevvg gapqheqigk ayifsideke lnilhemkgk 301 klgsyfgasv cavdlnadgf sdllvgapmq stireegrvf vyinsgsgav mnametnlvg 361 sdkyaarfge sivnlgdidn dgfedvaiga pqeddlqgai yiyngradgi sstfsqrieg 421 lqisksl smf gqsisgqida dnngyvdvav gafrsdsavl lrtrpvvivd aslshpesvn 481 rtkfdcveng wpsvcidltl cfsykgkevp gyivlfynms ldvnrkaesp prfyfssngt 541 sdvitgsiqv ssreancrth qafmrkdvrd iltpiqieaa yhlgphvisk rsteefpplq 601 pilqqkkekd imkktinfar fcahencsad lqvsakigfl kphenktyla vgsmktlmln 661 vslfnagdda yettlhvklp vglyfikile leekqincev tdnsgvvqld csigyiyvdh |
| | | 721 lsridisfll dvsslsraee dlsitvhatc eneeemdnlk hsrvtvaipl kyevkltvhg 781 fvnptsfvyg sndenepetc mvekmnltfh vintgnsmap nvsveimvpn sfspqtdklf 841 nildvqtttg echfenyqrv caleqqksam qtlkgivrfl sktdkrllyc ikadphclnf 901 lcnfgkmesg keasvhiqle grpsilemde tsalkfeira tgfpepnprv ielnkdenva 961 hvllleglhhq rpkryftivi isssllglli vllllisyvmw kagffkrqyk silqeenrrd 1021 swsyinsksn dd |
| 12 | NP_000880.1 Exemplary amino acid sequence for human β7 precursor | 1 mvalpmvlvl llvlsrgese ldakipstgd atewrnphls mlgscqpaps cqkcilshps 61 cawckqlnft asgeaearrc arreellarg cpleeleepr gqqevlqdqp lsqgargega 121 tqlapqrvrv tlrpgepqql qvrflraegy pvdlyylmdl sysmkddler vrqlghallv 181 rlqevthsvr igfgsfvdkt vlpfvstvps klrhpcptrl ercqspfsfh hvlsltgdaq 241 aferevgrqs vsgnldspeg gfdailqaal cqeqigwrnv srllvftsdd tfhtagdgkl 301 ggifmpsdgh chldsnglys rstefdypsv gqvaqalsaa niqpifavts aalpvyqels 361 klipksavge lsedssnvvq limdaynsls stvtlehssl ppgvhisyes qcegpekreg 421 kaedrgqcnh vrinqtvtfw vslqathclp ephllrlral gfseelivel htlcdcncsd 481 tqpqaphcsd gqghlqcgvc scapgrlgrl cecsvaelss pdlesgcrap ngtgplcsgk 541 ghcqcgrcsc sgqssghlce cddascerhe gilcggfgrc qcgvchchan rtgracecsg 601 dmdscispeg glcsghgrck cnrcqcldgy ygalcdqcpg cktpcerhrd caecgafrtg 661 platncstac ahtnvtlala pilddgwcke rtldnqlfff lveddargtv vlrvrpqekg 721 adhtqaivlg cvggivavgl glvlayrlsv eiydrreysr fekeqqqlnw kqdsnplyks 781 aitttinprf qeadsptl |
| 13 | NM_016602.2 Exemplary nucleic acid sequence encoding human CCR10 | 1 agagatgggg acggaggcca cagagcaggt ttcctggggc cattactctg gggatgaaga 61 ggacgcatac tcggctgagc cactgccgga gctttgctac aaggccgatg tccaggcctt 121 cagccgggcc ttccaaccca gtgtctccct gaccgtggct gcgctgggtc tggccggcaa 181 tggcctggtc ctggccaccc acctggcagc ccgacgcgca gcgcgctcgc ccacctctgc 241 ccacctgctc cagctggccc tggccgacct cttgctggcc ctgactctgc ccttcgcggc 301 agcaggggct cttcagggct ggagtctggg aagtgccacc tgccgcacca tctctggcct 361 ctactcggcc tccttccacg ccggcttcct cttcctggcc tgtatcagcg ccgaccgcta 421 cgtggccatc gcgcgagcgc tcccagccgg gccgcggccc tccactcccg gccgcgcaca 481 cttggtctcc gtcatcgtgt ggctgctgtc actgctcctg gcgctgcctg cgctgctctt 541 cagccaggat gggcagcggg aaggccaacg acgctgtcgc ctcatcttcc ccgagggcct 601 cacgcagacg gtgaaggggg cgagcgccgt ggcgcaggtg gccctgggct tcgcgctgcc 661 gctgggcgtc atggtagcct gctacgcgct tctgggccgc acgctgctgg ccgccagggg 721 gcccgagcgc cggcgtgcgc tgcgcgtcgt ggtggctctg gtggcggcct tcgtggtgct 781 gcagctgccc tacagcctcg ccctgctgct ggatactgcc gatctactgg ctgcgcgcga 841 gcggagctgc cctgccagca aacgcaagga tgtcgcactg ctggtgacca gcggcttggc 901 cctcgcccgc tgtggcctca atcccgttct ctacgccttc ctgggcctgc gcttccgcca 961 ggacctgcgg aggctgctac ggggtgggag ctgcccctca gggcctcaac cccgccgcgg 1021 ctgcccccgc cggccccgcc tttcttcctg ctcagctccc acggagaccc acagtctctc 1081 ctgggacaac tagggctgcg aatctagagg agggggcagg ctgagggtcg tgggaaaggg 1141 gagtaggtgg gggaacactg agaaagaggc agggacctaa agggactacc tctgtgcctt 1201 gccacattaa attgataaca tggaaatgag atgcaaccca acaa |
| 14 | AF215981.1 Exemplary nucleic | 1 agagatgggg acggaggcca cagagcaggt ttcctggggc cattactctg ggatgaaga 61 ggacgcatac tcggctgagc cactgccgga gctttgctac aaggccgatg tccaggcctt 121 cagccgggcc ttccaaccca gtgtctccct gaccgtggct gcgctgggtc tggccggcaa |
| | acid sequence encoding human CCR10 | 181 tggcctggtc ctggccaccc acctggcagc ccgacgcgca gcgcgctcgc ccacctctgc 241 ccacctgctc cagctggccc tggccgacct cttgctggcc ctgactctgc ccttcgcggc 301 agcaggggct cttcagggct ggagtctggg aagtgccacc tgccgcacca tctctggcct 361 ctactcggcc tccttccacg ccggcttcct cttcctggcc tgtatcagcg ccgaccgcta 421 cgtggccatc gcgcgagcgc tcccagccgg gccgcggccc tccactcccg gccgcgcaca 481 cttggtctcc gtcatcgtgt ggctgctgtc actgctcctg gcgctgcctg cgctgctctt 541 cagccaggat gggcagcggg aaggccaacg acgctgtcgc ctcatcttcc ccgagggcct 601 cacgcagacg gtgaaggggg cgagcgccgt ggcgcaggtg gccctgggct tcgcgctgcc 661 gctgggcgtc atggtagcct gctacgcgct tctgggccgc acgctgctgg ccgccagggg 721 gcccgagcgc cggcgtgcgc tgcgcgtcgt ggtggctctg gtggcggcct tcgtggtgct 781 gcagctgccc tacagcctcg ccctgctgct ggatactgcc gatctactgg ctgcgcgcga 841 gcggagctgc cctgccagca aacgcaagga tgtcgcactg ctggtgacca gcggcttggc 901 cctcgcccgc tgtggcctca atcccgttct ctacgccttc ctgggcctgc gcttccgcca 961 ggacctgcgg aggctgctac ggggtgggag ctcgccctca gggcctcaac cccgccgcgg 1021 ctgcccccgc cggccccgcc tttcttcctg ctcagctccc acggagaccc acagtctctc 1081 ctgggacaac tagggctgcg aatctagagg agggggcagg ctgagggtcg tgggaaaggg 1141 gagtaggtgg gggaacactg agaaagaggc agggacctaa agggactacc tctgtgcctt 1201 gccacattaa attgataaca tggaaatgaa aaaaaaaaaa aaaa |
| 15 | NP_057686.2 Exemplary amino acid sequence for human CCR10 precursor | 1 mgteateqvs wghysgdeed aysaeplpel cykadvqafs rafqpsvslt vaalglagng 61 lvlathlaar raarsptsah llqlaladll laltlpfaaa galqgwslgs atcrtisgly 121 sasfhagflf lacisadryv aiaralpagp rpstpgrahl vsvivwllsl llalpallfs 181 qdgqregqrr crlifpeglt qtvkgasava qvalgfalpl gvmvacyall grtllaargp 241 errralrvvv alvaafvvlq lpyslallld tadllaarer scpaskrkdv allvtsglal 301 arcglnpvly aflglrfrqd lrrllrggsc psgpqprrgc prrprlsscs aptethslsw 361 dn |
| 16 | P46092.3 Exemplary amino acid sequence for human CCR10 precursor | 1 mgteateqvs wghysgdeed aysaeplpel cykadvqafs rafqpsvslt vaalglagng 61 lvlathlaar raarsptsah llqlaladll laltlpfaaa galqgwslgs atcrtisgly 121 sasfhagflf lacisadryv aiaralpagp rpstpgrahl vsvivwllsl llalpallfs 181 qdgqregqrr crlifpeglt qtvkgasava qvalgfalpl gvmvacyall grtllaargp 241 errralrvvv alvaafvvlq lpyslallld tadllaarer scpaskrkdv allvtsglal 301 arcglnpvly aflglrfrqd lrrllrggsc psgpqprrgc prrprlsscs aptethslsw 361 dn |
| 17 | NM_ 005201.3 Exemplary nucleic acid sequence encoding human CCR8 | 1 tttgtagtgg gaggatacct ccagagaggc tgctgctcat tgagctgcac tcacatgagg 61 atacagactt tgtgaagaag gaattggcaa cactgaaacc tccagaacaa aggctgtcac 121 taaggtcccg ctgccttgat ggattataca cttgacctca gtgtgacaac agtgaccgac 181 tactactacc ctgatatctt ctcaagcccc tgtgatgcgg aacttattca gacaaatggc 241 aagttgctcc ttgctgtctt ttattgcctc ctgtttgtat tcagtcttct gggaaacagc 301 ctggtcatcc tggtccttgt ggtctgcaag aagctgagga gcatcacaga tgtatacctc 361 ttgaacctgg ccctgtctga cctgcttttt gtcttctcct tcccctttca gacctactat 421 ctgctggacc agtgggtgtt tgggactgta atgtgcaaag tggtgtctgg cttttattac 481 attggcttct acagcagcat gtttttcatc accctcatga gtgtggacag gtacctggct 541 gttgtccatg ccgtgtatgc cctaaaggtg aggacgatca ggatgggcac aacgctgtgc 601 ctggcagtat ggctaaccgc cattatggct accatcccat tgctagtgtt ttaccaagtg 661 gcctctgaag atggtgttct acagtgttat tcattttaca atcaacagac tttgaagtgg |
| | | 721 aagatcttca ccaacttcaa aatgaacatt ttaggcttgt tgatcccatt caccatcttt 781 atgttctgct acattaaaat cctgcaccag ctgaagaggt gtcaaaacca caacaagacc 841 aaggccatca ggttggtgct cattgtggtc attgcatctt tacttttctg ggtcccattc 901 aacgtggttc ttttcctcac ttccttgcac agtatgcaca tcttggatgg atgtagcata 961 agccaacagc tgacttatgc cacccatgtc acagaaatca tttcctttac tcactgctgt 1021 gtgaaccctg ttatctatgc ttttgttggg gagaagttca agaaacacct ctcagaaata 1081 tttcagaaaa gttgcagcca aatcttcaac tacctaggaa gacaaatgcc tagggagagc 1141 tgtgaaaagt catcatcctg ccagcagcac tcctcccgtt cctccagcgt agactacatt 1201 ttgtgaggat caatgaagac taaatataaa aaacattttc ttgaatggca tgctagtagc 1261 agtgagcaaa ggtgtgggtg tgaaaggttt ccaaaaaaag ttcagcatga aggatgccat 1321 atatgttgtt gccaacactt ggaacacaat gactaaagac atagttgtgc atgcctggca 1381 caacatcaag cctgtgattg tgtttattga tgatgttgaa caagtggtaa ctttaaagga 1441 ttctgtatgc caagtgaaaa aaaaagatgt ctgacctcct tacatat |
| 18 | BC107159.1 Exemplary nucleic acid sequence encoding human CCR8 | 1 ctttgtgaag aaggaattgg caacactgaa acctccagaa caaaggctgt cactaaggtc 61 ccgctgcctt gatggattat acacttgacc tcagtgtgac aacagtgacc gactactact 121 accctgatat cttctcaagc ccctgtgatg cggaacttat tcagacaaat ggcaagttgc 181 tccttgctgt cttttattgc ctcctgtttg tattcagtct tctgggaaac agcctggtca 241 tcctggtcct tgtggtctgc aagaagctga ggagcatcac agatgtatac ctcttgaacc 301 tggccctgtc tgacctgctt tttgtcttct ccttcccctt tcagacctac tatctgctgg 361 accagtgggt gtttgggact gtaatgtgca aagtggtgtc tggcttttat tacattggct 421 tctacagcag catgtttttc atcaccctca tgagtgtgga caggtacctg gctgttgtcc 481 atgccgtgta tgccctaaag gtgaggacga tcaggatggg cacaacgctg tgcctggcag 541 tatggctaac cgccattatg gctaccatcc cattgctagt gttttaccaa gtggcctctg 601 aagatggtgt tctacagtgt tattcatttt acaatcaaca gactttgaag tggaagatct 661 tcaccaactt caaaatgaac attttaggct tgttgatccc attcaccatc tttatgttct 721 gctacattaa aatcctgcac cagctgaaga ggtgtcaaaa ccacaacaag accaaggcca 781 tcaggttggt gctcattgtg gtcattgcat ctttactttt ctgggtccca ttcaacgtgg 841 ttcttttcct cacttccttg cacagtatgc acatcttgga tggatgtagc ataagccaac 901 agctgactta tgccacccat gtcacagaaa tcatttcctt tactcactgc tgtgtgaacc 961 ctgttatcta tgcttttgtt ggggagaagt tcaagaaaca cctctcagaa atatttcaga 1021 aaagttgcag ccaaatcttc aactacctag gaagacaaat gcctagggag agctgtgaaa 1081 agtcatcatc ctgccagcag cactcctccc gttcctccag cgtagactac attttgtgag 1141 gatcaatgaa gactaaatat aaaaaacatt ttcttgaatg gcatgctagt agcagtgagc 1201 aaaggtgtgg gtgtgaaagg tttccaaaaa aagttcagca tgaaggatgc cgtgtgtgtt 1261 gttgccaaca cttggaacac gatgactggg gacgtggttg tgcatgcctg gcacaacatc 1321 aagcctgtga ttgtgtttat tgatgatgtt gaacaagtgg tggctttgga ggattctgta 1381 tgccaagtga aaggggagat gtctgacctc cttcatatag |
| 19 | NP_005192.1 Exemplary amino acid sequence for human CCR8 precursor | 1 mdytldlsvt tvtdyyypdi fsspcdaeli qtngklllav fycllfvfsl lgnslvilvl 61 vvckklrsit dvyllnlals dllfvfsfpf qtyylldqwv fgtvmckvvs gfyyigfyss 121 mffitlmsvd rylavvhavy alkvrtirmg ttlclavwlt aimatipllv fyqvasedgv 181 lqcysfynqq tlkwkiftnf kmnilgllip ftifmfcyik ilhqlkrcqn hnktkairlv 241 livviasllf wvpfnvvlfl tslhsmhild gcsisqqlty athvteiisf thccvnpviy 301 afvgekfkkh lseifqkscs qifnylgrqm presceksss cqqhssrsss vdyil |
| 20 | AAI07160.1 | 1 mdytldlsvt tvtdyyypdi fsspcdaeli qtngklllav fycllfvfsl lgnslvilvl |
| | Exemplary amino acid sequence for human CCR8 precursor | 61 vvckklrsit dvyllnlals dllfvfsfpf qtyylldqwv fgtvmckvvs gfyyigfyss 121 mffitlmsvd rylavvhavy alkvrtirmg ttlclavwlt aimatipllv fyqvasedgv 181 lqcysfynqq tlkwkiftnf kmnilgllip ftifmfcyik ilhqlkrcqn hnktkairlv 241 livviasllf wvpfnvvlfl tslhsmhild gcsisqqlty athvteiisf thccvnpviy 301 afvgekfkkh lseifqkscs qifnylgrqm presceksss cqqhssrsss vdyil |
| 21 | NM_005508.4 Exemplary nucleic acid sequence encoding human CCR4 | 1 gctcacagga agccacgcac ccttgaaagg caccgggtcc ttcttagcat cgtgcttcct 61 gagcaagcct ggcattgcct cacagacctt cctcagagcc gctttcagaa aagcaagctg 121 cttctggttg ggcccagacc tgccttgagg agcctgtaga gttaaaaaat gaaccccacg 181 gatatagcag acaccaccct cgatgaaagc atatacagca attactatct gtatgaaagt 241 atccccaagc cttgcaccaa agaaggcatc aaggcatttg gggagctctt cctgccccca 301 ctgtattcct tggtttttgt atttggtctg cttggaaatt ctgtggtggt tctggtcctg 361 ttcaaataca agcggctcag gtccatgact gatgtgtacc tgctcaacct tgccatctcg 421 gatctgctct tcgtgttttc cctccctttt tggggctact atgcagcaga ccagtgggtt 481 tttgggctag gtctgtgcaa gatgatttcc tggatgtact tggtgggctt ttacagtggc 541 atattctttg tcatgctcat gagcattgat agatacctgg caattgtgca cgcggtgttt 601 tccttgaggg caaggacctt gacttatggg gtcatcacca gtttggctac atggtcagtg 661 gctgtgttcg cctcccttcc tggctttctg ttcagcactt gttatactga gcgcaaccat 721 acctactgca aaaccaagta ctctctcaac tccacgacgt ggaaggttct cagctccctg 781 gaaatcaaca ttctcggatt ggtgatcccc ttagggatca tgctgttttg ctactccatg 841 atcatcagga ccttgcagca ttgtaaaaat gagaagaaga acaaggcggt gaagatgatc 901 tttgccgtgg tggtcctctt ccttgggttc tggacacctt acaacatagt gctcttccta 961 gagaccctgg tggagctaga agtccttcag gactgcacct ttgaaagata cttggactat 1021 gccatccagg ccacagaaac tctggctttt gttcactgct gccttaatcc catcatctac 1081 ttttttctgg gggagaaatt tcgcaagtac atcctacagc tcttcaaaac ctgcaggggc 1141 ctttttgtgc tctgccaata ctgtgggctc ctccaaattt actctgctga cacccccagc 1201 tcatcttaca cgcagtccac catggatcat gatctccatg atgctctgta gaaaaatgaa 1261 atggtgaaat gcagagtcaa tgaactttcc acattcagag cttacttaaa attgtatttt 1321 agtaagagat tcctgagcca gtgtcaggag gaaggcttac acccacagtg gaaagacagc 1381 ttctcatcct gcaggcagct ttttctctcc cactagacaa gtccagcctg gcaagggttc 1441 acctgggctg aggcatcctt cctcacacca ggcttgcctg caggcatgag tcagtctgat 1501 gagaactctg agcagtgctt gaatgaagtt gtaggtaata ttgcaaggca aagactattc 1561 ccttctaacc tgaactgatg ggtttctcca gagggaattg cagagtactg gctgatggag 1621 taaatcgcta ccttttgctg tggcaaatgg gccctct |
| 22 | P51679.1 Exemplary amino acid sequence for human CCR4 precursor | 1 mnptdiadtt ldesiysnyy lyesipkpct kegikafgel flpplyslvf vfgllgnsvv 61 vlvlfkykrl rsmtdvylln laisdllfvf slpfwgyyaa dqwvfglglc kmiswmylvg 121 fysgiffvml msidrylaiv havfslrart ltygvitsla twsvavfasl pgflfstcyt 181 ernhtyckt k yslnsttwkv lssleinilg lviplgimlf cysmiirtlq hcknekknka 241 vkmifavvvl flgfwtpyni vlfletlvel evlqdctfer yldyaiqate tlafvhccln 301 piiyfflgek frkyilqlfk tcrglfvlcq ycgllqiysa dtpsssytqs tmdhdlhdal |
| 23 | NM_001206609.1 Exemplary nucleic acid sequence encoding human | 1 aatcatccga gaaccttgga gggtggacag tgcccctttt acagatgaga aaactgaggc 61 ttgaagggga gaagcagctg cctctggcgg catggcttct ggctgcagga tgcccatgga 121 gttcgtggtg accctaggcc tgtgtctcgg cttcctttgc tgaacttgaa caggaagatg 181 gcagtggggg ccagtggtct agaaggagat aagatggctg gtgccatgcc tctgcaactc 241 ctcctgttgc tgatcctact gggccctggc aacagcttgc agctgtggga cacctggcca |
| | CLA | 301 gatgaagccg agaaagcctt gggtcccctg cttgcccggg accggagaca ggccaccgaa 361 tatgagtacc tagattatga tttcctgcca gaaacggagc ctccagaaat gctgaggaac 421 agcactgaca ccactcctct gactgggcct ggaacccctg agtctaccac tgtggagcct 481 gctgcaaggc gttctactgg cctggatgca ggaggggcag tcacagagct gaccacggag 541 ctggccaaca tggggaacct gtccacggat tcagcagcta tggagataca gaccactcaa 601 ccagcagcca cggaggcaca gaccactcaa ccagtgccca cggaggcaca gaccactcca 661 ctggcagcca cagaggcaca gacaactcga ctgacggcca cggaggcaca gaccactcca 721 ctggcagcca cagaggcaca gaccactcca ccagcagcca cggaagcaca gaccactcaa 781 cccacaggcc tggaggcaca gaccactgca ccagcagcca tggaggcaca gaccactgca 841 ccagcagcca tggaagcaca gaccactcca ccagcagcca tggaggcaca gaccactcaa 901 accacagcca tggaggcaca gaccactgca ccagaagcca cggaggcaca gaccactcaa 961 cccacagcca cggaggcaca gaccactcca ctggcagcca tggaggccct gtccacagaa 1021 cccagtgcca cagaggccct gtccatggaa cctactacca aaagaggtct gttcataccc 1081 ttttctgtgt cctctgttac tcacaagggc attcccatgg cagccagcaa tttgtccgtc 1141 aactacccag tgggggcccc agaccacatc tctgtgaagc agtgcctgct ggccatccta 1201 atcttggcgc tggtggccac tatcttcttc gtgtgcactg tggtgctggc ggtccgcctc 1261 tcccgcaagg gccacatgta ccccgtgcgt aattactccc ccaccgagat ggtctgcatc 1321 tcatccctgt tgcctgatgg gggtgagggg ccctctgcca cagccaatgg gggcctgtcc 1381 aaggccaaga gcccgggcct gacgccagag cccagggagg accgtgaggg ggatgacctc 1441 accctgcaca gcttcctccc ttagctcact ctgccatctg ttttggcaag accccacctc 1501 cacgggctct cctgggccac ccctgagtgc ccagacccca ttccacagct ctgggcttcc 1561 tcggagaccc ctggggatgg ggatcttcag ggaaggaact ctggccaccc aaacaggaca 1621 agagcagcct ggggccaagc agacgggcaa gtggagccac ctctttcctc cctccgcgga 1681 tgaagcccag ccacatttca gccgaggtcc aaggcaggag gccatttact tgagacagat 1741 tctctccttt ttcctgtccc ccatcttctc tgggtccctc taacatctcc catggctctc 1801 cccgcttctc ctggtcactg gagtctcctc cccatgtacc caaggaagat ggagctcccc 1861 catcccacac gcactgcact gccattgtct tttggttgcc atggtcacca aacaggaagt 1921 ggacattcta agggaggagt actgaagagt gacggacttc tgaggctgtt tcctgctgct 1981 cctctgactt ggggcagctt gggtcttctt gggcacctct ctgggaaaac ccagggtgag 2041 gttcagcctg tgagggctgg gatgggtttc gtgggcccaa gggcagacct ttctttggga 2101 ctgtgtggac caaggagctt ccatctagtg acaagtgacc cccagctatc gcctcttgcc 2161 ttcccctgtg gccactttcc agggtggact ctgtcttgtt cactgcagta tcccaactgc 2221 aggtccagtg caggcaataa atatgtgatg gacaaacgat agcggaatcc ttcaaggttt 2281 caaggctgtc tccttcaggc agccttcccg gaattctcca tccctcagtg caggatgggg 2341 gctggtcctc agctgtctgc cctcagcccc tggcccccca ggaagcctct ttcatgggct 2401 gttaggttga cttcagtttt gcctcttgga caacaggggg tcttgtacat ccttgggtga 2461 ccaggaaaag ttcaggctat ggggggccaa agggagggct gccccttccc caccagtgac 2521 cactttattc cacttcctcc attacccagt tttggcccac agagtttggt cccccccaaa 2581 cctcggacca atatccctct aaacatcaat ctatcctcct gttaaagaaa aaaaaaaa |
| 24 | NM_003006.4 Exemplary nucleic acid sequence encoding human CLA | 1 acacacagcc attgggggtt gctcggatcc gggactgccg cagggggtgc cacagcagtg 61 cctggcagcg tgggctggga ccttgtcact aaagcagaga agccacttct tctgggccca 121 cgaggcagct gtcccatgct ctgctgagca cggtggtgcc atgcctctgc aactcctcct 181 gttgctgatc ctactgggcc ctggcaacag cttgcagctg tgggacacct gggcagatga 241 agccgagaaa gccttgggtc ccctgcttgc ccgggaccgg agacaggcca ccgaatatga 301 gtacctagat tatgatttcc tgccagaaac ggagcctcca gaaatgctga ggaacagcac 361 tgacaccact cctctgactg ggcctggaac ccctgagtct accactgtgg agcctgctgc |
| | | 421 aaggcgttct actggcctgg atgcaggagg ggcagtcaca gagctgacca cggagctggc 481 caacatgggg aacctgtcca cggattcagc agctatggag atacagacca ctcaaccagc 541 agccacggag gcacagacca ctcaaccagt gcccacggag gcacagacca ctccactggc 601 agccacagag gcacagacaa ctcgactgac ggccacggag gcacagacca ctccactggc 661 agccacagag gcacagacca ctccaccagc agccacggaa gcacagacca ctcaacccac 721 aggcctggag gcacagacca ctgcaccagc agccatggag gcacagacca ctgcaccagc 781 agccatggaa gcacagacca ctccaccagc agccatggag gcacagacca ctcaaaccac 841 agccatggag gcacagacca ctgcaccaga agccacggag gcacagacca ctcaacccac 901 agccacggag gcacagacca ctccactggc agccatggag gccctgtcca cagaacccag 961 tgccacagag gccctgtcca tggaacctac taccaaaaga ggtctgttca taccctttc 1021 tgtgtcctct gttactcaca agggcattcc catggcagcc agcaatttgt ccgtcaacta 1081 cccagtgggg gccccagacc acatctctgt gaagcagtgc ctgctggcca tcctaatctt 1141 ggcgctggtg gccactatct tcttcgtgtg cactgtggtg ctggcggtcc gcctctcccg 1201 caagggccac atgtaccccg tgcgtaatta ctcccccacc gagatggtct gcatctcatc 1261 cctgttgcct gatgggggtg aggggccctc tgccacagcc aatgggggcc tgtccaaggc 1321 caagagcccg ggcctgacgc cagagcccag ggaggaccgt gagggggatg acctcaccct 1381 gcacagcttc ctcccttagc tcactctgcc atctgttttg gcaagacccc acctccacgg 1441 gctctcctgg gccacccctg agtgcccaga ccccattcca cagctctggg cttcctcgga 1501 gacccctggg gatggggatc ttcagggaag gaactctggc cacccaaaca ggacaagagc 1561 agcctggggc caagcagacg ggcaagtgga gccacctctt tcctccctcc gcggatgaag 1621 cccagccaca tttcagccga ggtccaaggc aggaggccat ttacttgaga cagattctct 1681 cctttttcct gtcccccatc ttctctgggt ccctctaaca tctcccatgg ctctccccgc 1741 ttctcctggt cactggagtc tcctccccat gtacccaagg aagatggagc tcccccatcc 1801 cacacgcact gcactgccat tgtcttttgg ttgccatggt caccaaacag gaagtggaca 1861 ttctaaggga ggagtactga agagtgacgg acttctgagg ctgtttcctg ctgctcctct 1921 gacttggggc agcttgggtc ttcttggcca cctctctggg aaaacccagg gtgaggttca 1981 gcctgtgagg gctgggatgg gtttcgtggg cccaagggca gacctttctt tgggactgtg 2041 tggaccaagg agcttccatc tagtgacaag tgacccccag ctatcgcctc ttgccttccc 2101 ctgtggccac tttccagggt ggactctgtc ttgttcactg cagtatccca actgcaggtc 2161 cagtgcaggc aataaatatg tgatggacaa acgatagcgg aatccttcaa ggtttcaagg 2221 ctgtctcctt caggcagcct tcccggaatt ctccatccct cagtgcagga tgggggctgg 2281 tcctcagctg tctgccctca gcccctggcc ccccaggaag cctctttcat gggctgttag 2341 gttgacttca gttttgcctc ttggacaaca gggggtcttg tacatccttg ggtgaccagg 2401 aaaagttcag gctatggggg gccaaaggga gggctgcccc ttccccacca gtgaccactt 2461 tattccactt cctccattac ccagttttgg cccacagagt ttggtccccc ccaaacctcg 2521 gaccaatatc cctctaaaca tcaatctatc ctcctgttaa agaaaaaaaa aaa |
| 25 | NP_001193538.1 Exemplary amino acid sequence for human CLA precursor | 1 mavgasgleg dkmagamplq lllllillgp gnslqlwdtw adeaekalgp llardrrqat 61 eyeyldydfl peteppemlr nstdttpltg pgtpesttve paarrstgld aggavteltt 121 elanmgnlst dsaameiqtt qpaateaqtt qpvpteaqtt plaateaqtt rltateaqtt 181 plaateaqtt ppaateaqtt qptgleaqtt apaameaqtt apaameaqtt ppaameaqtt 241 qttameaqtt apeateaqtt qptateaqtt plaamealst epsatealsm epttkrglfi 301 pfsvssvthk gipmaasnls vnypvgapdh isvkqcllai lilalvatif fvctvvlavr 361 lsrkghmypv rnysptemvc issllpdgge gpsatanggl skakspgltp epredregdd 421 ltlhsflp |
| 26 | NP_002997.2 | 1 mplqllllli llgpgnslql wdtwadeaek algpllardr rqateyeyld ydflpetepp |
| | Exemplary amino acid sequence for human CLA precursor | 61 emlrnstdtt pltgpgtpes ttvepaarrs tgldaggavt elttelanmg nlstdsaame 121 iqttqpaate aqttqpvpte aqttplaate aqttrltate aqttplaate aqttppaate 181 aqttqptgle aqttapaame aqttapaame aqttppaame aqttqttame aqttapeate 241 aqttqptate aqttplaame alstepsate alsmepttkr glfipfsvss vthkgipmaa 301 snlsvnypvg apdhisvkqc llaililalv atiffvctvv lavrlsrkgh mypvrnyspt 361 emvcissllp dggegpsata ngglskaksp gltpepredr egddltlhsf lp |

### 4.4. Chimeric Antigen Receptors

When the cells provided herein are T lymphocytes which comprise homing receptors, as described above, such T lymphocytes can, in certain embodiments, comprise chimeric antigen receptors (CARs), which are artificial membrane-bound proteins that direct a T lymphocyte to an antigen, and stimulate the T lymphocyte to kill a cell displaying the antigen. See, e.g., Eshhar, U.S. Patent No. 7,741,465. At a minimum, the CAR comprises an extracellular domain that binds to an antigen, e.g., an antigen on a cell, a transmembrane domain, and an intracellular (cytoplasmic) signaling domain that transmits a primary activation signal to an immune cell. All other conditions being satisfied, when the CAR is expressed on the surface of, e.g., a T lymphocyte, for example, a primary T lymphocyte, and the extracellular domain of the CAR binds to an antigen, the intracellular signaling domain transmits a signal to the T lymphocyte to activate and/or proliferate, and, if the antigen is present on a cell surface, to kill the cell expressing the antigen. Because T lymphocytes require two signals, a primary activation signal and a costimulatory signal, in order to maximally activate, typically CARs also comprise a costimulatory domain such that binding of the antigen to the extracellular domain results in transmission of both a primary activation signal and a costimulatory signal.

### 4.4.1. General CAR Structure Intracellular Domain

In certain embodiments, the intracellular domain of the CAR is or comprises an intracellular domain or motif of a protein that is expressed on the surface of T lymphocytes and triggers activation and/or proliferation of said T lymphocytes. Such a domain or motif is able to transmit a primary antigen-binding signal that is necessary for the activation of a T lymphocyte in response to the antigen's binding to the CAR's extracellular portion. Typically, this domain or motif comprises, or is, an ITAM (immunoreceptor tyrosine-based activation motif). ITAM-containing polypeptides suitable for CARs include, for example, the zeta CD3 chain (CD3ζ) or ITAM-containing portions thereof. In a specific embodiment, the intracellular domain is a CD3ζ intracellular signaling domain. In other specific embodiments, the intracellular domain is from a lymphocyte receptor chain, a TCR/CD3 complex protein, an Fc receptor subunit or an IL-2 receptor subunit.

In certain embodiments, the CAR additionally comprises one or more co-stimulatory domains or motifs, e.g., as part of the intracellular domain of the polypeptide. The one or more co-stimulatory domains or motifs can be, or comprise, one or more of a co-stimulatory CD27 polypeptide sequence, a co-stimulatory CD28 polypeptide sequence, a co-stimulatory OX40 (CD134) polypeptide sequence, a co-stimulatory 4-1BB (CD137) polypeptide sequence, or a co-stimulatory inducible T-cell costimulatory (ICOS) polypeptide sequence, or other costimulatory domain or motif.

The transmembrane region can be any transmembrane region that can be incorporated into a functional CAR, typically a transmembrane region from a CD4 or a CD8 molecule.

### 4.4.2. CAR Extracellular Domain

The extracellular domain of the polypeptide binds to an antigen of interest. In certain embodiments of any of the polypeptides described herein, the extracellular domain comprises a receptor, or a portion of a receptor, that binds to said antigen. The extracellular domain may be, e.g., a receptor, or a portion of a receptor, that binds to said antigen. In certain embodiments, the extracellular domain comprises, or is, an antibody or an antigen-binding portion thereof. In specific embodiments, the extracellular domain comprises, or is, a single-chain Fv domain. The single-chain Fv domain can comprise, for example, a V*_{L}* linked to V*_{H}* by a flexible linker, wherein said V*_{L}* and V*_{H}* are from an antibody that binds said antigen.

The antigen to which the extracellular domain of the polypeptide binds can be any antigen of interest, e.g., can be an antigen on a tumor cell. The tumor cell may be, e.g., a cell in a solid tumor, or a cell of a blood cancer. The antigen can be any antigen that is expressed on a cell of any tumor or cancer type, e.g., cells of a lymphoma, a lung cancer, a breast cancer, a prostate cancer, an adrenocortical carcinoma, a thyroid carcinoma, a nasopharyngeal carcinoma, a melanoma, e.g., a malignant melanoma, a skin carcinoma, a colorectal carcinoma, a desmoid tumor, a desmoplastic small round cell tumor, an endocrine tumor, an Ewing sarcoma, a peripheral primitive neuroectodermal tumor, a solid germ cell tumor, a hepatoblastoma, a neuroblastoma, a non-rhabdomyosarcoma soft tissue sarcoma, an osteosarcoma, a retinoblastoma, a rhabdomyosarcoma, a Wilms tumor, a glioblastoma, a myxoma, a fibroma, a lipoma, or the like. In more specific embodiments, said lymphoma can be chronic lymphocytic leukemia (small lymphocytic lymphoma), B-cell prolymphocytic leukemia, lymphoplasmacytic lymphoma, Waldenstrom macroglobulinemia, splenic marginal zone lymphoma, plasma cell myeloma, plasmacytoma, extranodal marginal zone B cell lymphoma, MALT lymphoma, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma, T lymphocyte prolymphocytic leukemia, T lymphocyte large granular lymphocytic leukemia, aggressive NK cell leukemia, adult T lymphocyte leukemia/lymphoma, extranodal NK/T lymphocyte lymphoma, nasal type, enteropathy-type T lymphocyte lymphoma, hepatosplenic T lymphocyte lymphoma, blastic NK cell lymphoma, mycosis fungoides, Sezary syndrome, primary cutaneous anaplastic large cell lymphoma, lymphomatoid papulosis, angioimmunoblastic T lymphocyte lymphoma, peripheral T lymphocyte lymphoma (unspecified), anaplastic large cell lymphoma, Hodgkin lymphoma, or a non-Hodgkin lymphoma.

In certain embodiments, the antigen is a tumor-associated antigen or a tumor-specific antigen. In various specific embodiments, without limitation, the tumor-associated antigen or tumor-specific antigen is Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD19, CD34, CD45, CD99, CD117, chromogranin, cytokeratin, desmin, glial fibrillary acidic protein (GFAP), gross cystic disease fluid protein (GCDFP-15), HMB-45 antigen, high molecular weight melanoma-associated antigen (HMW-MAA), protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, or an abnormal p53 protein.

In certain embodiments, the TAA or TSA is a cancer/testis (CT) antigen, e.g., BAGE, CAGE, CTAGE, FATE, GAGE, HCA661, HOM-TES-85, MAGEA, MAGEB, MAGEC, NA88, NY-ESO-1, NY-SAR-35, OY-TES-1, SPANXB1, SPA17, SSX, SYCP1, or TPTE.

In certain other embodiments, the TAA or TSA is a carbohydrate or ganglioside, e.g., fuc-GM1, GM2 (oncofetal antigen-immunogenic-1; OFA-I-1); GD2 (OFA-I-2), GM3, GD3, and the like.

In certain other embodiments, the TAA or TSA is alpha-actinin-4, Bage-1, BCR-ABL, Bcr-Abl fusion protein, beta-catenin, CA 125, CA 15-3 (CA 27.29\BCAA), CA 195, CA 242, CA-50, CAM43, Casp-8, cdc27, cdk4, cdkn2a, CEA, coa-1, dek-can fusion protein, EBNA, EF2, Epstein Barr virus antigens, ETV6-AML1 fusion protein, HLA-A2, HLA-A11, hsp70-2, KIAAO205, Mart2, Mum-1, 2, and 3, neo-PAP, myosin class I, OS-9, pml-RARa fusion protein, PTPRK, K-ras, N-ras, triosephosphate isomerase, Gage 3,4,5,6,7, GnTV, Herv-K-mel, Lage-1, NA-88, NY-Eso-1/Lage-2, SP17, SSX-2, TRP2-Int2, gp100 (Pmel 17), tyrosinase, TRP-1, TRP-2, MAGE-1, MAGE-3, RAGE, GAGE-1, GAGE-2, p15(58), RAGE,, SCP-1, Hom/Mel-40, PRAME, p53, H-Ras, HER-2/neu, E2A-PRL, H4-RET, IGH-IGK, MYL-RAR, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, 13-Catenin, Mum-1, p16, TAGE, PSMA, CT7, telomerase, 43-9F, 5T4, 791Tgp72, 13HCG, BCA225, BTAA, CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90, TAAL6, TAG72, TLP, TPS, CD19, CD22, CD27, CD30, CD70, GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), an abnormal ras protein, or an abnormal p53 protein. In another specific embodiment, said tumor-associated antigen or tumor-specific antigen is integrin αvβ3 (CD61), galactin, K-Ras (V-Ki-ras2 Kirsten rat sarcoma viral oncogene), or Ral-B. Other tumor-associated and tumor-specific antigens are known to those in the art.

Antibodies, and scFvs, that bind to TSAs and TAAs are known in the art, as are nucleotide sequences that encode them.

In certain specific embodiments, the antigen is an antigen not considered to be a TSA or a TAA, but which is nevertheless associated with tumor cells, or damage caused by a tumor. In certain embodiments, for example, the antigen is, e.g., a growth factor, cytokine or interleukin, e.g., a growth factor, cytokine, or interleukin associated with angiogenesis or vasculogenesis. Such growth factors, cytokines, or interleukins can include, e.g., vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8). Tumors can also create a hypoxic environment local to the tumor. As such, in other specific embodiments, the antigen is a hypoxia-associated factor, e.g., HIF-1α, HIF-1β, HIF-2α, HIF-2β, HIF-3α, or HIF-3β. Tumors can also cause localized damage to normal tissue, causing the release of molecules known as damage associated molecular pattern molecules (DAMPs; also known as alarmins). In certain other specific embodiments, therefore, the antigen is a DAMP, e.g., a heat shock protein, chromatin-associated protein high mobility group box 1 (HMGB1), S100A8 (MRP8, calgranulin A), S100A9 (MRP14, calgranulin B), serum amyloid A (SAA), or can be a deoxyribonucleic acid, adenosine triphosphate, uric acid, or heparin sulfate.

In a specific embodiment, in which the cancer a gastrointestinal cancer, for example, liver cancer, stomach cancer, esophageal cancer, gallbladder cancer, colorectal cancer, anal cancer, or pancreatic cancer, the antigen is an antigen specific for or associated with a gastrointestinal cancer. In a specific embodiment, T lymphocytes provided herein express a gastrointestinal homing receptor and also express a CAR with an extracellular domain that binds to an antigen associated with a gastrointestinal cancer. In a specific embodiment, the extracellular domain of the CAR binds CEA. In other specific embodiments, the extracellular domain of the CAR binds Her2, CA242, MUC1, CA125, or CA19-9.

In a specific embodiment, in which the cancer is a skin cancer, for example, melanoma, squamous cell carcinoma, or basal cell carcinoma, the antigen is an antigen specific for or associated with a skin cancer. In a specific embodiment, T lymphocytes provided herein express a skin homing receptor and also express a CAR with an extracellular domain that binds to an antigen associated with a skin cancer. In a specific embodiment, the extracellular domain of the CAR binds HMW-MAA. In other specific embodiments, the extracellular domain of the CAR binds Her2, GD2, GD3, CEA, or SPAG9.

In certain embodiments of the polypeptides described herein, the extracellular domain is joined to said transmembrane domain by a linker, spacer or hinge polypeptide sequence, e.g., a sequence from CD28.

### 4.5. Isolated Nucleic Acids

In one embodiment, provided herein are polynucleotide sequences that encode the polypeptides provided herein (e.g., chimeric receptors and homing receptors). The polynucleotides may be contained within any polynucleotide vector suitable for the transformation of immune cells, e.g., T lymphocytes. For example, T lymphocytes may be transformed using synthetic vectors, lentiviral or retroviral vectors, autonomously replicating plasmids, a virus (e.g., a retrovirus, lentivirus, adenovirus, or herpes virus), or the like, containing polynucleotides encoding the first and second polypeptides (e.g., chimeric receptors). Lentiviral vectors suitable for transformation of T lymphocytes include, but are not limited to, e.g., the lentiviral vectors described in U.S. Patent Nos. 5,994,136; 6,165,782; 6,428,953; 7,083,981; and 7,250,299, the disclosures of which are hereby incorporated by reference in their entireties. HIV vectors suitable for transformation of T lymphocytes include, but are not limited to, e.g., the vectors described in U.S. Patent No. 5,665,577, the disclosure of which is hereby incorporated by reference in its entirety.

Nucleic acids useful in the production of the polypeptides provided herein, e.g., within a T lymphocyte, include DNA, RNA, or nucleic acid analogs. Nucleic acid analogs can be modified at the base moiety, sugar moiety, or phosphate backbone, and can include deoxyuridine substitution for deoxythymidine, 5-methyl-2'-deoxycytidine or 5-bromo-2'-deoxycytidine substitution for deoxycytidine. Modifications of the sugar moiety can include modification of the 2' hydroxyl of the ribose sugar to form 2'-O-methyl or 2'-O-allyl sugars. The deoxyribose phosphate backbone can be modified to produce morpholino nucleic acids, in which each base moiety is linked to a six membered, morpholino ring, or peptide nucleic acids, in which the deoxyphosphate backbone is replaced by a pseudopeptide backbone and the four bases are retained. See, for example, Summerton and Weller (1997) Antisense Nucleic Acid Drug Dev. 7:187-195; and Hyrup et al. (1996) Bioorgan. Med. Chain. 4:5-23. In addition, the deoxyphosphate backbone can be replaced with, for example, a phosphorothioate or phosphorodithioate backbone, a phosphoroamidite, or an alkyl phosphotriester backbone.

A nucleic acid encoding a polypeptide provided herein may be introduced into host cells as part of a vector, such as, *e.g.,* an expression vector. In addition, a polypeptide provided herein may be produced by transfecting a host cell with a nucleic acid encoding such a polypeptide, and such nucleic acid may be part of a vector. In a specific embodiment, the vector is an expression vector that is capable of directing the expression of a nucleic acid encoding a polypeptide provided herein. Non-limiting examples of expression vectors include, but are not limited to, plasmids and viral vectors, such as replication defective retroviruses, adenoviruses, adeno-associated viruses, Newcastle disease virus, vaccinia virus and baculoviruses. Standard molecular biology techniques may be used to introduce a nucleic acid encoding a polypeptide provided herein into an expression vector.

An expression vector comprises a nucleic acid encoding a polypeptide provided herein in a form suitable for expression of the nucleic acid in a host cell or non-human subject. In a specific embodiment, an expression vector includes one or more regulatory sequences, selected on the basis of the host cells to be used for expression, which is operably linked to the nucleic acid to be expressed. Within an expression vector, "operably linked" is intended to mean that a nucleic acid of interest is linked to the regulatory sequence(s) in a manner which allows for expression of the nucleic acid (*e.g.,* in an *in vitro* transcription/translation system or in a host cell when the vector is introduced into the host cell). Regulatory sequences include promoters, enhancers and other expression control elements (*e.g*., polyadenylation signals). Regulatory sequences include those which direct constitutive expression of a nucleic acid in many types of host cells, those which direct expression of the nucleic acid only in certain host cells (*e.g.,* tissue-specific regulatory sequences), and those which direct the expression of the nucleic acid upon stimulation with a particular agent (e.g., inducible regulatory sequences). It will be appreciated by those skilled in the art that the design of the expression vector can depend on such factors as, *e.g.*, the choice of the host cell to be transformed, the level of expression of protein desired, etc.

An expression vector can be introduced into host cells via conventional transformation or transfection techniques. Such techniques include, but are not limited to, calcium phosphate or calcium chloride co-precipitation, DEAE-dextran-mediated transfection, lipofection, and electroporation. Suitable methods for transforming or transfecting host cells can be found in Sambrook et al., 1989, Molecular Cloning - A Laboratory Manual, 2nd Edition, Cold Spring Harbor Press, New York, and other laboratory manuals. In certain embodiments, a host cell is transiently transfected with an expression vector containing a nucleic acid encoding a polypeptide provided herein. In other embodiments, a host cell is stably transfected with an expression vector containing a nucleic acid encoding a polypeptide provided herein.

### 4.6. Cells

Non-limiting examples of cells in which the homing receptors may be used include, but are not limited to, natural killer (NK) cells, dendritic cells (DC), placental stem cells (e.g., the placental stem cells disclosed in U.S. Patent Nos. 7,468,276; 8,057,788 and 8,202,703, the disclosures of which are hereby incorporated by reference in their entireties), mesenchymal-like stem cells from umbilical cord blood, placental blood, peripheral blood, bone marrow, dental pulp, adipose tissue, osteochondral tissue, and the like; embryonic stem cells, embryonic germ cells, neural crest stem cells, neural stem cells, and differentiated cells (e.g., fibroblasts, etc.). The homing receptors may also be used in tumor cell lines, e.g., for animal model experimental purposes.

In a specific embodiment, the cells comprising the polypeptides provided herein are T lymphocytes. The T lymphocytes comprising the polypeptides provided herein may be naive T lymphocytes or MHC-restricted T lymphocytes. In certain embodiments, the T lymphocytes are tumor infiltrating lymphocytes (TILs). In certain embodiments, the T lymphocytes have been isolated from a tumor biopsy, or have been expanded from T lymphocytes isolated from a tumor biopsy. In certain other embodiments, the T lymphocytes have been isolated from, or are expanded from T lymphocytes expanded from, peripheral blood, cord blood, or lymph.

In certain embodiments, the immune cells, e.g., T lymphocytes, used in the present methods are autologous to an individual to whom the T lymphocytes are to be administered. In certain embodiments, the T lymphocytes are allogeneic to an individual to whom the T lymphocytes are to be administered. Where allogeneic T lymphocytes are used to prepare T lymphocytes, it is preferable to select T lymphocytes that will reduce the possibility of graft-versus-host disease (GVHD) in the individual. For example, in certain embodiments, virus-specific T lymphocytes are selected for preparation of T lymphocytes; such lymphocytes will be expected to have a greatly reduced native capacity to bind to, and thus become activated by, any recipient antigens. In certain embodiments, recipient-mediated rejection of allogeneic T lymphocytes can be reduced by co-administration to the host of one or more immunosuppressive agents, e.g., cyclosporine, tacrolimus, sirolimus, cyclophosphamide, or the like.

In one embodiment, T lymphocytes are obtained from an individual, optionally then expanded, and then transformed with a polynucleotide encoding one or more homing receptors, and optionally one or more polynucleotides encoding one or more CAR(s), and optionally then expanded. In another embodiment, T lymphocytes are obtained from an individual, optionally then expanded, and then transformed with a polynucleotide encoding one or more homing receptors, and optionally one or more polynucleotides encoding one or more CAR(s), and optionally then expanded. Cells containing any of the polynucleotide may be selected using one or more selectable markers.

In certain embodiments, any of the T lymphocytes provided herein express or comprise native TCR proteins, e.g., TCR-α and TCR-β that are capable of forming native TCR complexes. In certain other embodiments, either or both of the native genes encoding TCR-α and TCR-β in the T lymphocytes are modified to be non-functional, e.g., a portion or all are deleted, a mutation is inserted, etc.

In certain embodiments, any of the T lymphocytes provided herein are isolated from a tumor lesion, e.g., are tumor-infiltrating lymphocytes; such T lymphocytes are expected to be specific for a TSA or TAA.

T lymphocytes, and T lymphocytes comprising a polypeptide comprising a CD3ζ signaling domain and a CD28 co-stimulatory domain can be expanded using antibodies to CD3 and CD28, e.g., antibodies attached to beads, or to the surface of a cell culture plate; see, e.g., U.S. Patent Nos. 5,948,893; 6,534,055; 6,352,694; 6,692,964; 6,887,466; and 6,905,681.

In certain embodiments, the antigen and/or antibody can exist free in the medium in which the T lymphocytes are cultured, or either or both can be attached to a solid support, e.g., tissue culture plastic surface, beads, or the like.

### 4.7. Methods of Using Cells Comprising Homing Receptors

In one aspect, the cells, e.g., T lymphocytes, provided herein that comprise one or more homing receptors and optionally one or more CARs, as described elsewhere herein, are used to treat an individual having one or more types of cells desired to be targeted by the cells described herein, e.g., one or more types of cells to be killed. In certain embodiments, the cells to be killed are cancer cells, e.g., tumor cells. In specific embodiments, the cancer cells are cells of a solid tumor. In specific embodiments, the cells are cells of a lymphoma, a lung cancer, a breast cancer, a prostate cancer, an adrenocortical carcinoma, a thyroid carcinoma, a nasopharyngeal carcinoma, a melanoma, e.g., a malignant melanoma, a skin carcinoma, a colorectal carcinoma, a desmoid tumor, a desmoplastic small round cell tumor, an endocrine tumor, an Ewing sarcoma, a peripheral primitive neuroectodermal tumor, a solid germ cell tumor, a hepatoblastoma, a neuroblastoma, a non-rhabdomyosarcoma soft tissue sarcoma, an osteosarcoma, a retinoblastoma, a rhabdomyosarcoma, a Wilms tumor, a glioblastoma, a myxoma, a fibroma, a lipoma, or the like. In more specific embodiments, said lymphoma can be chronic lymphocytic leukemia (small lymphocytic lymphoma), B-cell prolymphocytic leukemia, lymphoplasmacytic lymphoma, Waldenstrom macroglobulinemia, splenic marginal zone lymphoma, plasma cell myeloma, plasmacytoma, extranodal marginal zone B cell lymphoma, MALT lymphoma, nodal marginal zone B cell lymphoma, follicular lymphoma, mantle cell lymphoma, diffuse large B cell lymphoma, mediastinal (thymic) large B cell lymphoma, intravascular large B cell lymphoma, primary effusion lymphoma, Burkitt's lymphoma, T lymphocyte prolymphocytic leukemia, T lymphocyte large granular lymphocytic leukemia, aggressive NK cell leukemia, adult T lymphocyte leukemia/lymphoma, extranodal NK/T lymphocyte lymphoma, nasal type, enteropathy-type T lymphocyte lymphoma, hepatosplenic T lymphocyte lymphoma, blastic NK cell lymphoma, mycosis fungoides, Sezary syndrome, primary cutaneous anaplastic large cell lymphoma, lymphomatoid papulosis, angioimmunoblastic T lymphocyte lymphoma, peripheral T lymphocyte lymphoma (unspecified), anaplastic large cell lymphoma, Hodgkin lymphoma, or a non-Hodgkin lymphoma.

In certain embodiments, the modified cells, e.g., modified T lymphocytes described herein are administered to a subject in need thereof, such that the combination of homing receptors selected is compatible with the patient population (or subpopulation) in which the cells, e.g., T lymphocytes, have been administered. For example, the combination of homing receptors is chosen based on the type of tumor or cancer present in the patient.

In specific embodiments, T lymphocytes expressing gastrointestinal homing receptors are administered to patients having a gastrointestinal tumor or cancer. In certain embodiments, the gastrointestinal tumor or cancer is esophageal cancer, stomach cancer, liver cancer, gallbladder cancer, pancreatic cancer, colorectal cancer, or anal cancer. In a specific embodiment, said T cells further comprise a CAR with an extracellular domain that binds to an antigen associated with a gastrointestinal tumor or cancer.

In other specific embodiments, T lymphocytes expressing skin homing receptors are administered to patients having a skin tumor or cancer. In certain embodiments, the skin tumor or cancer is melanoma, basal cell carcinoma, or squamous cell carcinoma. In a specific embodiment, said T cells further comprise a CAR with an extracellular domain that binds to an antigen associated with a skin tumor or cancer. In specific embodiments, the T lymphocytes comprising a homing receptor also comprise a CAR with an extracellular domain that binds to an antigen expressed in the tumor or cancer cells of the patient.

Efficacy of the cells, e.g., T lymphocytes, after administration to an individual having a disease or disorder remediable by such cells, e.g., T lymphocytes, e.g., an individual having cancer, can be assessed by one or more criteria, specific to the particular disease or disorder, known to those of ordinary skill in the art, to be indicative of progress of the disease or disorder. Generally, administration of the cells to such an individual is effective when one or more of said criteria detectably, e.g., significantly, moves from a disease state value or range to, or towards, a normal value or range.

In certain embodiments, the cells, e.g., T lymphocytes, are formulated in a pharmaceutically-acceptable solution. In preferred embodiments, the pharmaceutically-acceptable solution is suitable for the delivery of living cells. In specific embodiments, the pharmaceutically-acceptable solution is, for example, saline solution (such as Ringer's solution), gelatins, carbohydrates (e.g., lactose, amylose, starch, or the like), fatty acid esters, hydroxymethylcellulose, or polyvinyl pyrolidine. In more specific embodiments, the pharmaceutically-acceptable solution is sterilized prior to addition of the cells. In other more specific embodiments, the pharmaceutically-acceptable solution may be mixed with auxiliary agents such as lubricants, preservatives, stabilizers, emulsifiers, salts for influencing osmotic pressure, buffers, and coloring. Pharmaceutical carriers suitable for use in formulating the cells are known in the art and are described, for example, in WO 96/05309.

In certain embodiments, the cells, e.g., T lymphocytes, are formulated into individual doses, wherein said individual doses comprise at least, at most, or about 1×10⁴, 5×10⁴, 1×10⁵, 5×10⁵, 1×10⁶, 5×10⁶, 1×10⁷, 5×10⁷, 1×10⁸, 5×10⁸, 1×10⁹, 5×10⁹, 1×10¹⁰, 5×10¹⁰, or 1×10¹¹ T lymphocytes. In certain embodiments, the cells, e.g., T lymphocytes, are formulated into individual doses, wherein said individual does comprise a range from 1×10⁴ to 5×10⁴, 5×10⁴ to 1×10⁵, 1×10⁵ to 5×10⁵, 5×10⁵ to 1×10⁶, 1×10⁶ to 5×10⁶ 5×10⁶ to 1×10⁷, 1×10⁷ to 5×10⁷ 5×10⁷ to 1×10⁸, 1×10⁸ to 5×10⁸, 5×10⁸ to 1×10⁹, 1×10⁹ to 5×10⁹, 5×10⁹ to 1×10¹⁰, 1×10¹⁰ to 5×10¹⁰, or 5×10¹⁰ to 1×10¹¹ T lymphocytes. In certain embodiments, the cells are formulated for intravenous, intra-arterial, parenteral, intramuscular, subcutaneous, intrathecal, or intraocular administration, or administration within a particular organ or tissue.

### 5. EXAMPLES

### 5.1. Example 1: Generation of Murine CXCR5⁺ CAR T Cells and in vivo Study

T lymphocytes are obtained from the spleen of B6 Thy 1.1 mice. A lentiviral construct comprising a chimeric antigen receptor (CAR) comprising humanized mouse anti-human CEA-scFv or anti-human HER2-scFv and mouse co-stimulation intracellular domain and CD3ζ is generated. Validation of the CAR T cells is done via phenotypic characterization and functional evaluation via a mouse CXCL13 chemotaxis assay.

For the *in vivo* assay, the activities of murine CXCR5+ CAR T cells are compared to the activities of CXCR5- CAR T cells and non-transduced T cells. Human CEA (or HER2)-transgenic mice that can spontaneously develop CEA+ gastrointestinal tumors (or HER2+ mammary tumors) are used as subjects. Adoptive transfer of the cells is accomplished with one or more doses by intravenous administration.

To examine the location of adoptively transferred CAR T cells in draining lymph nodes, (mesenteric lymph nodes for CEA transgenic mice or axillary lymph nodes for HER2 transgenic mice), a readout assay is done via draining lymph node immunohistochemistry. Animals are sacrificed and the draining lymph nodes are identified. Lymph nodes are grasped with curved forceps and pulled free of attached tissue. Lymph nodes are embedded in optimal cutting temperature compound and frozen on dry ice, and then stored at -80°C. Cryosections are cut, air-dried, and fixed in acetone. Sections are pre-incubated with rabbit (or goat) serum at room temperature, and subsequently incubated with primary antibodies (rat anti-mouse CD3, CD8, CD4, biotinylated mouse anti-mouse Thy-1.1, biotinylated goat-anti-human IgG (H+L)), and biotinylated rabbit (or goat) anti-rat IgG (mouse adsorbed) followed by avidin-peroxidase (or fluorochrome) conjugate. Then sections are incubated with substrate of peroxidase for color development. The images of immunohistochemistry staining results are acquired using confocal microscopy. Donor T cells are identified via anti-mouse Thy1.1 and CAR T cells are identified with anti-human IgG.

To examine the antigen-specific immune response establishment in the draining lymph nodes, a readout assay is done via flow cytometry of CAR T cells isolated from draining lymph nodes. Phenotypic characterization of surface markers Thy 1.1, anti-human IgG, CXCR5, CD69, and HLA-DR is performed. CD69 is a marker for activated T cells. HLA-DR is a marker for antigen-presenting cells. Functional evaluation is done to assay proliferation, Ki67 expression, and numerization of CAR T cells. Cytokine production is evaluated by intracellular cytokine staining upon stimulation.

To examine tumor killing activity of CAR T cells in a tumor site, tumor volume and survival are assayed. Volumes of tumors are determined *in vivo* by external caliper. The greatest longitudinal diameter (length) and the greatest transverse diameter (width) are determined. Tumor volume based on caliper measurements are calculated by the modified ellipsoidal formula: tumor volume = ½(length × width²).

### 5.2. Example 2: Generation of Human CXCR5⁺ CAR T Cells

T lymphocytes are obtained from human PBMCs. A lentiviral construct comprising a chimeric antigen receptor (CAR) composed of humanized mouse anti-human CEA-scFv or anti-human HER2-scFv and human co-stimulation intracellular domain and CD3ζ is generated and used to transduce the T cells to create a population of CEA-specific CAR T cells. The CAR T cells are further transduced with lentiviral vectors containing nucleic acid sequences encoding human CXCR5; CAR T cells expressing CXCR5 are selected for further study. Validation of the CAR T cells is done via phenotypic characterization and functional evaluation via a human CXCL13 chemotaxis assay.

### 5.3. Example 3: Generation of Murine Gastrointestinal Homing CAR T Cells and in vivo Study

T lymphocytes are obtained from the spleen of mice. CAR T cells are generated using a lentiviral construct, as described above, containing a nucleotide sequence encoding a CAR comprising a humanized mouse anti-human CEA-scFv and mouse co-stimulation intracellular domain and CD3ζ. The gastrointestinal homing receptors are introduced via lentiviral transduction of mouse CCR9 and α4β7 nucleic acid sequences. Retinoic acid treatment is performed during T cell activation. Validation of the CAR T cells is done via phenotypic characterization and functional evaluation via a mouse TECK (thymus expressed chemokine, or CCL25) chemotaxis assay (Binger, et al., 2009, Experimental Cell Research 315:1468-79). The chemotactic response to TECK is assessed using 5 µm pore-size polycarbonate membranes in 96-multiwell format chemotaxis plates. Lower wells containing TECK in deprivation medium are separated from the upper wells by the membrane. The T cells, in deprivation medium, are seeded into the upper wells and incubated at 37°C for 20 hours. Non-migrated cells left on top of the membrane are removed. Migrated cells are fixed, stained, and enumerated microscopically or are enumerated via flow cytometry with counting beads. Negative controls are performed with deprivation medium without chemokine, and positive controls are performed with medium for cell culturing containing 10% FBS.

For an *in vivo* assay, human CEA-transgenic mice that can spontaneously develop CEA+ gastrointestinal tumors are used as subjects. Adoptive transfer of the cells is accomplished with one or more doses by intravenous administration.

To examine the location of adoptively transferred CAR T cells in established gastrointestinal tract tumors, a readout assay is done via gastrointestinal tract immunohistochemistry. Animals are sacrificed and the gastrointestinal tracts are removed. The small intestine is sectioned into 4-5 parts before immersion fixation. Sections are pre-incubated with rabbit (or goat) serum at room temperature, and subsequently incubated with primary antibodies (rat anti-mouse CD3, biotinylated mouse anti-mouse Thy-1.1, biotinylated goat-anti-human IgG (H+L)), and biotinylated rabbit (or goat) anti-rat IgG (mouse adsorbed) followed by avidin-peroxidase (or fluorochrome) conjugate. Then sections are incubated with substrate of peroxidase for color development. The images of immunohistochemistry staining results are acquired using microscopy. Donor T cells are identified via anti-mouse Thy1.1 and CAR T cells are identified with anti-human IgG. qRT-PCR or PCR of gastrointestinal tract for detection of migrated CAR T cells in the tissue is also performed.

To examine the antigen-specific immune response establishment in the gastrointestinal tract, a readout assay is done via flow cytometry of CAR T cells isolated from the gastrointestinal tract. Phenotypic characterization of surface markers Thy 1.1, anti-human IgG, CCR9, α4β7, CD69, and HLA-DR is performed. Functional evaluation is done to assay proliferation, Ki67 expression, and numerization of CAR T cells. Cytokine production is evaluated by intracellular cytokine staining upon stimulation.

To examine tumor killing activity of CAR T cells in a tumor site, tumor volume and survival are assayed.

### 5.4. Example 4: Generation of Human Gastrointestinal Homing CAR T Cells

T lymphocytes are obtained from human PBMCs. Generation of CAR T cells is performed using a CAR comprising humanized mouse anti-human CEA-scFv and mouse co-stimulation intracellular domain and CD3ζ. The CAR T cells are further transduced with lentiviral vectors containing nucleic acid sequences encoding human CCR9 and α4β7; CAR T cells expressing both of these proteins are selected for further study. During subsequent T cell activation, the CAR T cells are contacted with retinoic acid such that the level of the CCR9 and α4β7 are increased as compared to CAR T cells expressing these two proteins, but not contacted with retinoic acid. Validation of the CAR T cells is done via phenotypic characterization and functional evaluation via a human TECK chemotaxis assay.

### 5.5. Example 5: Generation of Mouse Skin Homing CAR T cells and in vivo Study

T lymphocytes are obtained from mouse spleen. Generation of CAR T cells is performed using a CAR comprising humanized mouse anti-human HMW-MAA-scFv and mouse co-stimulation intracellular domain and CD3ζ. The CAR T cell s are further transduced with lentiviral vectors containing nucleic acid sequences encoding human CCR10 or CCR4, and/or CLA; CAR T cells expressing both of these proteins are selected for further study. Validation of the CAR T cells is done via phenotypic characterization and functional evaluation via a mouse CCL27 chemotaxis assay. The chemotactic response to CCL27 is assessed using 5 µm pore-size polycarbonate membranes in 96-multiwell format chemotaxis plates. Lower wells containing CCL27 in deprivation medium are separated from the upper wells by the membrane. The T cells, in deprivation medium, are seeded into the upper wells and incubated at 37°C for 20 hours. Non-migrated cells left on top of the membrane are removed. Migrated cells are fixed, stained, and enumerated microscopically or are enumerated via flow cytometry with counting beads. Negative controls are performed with deprivation medium without chemokine, and positive controls are performed with medium for cell culturing containing 10% FBS.

For the *in vivo* assay, immunodeficient mice engrafted with human melanoma cell line that expresses HMW-MAA are used as subjects. Cells are administered through adoptive transfer with multiple doses of intravenous administration.

To examine the location of adoptively transferred CAR T cells in established melanoma, a readout assay is done via immunohistochemistry. Donor T cells are identified via anti-CD3 and CAR T cells are identified with anti-mouse IgG. qRT-PCR or PCR for detection of migrated CAR T cells in the tissue is also performed.

To examine the antigen-specific immune response establishment in the melanoma, a readout assay is done via flow cytometry of CAR T cells isolated from the melanoma. Phenotypic characterization of surface markers CD3, anti-mouse IgG, CCR10 (or CCR4), CLA, CD69, and HLA-DR is performed. Functional evaluation is done to assay proliferation, Ki67 expression, and numerization of CAR T cells. Cytokine production is evaluated by intracellular cytokine staining upon stimulation.

To examine tumor killing activity of CAR T cells in a tumor site, tumor volume and survival are assayed.

### 5.6. Example 6: Generation of Human Skin Homing CAR T cells

T lymphocytes are obtained from human PBMCs. CAR T cells generation is performed using a CAR composed of humanized mouse anti-human HMW-MAA-scFv and human co-stimulation intracellular domain and CD3ζ. The gastrointestinal homing receptors are introduced via lentiviral transduction of human CCR10 or CCR4, and CLA nucleic acid sequences. Treatment with 1,25(OH)₂VD₃ and IL-12 is performed during T cell activation. Validation of the CAR T cells is done via phenotypic characterization and functional evaluation via a human CCL27 chemotaxis assay, as in Example 5.

### 5.7. Example 7: Treatment of Gastrointestinal Tumor

An individual presents with a gastrointestinal tumor, for example, adenomatous polyposis coli. Testing of tumor cells from the individual determines that the tumor cells express CEA. T lymphocytes are obtained from the individual, transfected with a lentiviral vector comprising a nucleotide sequence that encodes a chimeric antigen receptor (CAR), and transfected with a second lentiviral vector comprising a nucleotide sequence encoding human CCR9 and/or α4β7 nucleic acid sequences. The T lymphocytes are expanded using CD3+CD28-coated beads in the presence of retinoic acid to sufficient numbers for administration. The chimeric receptor comprises an extracellular antigen-binding region that binds to CEA; a transmembrane domain; an intracellular co-stimulatory domain from CD28; and an intracellular CD3ζ domain. The individual is administered between 10⁹ and 10¹⁰ of the T lymphocytes in 200 mL saline solution by intravenous infusion over 30 minutes. The individual is re-assessed for the gastrointestinal tumor stage and spread to lymph nodes, and histology of biopsied gastrointestinal tissue is performed at 30, 60, and 90 days post-administration.

### 5.8. Example 8: Treatment of Melanoma

An individual presents with melanoma. Testing of tumor cells from the individual determines that the tumor cells express HMW-MAA. T lymphocytes are obtained from the individual, transfected with a lentiviral vector comprising a nucleotide sequence that encodes a chimeric antigen receptor (CAR), and transfected with a second lentiviral vector comprising a nucleotide sequence encoding human CCR10 (or CCR4) and CLA nucleic acid sequences. The T lymphocytes are expanded using CD3+CD28-coated beads in the presence of 1,25(OH)₂VD₃ and IL-12 to sufficient numbers for administration. The chimeric receptor comprises an extracellular antigen-binding region that binds to HMW-MAA; a transmembrane domain; an intracellular co-stimulatory domain from CD28; and an intracellular CD3ζ domain. The individual is administered between 10⁹ and 10¹⁰ of the T lymphocytes in 200 mL saline solution by intravenous infusion over 30 minutes. The individual is re-assessed for the melanoma stage and spread to lymph nodes, and histology of biopsied skin tissue is performed at 30, 60, and 90 days post-administration.

### EQUIVALENTS

The present disclosure is not to be limited in scope by the specific embodiments described herein. Indeed, various modifications of the subject matter provided herein, in addition to those described, will become apparent to those skilled in the art from the foregoing description. Such modifications are intended to fall within the scope of the appended claims.

Various publications, patents and patent applications are cited herein, the disclosures of which are incorporated by reference in their entireties.

The T lymphocytes of the invention are for use in the methods disclosed herein
Also disclosed herein are *inter alia* the following items (said items are not claims):
1. A T lymphocyte expressing a homing receptor not normally expressed by the T lymphocyte, and a chimeric antigen receptor (CAR).
2. The T lymphocyte of item 1, wherein the homing receptor is a B cell zone homing receptor.
3. The T lymphocyte of item 2, wherein the B cell zone homing receptor is CXCR5.
4. The T lymphocyte of item 1, wherein the homing receptor is a gastrointestinal homing receptor.
5. The T lymphocyte of item 4, wherein the gastrointestinal homing receptor is α4β7.
6. The T lymphocyte of item 4, wherein the gastrointestinal homing receptor is CCR9.
7. The T lymphocyte of item 4, comprising a second gastrointestinal homing receptor.
8. The T lymphocyte of any one of items 4-7, wherein the T lymphocyte is activated, expanded, or both activated and expanded in the presence of a Vitamin A metabolite, for a time and in an amount sufficient to cause increased expression of one or more gastrointestinal homing receptors.
9. The T lymphocyte of item 8, wherein the Vitamin A metabolite is retinoic acid.
10. The T lymphocyte of item 1, wherein the homing receptor is a skin homing receptor.
11. The T lymphocyte of item 10, wherein the skin homing receptor is CLA.
12. The T lymphocyte of item 10, wherein the skin homing receptor is CCR4.
13. The T lymphocyte of item 10, wherein the skin homing receptor is CCR10.
14. The T lymphocyte of item 10, comprising a second skin homing receptor.
15. The T lymphocyte of item 14, comprising a third skin homing receptor.
16. The T lymphocyte of any one of items 10-15, wherein the T lymphocyte is activated, expanded, or both activated and expanded in the presence of a Vitamin D metabolite for a time and in an amount sufficient to cause increased expression of one or more skin homing receptors.
17. The T lymphocyte of item 16, wherein the Vitamin D metabolite is 1,26-dihydroxycholecalciferol (1,25(OH)₂D₃).
18. The T lymphocyte of any one of items 10-17, wherein the T lymphocyte is activated, expanded, or both activated and expanded in the presence of IL-12 for a time and in an amount sufficient to cause increased expression of one or more skin homing receptors
19. The T lymphocyte of any one of items 4-18, additionally comprising a B cell zone homing receptor.
20. The T lymphocyte of item 19, wherein the B cell zone homing receptor is CXCR5.
21. A method of generating a population of T lymphocytes, wherein a plurality of said T lymphocytes home to the B cell zone of the lymph nodes, said method comprising engineering a population of T lymphocytes to express a B cell zone homing receptor.
22. The method of item 21, wherein the receptor is CXCR5.
23. A method of generating a population of T lymphocytes, wherein a plurality of said T lymphocytes home to the gastrointestinal tract, said method comprising engineering a population of T lymphocytes to express a gastrointestinal homing receptor.
24. The method of item 23, wherein the gastrointestinal homing receptor is α4β7.
25. The method of item 23, wherein the gastrointestinal homing receptor is CCR9.
26. The method of item 23, wherein the population of T lymphocytes is engineered to express a second gastrointestinal homing receptor.
27. The method of any one of items 23-26, the method further comprising a step wherein the population of T lymphocytes is activated, expanded, or both activated and expanded in the presence of a Vitamin A metabolite for a time and in an amount sufficient to cause increased expression of one or more gastrointestinal homing receptors.
28. The method of item 27, wherein the Vitamin A metabolite is retinoic acid.
29. A method of generating a population of T lymphocytes, wherein a plurality of said T lymphocytes home to skin tissue or cells, said method comprising engineering a population of T lymphocytes to express a receptor which homes to skin tissue or cells.
30. The method of item 29, wherein the skin homing receptor CLA.
31. The method of item 29, wherein the skin homing receptor is CCR4.
32. The method of item 29, wherein the skin homing receptor is CCR10.
33. The method of item 29, wherein the population of T lymphocytes is engineered to express a second skin homing receptor.
34. The method of item 33, wherein the population of T lymphocytes is engineered to express a third skin homing receptor.
35. The method of any one of items 29-34, wherein the method further comprises a step wherein the population of T lymphocytes is activated, expanded, or both activated and expanded in the presence of a Vitamin D metabolite for a time and in an amount sufficient to cause increased expression of one or more skin homing receptors.
36. The method of item 35, wherein the Vitamin D metabolite is 1,26-dihydroxycholecalciferol (1,25(OH)₂D₃).
37. The method of any one of items 29-36, wherein the method further comprises a step wherein the population of T lymphocytes is activated, expanded, or both activated and expanded in the presence of IL-12 for a time and in an amount sufficient to cause increased expression of one or more skin homing receptors.
38. The method of any one of items 23-37, wherein the method comprises a step of engineering the population of T lymphocytes to express a B cell zone homing receptor.
39. The method of item 38, wherein the B cell zone homing receptor is CXCR5.
40. The method of any of items 21-39, wherein the method further comprises a step of administering a lentiviral vector encoding a chimeric antigen receptor (CAR).
41. A method of treating a cancer or tumor in an individual comprising administering to an individual in need thereof a T lymphocyte comprising (i) a B cell zone homing receptor; and (ii) a CAR.
42. The method of item 41, wherein the B cell zone homing receptor is CXCR5.
43. A method of treating a gastrointestinal cancer or tumor in an individual comprising administering to an individual in need thereof a T lymphocyte comprising (i) a gastrointestinal homing receptor; and (ii) a CAR.
44. The method of item 43, wherein the gastrointestinal homing receptor is α4β7.
45. The method of item 43, wherein the gastrointestinal homing receptor is CCR9.
46. The method of item 43, wherein the T lymphocyte comprises a second gastrointestinal homing receptor.
47. The method of any one of items 43 to 46, wherein the extracellular domain of the CAR binds an antigen associated with a gastrointestinal tumor or cancer.
48. A method of treating a skin cancer or a skin tumor in an individual comprising administering to an individual in need thereof a T lymphocyte comprising (i) a skin homing receptor; and (ii) a CAR.
49. The method of item 48, wherein the skin homing receptor is CLA.
50. The method of item 48, wherein the skin homing receptor is CCR4.
51. The method of item 48, wherein the skin homing receptor is CCR10.
52. The method of item 48, wherein the T lymphocyte comprises a second skin homing receptor.
53. The method of item 52, wherein the T lymphocyte comprises a third skin homing receptor.
54. The method of any one of items 48 to 53, wherein the extracellular domain of the CAR binds an antigen associated with a skin tumor or cancer.
55. The method of any one of items 41-46 or 48-53, wherein the extracellular domain of the CAR binds an antigen selected from the group consisting of Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD19, CD34, CD45, CD99, CD117, chromogranin, cytokeratin, desmin, glial fibrillary acidic protein (GFAP), gross cystic disease fluid protein (GCDFP-15), HMB-45 antigen, high molecular weight melanoma-associated antigen (HMW-MAA), protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, an abnormal p53 protein, fuc-GM1, GM2 (oncofetal antigen-immunogenic-1; OFA-I-1); GD2 (OFA-I-2), GM3, GD3, alpha-actinin-4, Bage-1, BCR-ABL, Bcr-Abl fusion protein, beta-catenin, CA 15-3 (CA 27.29\BCAA), CA 195, CA 242, CA-50, CAM43, Casp-8, cdc27, cdk4, cdkn2a, coa-1, dek-can fusion protein, EBNA, EF2, Epstein Barr virus antigens, ETV6-AML1 fusion protein, HLA-A2, HLA-A11, hsp70-2, KlAAO205, Mart2, Mum-1, 2, and 3, neo-PAP, myosin class I, OS-9, pml-RARa fusion protein, PTPRK, triosephosphate isomerase, Gage 3,4,5,6,7, GnTV, Herv-K-mel, Lage-1, NA-88, NY-Eso-1/Lage-2, SP17, SSX-2, TRP2-Int2, , gp100 (Pmel 17), tyrosinase, TRP-1, TRP-2, MAGE-1, MAGE-3, RAGE, GAGE-1, GAGE-2, p15(58), RAGE,, SCP-1, Hom/Mel-40, PRAME, HER-2/neu, E2A-PRL, H4-RET, IGH-IGK, MYL-RAR, human papillomavirus (HPV) antigens E6 and E7, TSP-180, p185erbB2, p180erbB-3, c-met, nm-23H1, PSA, TAG-72-4, CA 19-9, CA 72-4, CAM 17.1, NuMa, 13-Catenin, Mum-1, p16, TAGE, PSMA, CT7, telomerase, 43-9F, 5T4, 791Tgp72, 13HCG, BCA225, BTAA, , CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90, TAAL6, TAG72, TLP, TPS, CD19, CD22, CD27, CD30, CD70, EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), integrin αvβ3 (CD61), galactin, and Ral-B.
56. The method of any one of items 43-54, wherein the T lymphocyte further comprises a B cell zone homing receptor.
57. The method of item 56, wherein the B cell zone homing receptor is CXCR5.
58. A T lymphocyte comprising (i) a B cell zone homing receptor; and (ii) a CAR for use in a method of treating a cancer or tumor in an individual comprising administering said T lymphocyte to an individual in need thereof.
59. The T lymphocyte for use according to item 58, wherein the B cell zone homing receptor is CXCR5.
60. A T lymphocyte comprising (i) a gastrointestinal homing receptor; and (ii) a CAR for use in a method of treating a gastrointestinal cancer or tumor in an individual comprising administering said T lymphocyte to an individual in need thereof.
61. The T lymphocyte for use according to item 60, wherein the gastrointestinal homing receptor is α4β7.
62. The T lymphocyte for use according to item 60, wherein the gastrointestinal homing receptor is CCR9.
63. The T lymphocyte for use according to item 60, wherein said T lymphocyte comprises a second gastrointestinal homing receptor.
64. The T lymphocyte for use according to any one of items 60 to 63, wherein the extracellular domain of the CAR binds an antigen associated with a gastrointestinal tumor or cancer.
65. A T lymphocyte comprising (i) a skin homing receptor; and (ii) a CAR for use in a method of treating a skin cancer or a skin tumor in an individual comprising administering said T lymphocyte to an individual in need thereof.
66. The T lymphocyte for use according to item 65, wherein the skin homing receptor is CLA.
67. The T lymphocyte for use according to item 65, wherein the skin homing receptor is CCR4.
68. The T lymphocyte for use according to item 65, wherein the skin homing receptor is CCR10.
69. The T lymphocyte for use according to item 65, wherein said T lymphocyte comprises a second skin homing receptor.
70. The T lymphocyte for use according to item 69, wherein said T lymphocyte comprises a third skin homing receptor.
71. The T lymphocyte for use according to any one of items 65 to 70, wherein the extracellular domain of the CAR binds an antigen associated with a skin tumor or cancer.
72. The T lymphocyte for use according to any one of items 58-63 or 65-70, wherein the extracellular domain of the CAR binds an antigen selected from the group consisting of Her2, prostate stem cell antigen (PSCA), alpha-fetoprotein (AFP), carcinoembryonic antigen (CEA), cancer antigen-125 (CA-125), CA19-9, calretinin, MUC-1, epithelial membrane protein (EMA), epithelial tumor antigen (ETA), tyrosinase, melanoma-associated antigen (MAGE), CD19, CD34, CD45, CD99, CD117, chromogranin, cytokeratin, desmin, glial fibrillary acidic protein (GFAP), gross cystic disease fluid protein (GCDFP-15), HMB-45 antigen, high molecular weight melanoma-associated antigen (HMW-MAA), protein melan-A (melanoma antigen recognized by T lymphocytes; MART-1), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, an abnormal p53 protein, fuc-GM1, GM2 (oncofetal antigen-immunogenic-1; OFA-I-1); GD2 (OFA-I-2), GM3, GD3, alpha-actinin-4, Bage-1, BCR-ABL, Bcr-Abl fusion protein, beta-catenin, CA 15-3 (CA 27.29\BCAA), CA 195, CA 242, CA-50, CAM43, Casp-8, cdc27, cdk4, cdkn2a, coa-1, dek-can fusion protein, EBNA, EF2, Epstein Barr virus antigens, ETV6-AML1 fusion protein, HLA-A2, HLA-A11, hsp70-2, KIAAO205, Mart2, Mum-1, 2, and 3, neo-PAP, myosin class I, OS-9, pml-RARa fusion protein, PTPRK, triosephosphate isomerase, Gage 3,4,5,6,7, GnTV, Herv-K-mel, Lage-1, NA-88, NY-Eso-1/Lage-2, SP17, SSX-2, TRP2-Int2, , gp100 (Pmel 17), tyrosinase, TRP-1, TRP-2, MAGE-1, MAGE-3, RAGE, GAGE-1, GAGE-2, p15(58), RAGE,, SCP-1, Hom/Mel-40, PRAME, HER-2/neu, E2A-PRL, H4-RET, IGH-IGK, MYL-RAR, human papillomavirus (HPV) antigens E6 and E7, TSP-180, p185erbB2, p180erbB-3, c-met, nm-23H1, PSA, TAG-72-4, CA 19-9, CA 72-4, CAM 17.1, NuMa, 13-Catenin, Mum-1, p16, TAGE, PSMA, CT7, telomerase, 43-9F, 5T4, 791Tgp72, 13HCG, BCA225, BTAA, , CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90, TAAL6, TAG72, TLP, TPS, CD19, CD22, CD27, CD30, CD70, EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of the prostate 1), integrin αvβ3 (CD61), galactin, and Ral-B.
73. The T lymphocyte for use according to any one of items 60-71, wherein said T lymphocyte further comprises a B cell zone homing receptor.
74. The T lymphocyte for use according to item 73, wherein the B cell zone homing receptor is CXCR5.

## Claims

1. A T lymphocyte comprising: (i) a skin homing receptor; and (ii) a chimeric antigen receptor (CAR).

2. The T lymphocyte of claim 1, wherein the skin homing receptor is CLA, CCR4, CCR8 or CCR10.

3. The T lymphocyte of claim 1 or 2, wherein the T lymphocyte further comprises a second skin homing receptor; or
wherein the T lymphocyte further comprises a second and a third skin homing receptor; or
wherein the T lymphocyte further comprises a second, a third and a fourth skin homing receptor; preferably wherein the second, the third or the fourth skin homing receptor is CLA, CCR4, CCR8 or CCR10.

4. The T lymphocyte of any one of claims 1 to 3, wherein the T lymphocyte further comprises a B cell zone homing receptor, preferably wherein the B cell zone homing receptor is CXCR5.

5. The T lymphocyte of any one of claims 1 to 4, wherein the extracellular domain of the CAR binds an antigen associated with a skin tumor or cancer; or
wherein the extracellular domain of the CAR binds high molecular weight melanoma-associated antigen (HMW-MAA), Her2, GD2, GD3, carcinoembryonic antigen (CEA) or sperm associated antigen 9 (SPAG9).

6. A T lymphocyte as defined in any one of claims 1 to 5 for use in a method of treating a skin tumor or cancer in an individual.

7. A method of generating a population of T lymphocytes, wherein a plurality of said T lymphocytes home to skin tissue or skin cells; and wherein said method comprises engineering a population of T lymphocytes to express a skin homing receptor, preferably wherein the skin homing receptor is CLA, CCR4, CCR8 or CCR10; optionally
wherein the population of T lymphocytes is engineered to express a second skin homing receptor; or optionally
wherein the population of T lymphocytes is engineered to express a second and a third skin homing receptor; or optionally
wherein the population of T lymphocytes is engineered to express a second, a third and a fourth skin homing receptor; preferably wherein the second, the third or the fourth skin homing receptor is CLA, CCR4, CCR8 or CCR10.

8. The method of claim 7, wherein the method further comprises a step wherein the population of T lymphocytes is activated, expanded, or both activated and expanded in the presence of a Vitamin D metabolite for a time and in an amount sufficient to cause increased expression of one or more skin homing receptors, preferably wherein the Vitamin D metabolite is 1,26- dihydroxycholecalciferol (1,25(OH)₂D₃); and/or
wherein the method further comprises a step wherein the population of T lymphocytes is activated, expanded, or both activated and expanded in the presence of IL-12 for a time and in an amount sufficient to cause increased expression of one or more skin homing receptors; and/or
wherein the method further comprises a step of engineering the population of T lymphocytes to express a B cell zone homing receptor, preferably wherein the B cell zone homing receptor is CXCR5; and/or
wherein the method further comprises a step of administering a lentiviral vector encoding a CAR.

9. A T lymphocyte comprising: (i) a gastrointestinal homing receptor; and (ii) a chimeric antigen receptor (CAR).

10. The T lymphocyte of claim 9, wherein the gastrointestinal homing receptor is α4β7 or CCR9.

11. The T lymphocyte of claim 9 or 10, wherein the T lymphocyte further comprises a second gastrointestinal homing receptor, preferably wherein the second gastrointestinal homing receptor is α4β7 or CCR9; and/or
wherein the T lymphocyte further comprises a B cell zone homing receptor, preferably wherein the B cell zone homing receptor is CXCR5.

12. The T lymphocyte of any one of claims 9 to 11, wherein the extracellular domain of the CAR binds an antigen associated with a gastrointestinal tumor or cancer; or
wherein the extracellular domain of the CAR binds CEA, cancer antigen-125 (CA-125), CA19-9, CD117, CA72-4, Her2, CA242, or MUC1.

13. A T lymphocyte as defined in any one of claims 9 to 12 for use in a method of treating a gastrointestinal tumor or cancer in an individual.

14. A method of generating a population of T lymphocytes, wherein a plurality of said T lymphocytes home to gastrointestinal tract; and wherein said method comprises engineering a population of T lymphocytes to express a gastrointestinal homing receptor, preferably wherein the gastrointestinal homing receptor is α4β7 or CCR9; optionally
wherein the population of T lymphocytes is engineered to express a second gastrointestinal homing receptor, preferably wherein the second gastrointestinal homing receptor is α4β7 or CCR9.

15. The method of claim 14, wherein the method further comprises a step wherein the population of T lymphocytes is activated, expanded, or both activated and expanded in the presence of a Vitamin A metabolite for a time and in an amount sufficient to cause increased expression of one or more gastrointestinal homing receptors, preferably wherein the Vitamin A metabolite is retinoic acid; and/or
wherein the method further comprises a step of engineering the population of T lymphocytes to express a B cell zone homing receptor, preferably wherein the B cell zone homing receptor is CXCR5; and/or
wherein the method further comprises a step of administering a lentiviral vector encoding a CAR.

16. A T lymphocyte comprising: (i) a B cell zone homing receptor; and (ii) a chimeric antigen receptor (CAR), preferably wherein the B cell zone homing receptor is CXCR5.


SEQUENCE LISTING

<110>  CELGENE CORPORATION

<120>  CAR-T LYMPHOCYTES ENGINEERED TO HOME TO LYMPH NODE B CELL ZONE,
       SKIN, OR GASTROINTESTINAL TRACT

<130>  104832PCEPT1

<140>  to be assigned
<141>  2015-08-11

<150>  US 62/036,447
<151>  2014-08-12

<160>  26

<170>  PatentIn version 3.5

<210>  1
<211>  2919
<212>  DNA
<213>  Homo sapiens

<220>
<223>  Exemplary nucleic acid sequence encoding human CXCR5

<300>
<308>  NM_001716.4
<309>  2015-03-15
<313>  (1)..(2919)

<400>  1
aaaaaaaaaa agtgatgagt tgtgaggcag gtcgcggccc tactgcctca ggagacgatg      60

cgcagctcat ttgcttaaat ttgcagctga cggctgccac ctctctagag gcacctggcg     120

gggagcctct caacataaga cagtgaccag tctggtgact cacagccggc acagccatga     180

actacccgct aacgctggaa atggacctcg agaacctgga ggacctgttc tgggaactgg     240

acagattgga caactataac gacacctccc tggtggaaaa tcatctctgc cctgccacag     300

aggggcccct catggcctcc ttcaaggccg tgttcgtgcc cgtggcctac agcctcatct     360

tcctcctggg cgtgatcggc aacgtcctgg tgctggtgat cctggagcgg caccggcaga     420

cacgcagttc cacggagacc ttcctgttcc acctggccgt ggccgacctc ctgctggtct     480

tcatcttgcc ctttgccgtg gccgagggct ctgtgggctg ggtcctgggg accttcctct     540

gcaaaactgt gattgccctg cacaaagtca acttctactg cagcagcctg ctcctggcct     600

gcatcgccgt ggaccgctac ctggccattg tccacgccgt ccatgcctac cgccaccgcc     660

gcctcctctc catccacatc acctgtggga ccatctggct ggtgggcttc ctccttgcct     720

tgccagagat tctcttcgcc aaagtcagcc aaggccatca caacaactcc ctgccacgtt     780

gcaccttctc ccaagagaac caagcagaaa cgcatgcctg gttcacctcc cgattcctct     840

accatgtggc gggattcctg ctgcccatgc tggtgatggg ctggtgctac gtgggggtag     900

tgcacaggtt gcgccaggcc cagcggcgcc ctcagcggca gaaggcagtc agggtggcca 960

tcctggtgac aagcatcttc ttcctctgct ggtcacccta ccacatcgtc atcttcctgg 1020

acaccctggc gaggctgaag gccgtggaca atacctgcaa gctgaatggc tctctccccg 1080

tggccatcac catgtgtgag ttcctgggcc tggcccactg ctgcctcaac cccatgctct 1140

acactttcgc cggcgtgaag ttccgcagtg acctgtcgcg gctcctgacg aagctgggct 1200

gtaccggccc tgcctccctg tgccagctct tccctagctg gcgcaggagc agtctctctg 1260

agtcagagaa tgccacctct ctcaccacgt tctaggtccc agtgtcccct tttattgctg 1320

cttttccttg gggcaggcag tgatgctgga tgctccttcc aacaggagct gggatcctaa 1380

gggctcaccg tggctaagag tgtcctagga gtatcctcat ttggggtagc tagaggaacc 1440

aacccccatt tctagaacat ccctgccagc tcttctgccg gccctggggc taggctggag 1500

cccagggagc ggaaagcagc tcaaaggcac agtgaaggct gtccttaccc atctgcaccc 1560

ccctgggctg agagaacctc acgcacctcc catcctaatc atccaatgct caagaaacaa 1620

cttctacttc tgcccttgcc aacggagagc gcctgcccct cccagaacac actccatcag 1680

cttaggggct gctgacctcc acagcttccc ctctctcctc ctgcccacct gtcaaacaaa 1740

gccagaagct gagcaccagg ggatgagtgg aggttaaggc tgaggaaagg ccagctggca 1800

gcagagtgtg gccttcggac aactcagtcc ctaaaaacac agacattctg ccaggccccc 1860

aagcctgcag tcatcttgac caagcaggaa gctcagactg gttgagttca ggtagctgcc 1920

cctggctctg accgaaacag cgctgggtcc accccatgtc accggatcct gggtggtctg 1980

caggcagggc tgactctagg tgcccttgga ggccagccag tgacctgagg aagcgtgaag 2040

gccgagaagc aagaaagaaa cccgacagag ggaagaaaag agctttcttc ccgaacccca 2100

aggagggaga tggatcaatc aaacccggcg gtcccctccg ccaggcgaga tggggtgggg 2160

tggagaactc ctagggtggc tgggtccagg ggatgggagg ttgtgggcat tgatggggaa 2220

ggaggctggc ttgtcccctc ctcactccct tcccataagc tatagacccg aggaaactca 2280

gagtcggaac ggagaaaggt ggactggaag gggcccgtgg gagtcatctc aaccatcccc 2340

tccgtggcat caccttaggc agggaagtgt aagaaacaca ctgaggcagg gaagtcccca 2400

ggccccagga agccgtgccc tgcccccgtg aggatgtcac tcagatggaa ccgcaggaag 2460

ctgctccgtg cttgtttgct cacctggggt gtgggaggcc cgtccggcag ttctgggtgc 2520

tccctaccac ctccccagcc tttgatcagg tggggagtca gggacccctg cccttgtccc 2580

actcaagcca agcagccaag ctccttggga ggccccactg gggaaataac agctgtggct 2640

cacgtgagag tgtcttcacg gcaggacaac gaggaagccc taagacgtcc cttttttctc 2700

tgagtatctc ctcgcaagct gggtaatcga tgggggagtc tgaagcagat gcaaagaggc 2760

aagaggctgg attttgaatt ttctttttaa taaaaaggca cctataaaac aggtcaatac 2820

agtacaggca gcacagagac ccccggaaca agcctaaaaa ttgtttcaaa ataaaaacca 2880

agaagatgtc ttcacatatt gtaaaaaaaa aaaaaaaaa 2919

<210> 2
<211> 2896
<212> DNA
<213> Homo sapiens

<220>
<223> Exemplary nucleic acid sequence encoding human CXCR5

<300>
<308> NM_032966.2
<309> 2014-05-03
<313> (1)..(2896)

<400> 2
ccactctaag gaatgcggtc cctttgacag gcgaaaaact gaagttggaa aagacaaagt 60

gatttgttca aaattgaaat ttgaaacttg acatttggtc agtgggccct atgtaggaaa 120

aaacctccaa gagagctagg gttcctctca gagaggaaag acaggtcctt aggtcctcac 180

cctcccgtct ccttgccctt gcagttctgg gaactggaca gattggacaa ctataacgac 240

acctccctgg tggaaaatca tctctgccct gccacagagg ggcccctcat ggcctccttc 300

aaggccgtgt tcgtgcccgt ggcctacagc ctcatcttcc tcctgggcgt gatcggcaac 360

gtcctggtgc tggtgatcct ggagcggcac cggcagacac gcagttccac ggagaccttc 420

ctgttccacc tggccgtggc cgacctcctg ctggtcttca tcttgccctt tgccgtggcc 480

gagggctctg tgggctgggt cctggggacc ttcctctgca aaactgtgat tgccctgcac 540

aaagtcaact tctactgcag cagcctgctc ctggcctgca tcgccgtgga ccgctacctg 600

gccattgtcc acgccgtcca tgcctaccgc caccgccgcc tcctctccat ccacatcacc 660

tgtgggacca tctggctggt gggcttcctc cttgccttgc cagagattct cttcgccaaa 720

gtcagccaag gccatcacaa caactccctg ccacgttgca ccttctccca agagaaccaa 780

gcagaaacgc atgcctggtt cacctcccga ttcctctacc atgtggcggg attcctgctg 840

cccatgctgg tgatgggctg gtgctacgtg ggggtagtgc acaggttgcg ccaggcccag 900

cggcgccctc agcggcagaa ggcagtcagg gtggccatcc tggtgacaag catcttcttc 960

ctctgctggt caccctacca catcgtcatc ttcctggaca ccctggcgag gctgaaggcc 1020

gtggacaata cctgcaagct gaatggctct ctccccgtgg ccatcaccat gtgtgagttc 1080

ctgggcctgg cccactgctg cctcaacccc atgctctaca ctttcgccgg cgtgaagttc 1140

cgcagtgacc tgtcgcggct cctgacgaag ctgggctgta ccggccctgc ctccctgtgc 1200

cagctcttcc ctagctggcg caggagcagt ctctctgagt cagagaatgc cacctctctc 1260

accacgttct aggtcccagt gtcccctttt attgctgctt ttccttgggg caggcagtga 1320

tgctggatgc tccttccaac aggagctggg atcctaaggg ctcaccgtgg ctaagagtgt 1380

cctaggagta tcctcatttg gggtagctag aggaaccaac ccccatttct agaacatccc 1440

tgccagctct tctgccggcc ctggggctag gctggagccc agggagcgga aagcagctca 1500

aaggcacagt gaaggctgtc cttacccatc tgcacccccc tgggctgaga gaacctcacg 1560

cacctcccat cctaatcatc caatgctcaa gaaacaactt ctacttctgc ccttgccaac 1620

ggagagcgcc tgcccctccc agaacacact ccatcagctt aggggctgct gacctccaca 1680

gcttcccctc tctcctcctg cccacctgtc aaacaaagcc agaagctgag caccagggga 1740

tgagtggagg ttaaggctga ggaaaggcca gctggcagca gagtgtggcc ttcggacaac 1800

tcagtcccta aaaacacaga cattctgcca ggcccccaag cctgcagtca tcttgaccaa 1860

gcaggaagct cagactggtt gagttcaggt agctgcccct ggctctgacc gaaacagcgc 1920

tgggtccacc ccatgtcacc ggatcctggg tggtctgcag gcagggctga ctctaggtgc 1980

ccttggaggc cagccagtga cctgaggaag cgtgaaggcc gagaagcaag aaagaaaccc 2040

gacagaggga agaaaagagc tttcttcccg aaccccaagg agggagatgg atcaatcaaa 2100

cccggcggtc ccctccgcca ggcgagatgg ggtggggtgg agaactccta gggtggctgg 2160

gtccagggga tgggaggttg tgggcattga tggggaagga ggctggcttg tcccctcctc 2220

actcccttcc cataagctat agacccgagg aaactcagag tcggaacgga gaaaggtgga 2280

ctggaagggg cccgtgggag tcatctcaac catcccctcc gtggcatcac cttaggcagg 2340

gaagtgtaag aaacacactg aggcagggaa gtccccaggc cccaggaagc cgtgccctgc 2400

ccccgtgagg atgtcactca gatggaaccg caggaagctg ctccgtgctt gtttgctcac 2460

ctggggtgtg ggaggcccgt ccggcagttc tgggtgctcc ctaccacctc cccagccttt 2520

gatcaggtgg ggagtcaggg acccctgccc ttgtcccact caagccaagc agccaagctc 2580

cttgggaggc cccactgggg aaataacagc tgtggctcac gtgagagtgt cttcacggca 2640

ggacaacgag gaagccctaa gacgtccctt ttttctctga gtatctcctc gcaagctggg 2700

taatcgatgg gggagtctga agcagatgca aagaggcaag aggctggatt ttgaattttc 2760

tttttaataa aaaggcacct ataaaacagg tcaatacagt acaggcagca cagagacccc 2820

cggaacaagc ctaaaaattg tttcaaaata aaaaccaaga agatgtcttc acatattgta 2880

aaaaaaaaaa aaaaaa 2896

<210> 3
<211> 327
<212> PRT
<213> Homo sapiens

<220>
<223> Exemplary amino acid sequence for human CXCR5 precursor

<300>
<308>  NP_116743.1
<309>  2014-05-03
<313>  (1)..(327)

<400>  3

Met Ala Ser Phe Lys Ala Val Phe Val Pro Val Ala Tyr Ser Leu Ile
1               5                   10                  15

Phe Leu Leu Gly Val Ile Gly Asn Val Leu Val Leu Val Ile Leu Glu
            20                  25                  30

Arg His Arg Gln Thr Arg Ser Ser Thr Glu Thr Phe Leu Phe His Leu
        35                  40                  45

Ala Val Ala Asp Leu Leu Leu Val Phe Ile Leu Pro Phe Ala Val Ala
    50                  55                  60

Glu Gly Ser Val Gly Trp Val Leu Gly Thr Phe Leu Cys Lys Thr Val
65                  70                  75                  80

Ile Ala Leu His Lys Val Asn Phe Tyr Cys Ser Ser Leu Leu Leu Ala
                85                  90                  95

Cys Ile Ala Val Asp Arg Tyr Leu Ala Ile Val His Ala Val His Ala
            100                 105                 110

Tyr Arg His Arg Arg Leu Leu Ser Ile His Ile Thr Cys Gly Thr Ile
        115                 120                 125

Trp Leu Val Gly Phe Leu Leu Ala Leu Pro Glu Ile Leu Phe Ala Lys
    130                 135                 140

Val Ser Gln Gly His His Asn Asn Ser Leu Pro Arg Cys Thr Phe Ser
145                 150                 155                 160

Gln Glu Asn Gln Ala Glu Thr His Ala Trp Phe Thr Ser Arg Phe Leu
                165                 170                 175

Tyr His Val Ala Gly Phe Leu Leu Pro Met Leu Val Met Gly Trp Cys
            180                 185                 190

Tyr Val Gly Val Val His Arg Leu Arg Gln Ala Gln Arg Arg Pro Gln
        195                 200                 205

Arg Gln Lys Ala Val Arg Val Ala Ile Leu Val Thr Ser Ile Phe Phe
    210                 215                 220

Leu Cys Trp Ser Pro Tyr His Ile Val Ile Phe Leu Asp Thr Leu Ala
225                 230                 235                 240

Arg Leu Lys Ala Val Asp Asn Thr Cys Lys Leu Asn Gly Ser Leu Pro
                245                 250                 255

Val Ala Ile Thr Met Cys Glu Phe Leu Gly Leu Ala His Cys Cys Leu
            260                 265                 270

Asn Pro Met Leu Tyr Thr Phe Ala Gly Val Lys Phe Arg Ser Asp Leu
        275                 280                 285

Ser Arg Leu Leu Thr Lys Leu Gly Cys Thr Gly Pro Ala Ser Leu Cys
    290                 295                 300

Gln Leu Phe Pro Ser Trp Arg Arg Ser Ser Leu Ser Glu Ser Glu Asn
305                 310                 315                 320

Ala Thr Ser Leu Thr Thr Phe
                325

<210>  4
<211>  372
<212>  PRT
<213>  Homo sapiens

<220>
<223>  Exemplary amino acid sequence for human CXCR5 precursor

<300>
<308>  NP_001707.1
<309>  2015-03-15
<313>  (1)..(372)

<400>  4

Met Asn Tyr Pro Leu Thr Leu Glu Met Asp Leu Glu Asn Leu Glu Asp
1               5                   10                  15

Leu Phe Trp Glu Leu Asp Arg Leu Asp Asn Tyr Asn Asp Thr Ser Leu
            20                  25                  30

Val Glu Asn His Leu Cys Pro Ala Thr Glu Gly Pro Leu Met Ala Ser
        35                  40                  45

Phe Lys Ala Val Phe Val Pro Val Ala Tyr Ser Leu Ile Phe Leu Leu
    50                  55                  60

Gly Val Ile Gly Asn Val Leu Val Leu Val Ile Leu Glu Arg His Arg
65                  70                  75                  80

Gln Thr Arg Ser Ser Thr Glu Thr Phe Leu Phe His Leu Ala Val Ala
                85                  90                  95

Asp Leu Leu Leu Val Phe Ile Leu Pro Phe Ala Val Ala Glu Gly Ser
            100                 105                 110

Val Gly Trp Val Leu Gly Thr Phe Leu Cys Lys Thr Val Ile Ala Leu
        115                 120                 125

His Lys Val Asn Phe Tyr Cys Ser Ser Leu Leu Leu Ala Cys Ile Ala
    130                 135                 140

Val Asp Arg Tyr Leu Ala Ile Val His Ala Val His Ala Tyr Arg His
145                 150                 155                 160

Arg Arg Leu Leu Ser Ile His Ile Thr Cys Gly Thr Ile Trp Leu Val
                165                 170                 175

Gly Phe Leu Leu Ala Leu Pro Glu Ile Leu Phe Ala Lys Val Ser Gln
            180                 185                 190

Gly His His Asn Asn Ser Leu Pro Arg Cys Thr Phe Ser Gln Glu Asn
        195                 200                 205

Gln Ala Glu Thr His Ala Trp Phe Thr Ser Arg Phe Leu Tyr His Val
    210                 215                 220

Ala Gly Phe Leu Leu Pro Met Leu Val Met Gly Trp Cys Tyr Val Gly
225                 230                 235                 240

Val Val His Arg Leu Arg Gln Ala Gln Arg Arg Pro Gln Arg Gln Lys
                245                 250                 255

Ala Val Arg Val Ala Ile Leu Val Thr Ser Ile Phe Phe Leu Cys Trp
            260                 265                 270

Ser Pro Tyr His Ile Val Ile Phe Leu Asp Thr Leu Ala Arg Leu Lys
        275                 280                 285

Ala Val Asp Asn Thr Cys Lys Leu Asn Gly Ser Leu Pro Val Ala Ile
    290                 295                 300

Thr Met Cys Glu Phe Leu Gly Leu Ala His Cys Cys Leu Asn Pro Met
305                 310                 315                 320

Leu Tyr Thr Phe Ala Gly Val Lys Phe Arg Ser Asp Leu Ser Arg Leu
                325                 330                 335

Leu Thr Lys Leu Gly Cys Thr Gly Pro Ala Ser Leu Cys Gln Leu Phe
            340                 345                 350

Pro Ser Trp Arg Arg Ser Ser Leu Ser Glu Ser Glu Asn Ala Thr Ser
        355                 360                 365

Leu Thr Thr Phe
    370

<210>  5
<211>  2567
<212>  DNA
<213>  Homo sapiens

<220>
<223>  Exemplary nucleic acid sequence encoding human CCR9

<300>
<308>  NM_031200.2
<309>  2015-03-15
<313>  (1)..(2567)

<400>  5
gcttcctttc tcgtgttgtt atcgggtagc tgcctgctca gaacccacaa agcctgcccc      60

tcatcccagg cagagagcaa cccagctctt tccccagaca ctgagagctg gtggtgcctg     120

ctgtcccagg gagagttgca tcgccctcca cagagcaggc ttgcatctga ctgacccacc     180

atgacaccca cagacttcac aagccctatt cctaacatgg ctgatgacta tggctctgaa     240

tccacatctt ccatggaaga ctacgttaac ttcaacttca ctgacttcta ctgtgagaaa     300

aacaatgtca ggcagtttgc gagccatttc ctcccaccct tgtactggct cgtgttcatc     360

gtgggtgcct tgggcaacag tcttgttatc cttgtctact ggtactgcac aagagtgaag     420

accatgaccg acatgttcct tttgaatttg gcaattgctg acctcctctt tcttgtcact     480

cttcccttct gggccattgc tgctgctgac cagtggaagt tccagacctt catgtgcaag     540

gtggtcaaca gcatgtacaa gatgaacttc tacagctgtg tgttgctgat catgtgcatc     600

agcgtggaca ggtacattgc cattgcccag gccatgagag cacatacttg gagggagaaa     660

aggcttttgt acagcaaaat ggtttgcttt accatctggg tattggcagc tgctctctgc     720

atcccagaaa tcttatacag ccaaatcaag gaggaatccg gcattgctat ctgcaccatg     780

gtttacccta gcgatgagag caccaaactg aagtcagctg tcttgaccct gaaggtcatt     840

ctggggttct tccttccctt cgtggtcatg gcttgctgct ataccatcat cattcacacc     900

ctgatacaag ccaagaagtc ttccaagcac aaagccctaa aagtgaccat cactgtcctg     960

accgtctttg tcttgtctca gtttccctac aactgcattt tgttggtgca gaccattgac    1020

gcctatgcca tgttcatctc caactgtgcc gtttccacca acattgacat ctgcttccag    1080

gtcacccaga ccatcgcctt cttccacagt tgcctgaacc ctgttctcta tgtttttgtg 1140

ggtgagagat tccgccggga tctcgtgaaa accctgaaga acttgggttg catcagccag 1200

gcccagtggg tttcatttac aaggagagag ggaagcttga agctgtcgtc tatgttgctg 1260

gagacaacct caggagcact ctccctctga ggggtcttct ctgaggtgca tggttctttt 1320

ggaagaaatg agaaatacag aaacagtttc cccactgatg ggaccagaga gagtgaaaga 1380

gaaaagaaaa ctcagaaagg gatgaatctg aactatatga ttacttgtag tcagaatttg 1440

ccaaagcaaa tatttcaaaa tcaactgact agtgcaggag gctgttgatt ggctcttgac 1500

tgtgatgccc gcaattctca aaggaggact aaggaccggc actgtggagc accctggctt 1560

tgccactcgc cggagcatca atgccgctgc ctctggagga gcccttggat tttctccatg 1620

cactgtgaac ttctgtggct tcagttctca tgctgcctct tccaaaaggg gacacagaag 1680

cactggctgc tgctacagac cgcaaaagca gaaagtttcg tgaaaatgtc catctttggg 1740

aaattttcta ccctgctctt gagcctgata acccatgcca ggtcttatag attcctgatc 1800

tagaaccttt ccaggcaatc tcagacctaa tttccttctg ttctccttgt tctgttctgg 1860

gccagtgaag gtccttgttc tgattttgaa acgatctgca ggtcttgcca gtgaacccct 1920

ggacaactga ccacacccac aaggcatcca aagtctgttg gcttccaatc catttctgtg 1980

tcctgctgga ggttttaacc tagacaagga ttccgcttat tccttggtat ggtgacagtg 2040

tctctccatg gcctgagcag ggagattata acagctgggt tcgcaggagc cagccttggc 2100

cctgttgtag gcttgttctg ttgagtggca cttgctttgg gtccaccgtc tgtctgctcc 2160

ctagaaaatg ggctggttct tttggccctc ttctttctga ggcccacttt attctgagga 2220

atacagtgag cagatatggg cagcagccag gtagggcaaa ggggtgaagc gcaggccttg 2280

ctggaaggct atttacttcc atgcttctcc ttttcttact ctatagtggc aacattttaa 2340

aagcttttaa cttagagatt aggctgaaaa aaataagtaa tggaattcac ctttgcatct 2400

tttgtgtctt tcttatcatg atttggcaaa atgcatcacc tttgaaaata tttcacatat 2460

tggaaaagtg ctttttaatg tgtatatgaa gcattaatta cttgtcactt tctttaccct 2520

gtctcaatat tttaagtgtg tgcaattaaa gatcaaatag atacatt 2567

<210> 6
<211> 2653
<212> DNA
<213> Homo sapiens

<220>
<223> Exemplary nucleic acid sequence encoding human CCR9

<300>
<308> NM001256369.1
<309> 2015-03-15

<313>   (1)..(2653)

<400>   6
gcttcctttc tcgtgttgtt atcgggtagc tgcctgctca gaacccacaa agcctgcccc      60

tcatcccagg cagagagcaa cccagctctt tccccagaca ctgagagctg gtggtgcctg     120

ctgtcccagg gagagttgca tcgccctcca cagagcaggc ttgcatctga ctgacccacc     180

atgacaccca cagacttcac atctcctcca ggccccgctc cagatcacct tccctcgctg     240

gcccaggaat ccatctcctt ccaggacctt agcccaggac taacacaagc cctattccta     300

acatggctga tgactatggc tctgaatcca catcttccat ggaagactac gttaacttca     360

acttcactga cttctactgt gagaaaaaca atgtcaggca gtttgcgagc catttcctcc     420

cacccttgta ctggctcgtg ttcatcgtgg gtgccttggg caacagtctt gttatccttg     480

tctactggta ctgcacaaga gtgaagacca tgaccgacat gttccttttg aatttggcaa     540

ttgctgacct cctctttctt gtcactcttc ccttctgggc cattgctgct gctgaccagt     600

ggaagttcca gaccttcatg tgcaaggtgg tcaacagcat gtacaagatg aacttctaca     660

gctgtgtgtt gctgatcatg tgcatcagcg tggacaggta cattgccatt gcccaggcca     720

tgagagcaca tacttggagg gagaaaaggc ttttgtacag caaaatggtt tgctttacca     780

tctgggtatt ggcagctgct ctctgcatcc cagaaatctt atacagccaa atcaaggagg     840

aatccggcat tgctatctgc accatggttt accctagcga tgagagcacc aaactgaagt     900

cagctgtctt gaccctgaag gtcattctgg ggttcttcct tcccttcgtg gtcatggctt     960

gctgctatac catcatcatt cacaccctga tacaagccaa gaagtcttcc aagcacaaag    1020

ccctaaaagt gaccatcact gtcctgaccg tctttgtctt gtctcagttt ccctacaact    1080

gcattttgtt ggtgcagacc attgacgcct atgccatgtt catctccaac tgtgccgttt    1140

ccaccaacat tgacatctgc ttccaggtca cccagaccat cgccttcttc cacagttgcc    1200

tgaaccctgt tctctatgtt tttgtgggtg agagattccg ccgggatctc gtgaaaaccc    1260

tgaagaactt gggttgcatc agccaggccc agtgggtttc atttacaagg agagagggaa    1320

gcttgaagct gtcgtctatg ttgctggaga caacctcagg agcactctcc ctctgagggg    1380

tcttctctga ggtgcatggt tcttttggaa gaaatgagaa atacagaaac agtttcccca    1440

ctgatgggac cagagagagt gaaagagaaa agaaaactca gaaagggatg aatctgaact    1500

atatgattac ttgtagtcag aatttgccaa agcaaatatt tcaaaatcaa ctgactagtg    1560

caggaggctg ttgattggct cttgactgtg atgcccgcaa ttctcaaagg aggactaagg    1620

accggcactg tggagcaccc tggctttgcc actcgccgga gcatcaatgc cgctgcctct    1680

ggaggagccc ttggattttc tccatgcact gtgaacttct gtggcttcag ttctcatgct    1740

gcctcttcca aaaggggaca cagaagcact ggctgctgct acagaccgca aaagcagaaa    1800

gtttcgtgaa aatgtccatc tttgggaaat tttctaccct gctcttgagc ctgataaccc 1860

atgccaggtc ttatagattc ctgatctaga acctttccag gcaatctcag acctaatttc 1920

cttctgttct ccttgttctg ttctgggcca gtgaaggtcc ttgttctgat tttgaaacga 1980

tctgcaggtc ttgccagtga acccctggac aactgaccac acccacaagg catccaaagt 2040

ctgttggctt ccaatccatt tctgtgtcct gctggaggtt ttaacctaga caaggattcc 2100

gcttattcct tggtatggtg acagtgtctc tccatggcct gagcagggag attataacag 2160

ctgggttcgc aggagccagc cttggccctg ttgtaggctt gttctgttga gtggcacttg 2220

ctttgggtcc accgtctgtc tgctccctag aaaatgggct ggttcttttg gccctcttct 2280

ttctgaggcc cactttattc tgaggaatac agtgagcaga tatgggcagc agccaggtag 2340

ggcaaagggg tgaagcgcag gccttgctgg aaggctattt acttccatgc ttctcctttt 2400

cttactctat agtggcaaca ttttaaaagc ttttaactta gagattaggc tgaaaaaaat 2460

aagtaatgga attcaccttt gcatcttttg tgtctttctt atcatgattt ggcaaaatgc 2520

atcacctttg aaaatatttc acatattgga aaagtgcttt ttaatgtgta tatgaagcat 2580

taattacttg tcactttctt taccctgtct caatatttta agtgtgtgca attaaagatc 2640

aaatagatac att 2653

<210> 7
<211> 369
<212> PRT
<213> Homo sapiens

<220>
<223> Exemplary amino acid sequence for human CCR9 precursor

<300>
<308> NP_112477.1
<309> 2015-03-15
<313> (1)..(369)

<400> 7

Met Thr Pro Thr Asp Phe Thr Ser Pro Ile Pro Asn Met Ala Asp Asp
1               5                   10                  15

Tyr Gly Ser Glu Ser Thr Ser Ser Met Glu Asp Tyr Val Asn Phe Asn
            20                  25                  30

Phe Thr Asp Phe Tyr Cys Glu Lys Asn Asn Val Arg Gln Phe Ala Ser
        35                  40                  45

His Phe Leu Pro Pro Leu Tyr Trp Leu Val Phe Ile Val Gly Ala Leu
    50                  55                  60

Gly Asn Ser Leu Val Ile Leu Val Tyr Trp Tyr Cys Thr Arg Val Lys

65                  70                  75                  80

Thr Met Thr Asp Met Phe Leu Leu Asn Leu Ala Ile Ala Asp Leu Leu
                85                  90                  95

Phe Leu Val Thr Leu Pro Phe Trp Ala Ile Ala Ala Ala Asp Gln Trp
            100                 105                 110

Lys Phe Gln Thr Phe Met Cys Lys Val Val Asn Ser Met Tyr Lys Met
        115                 120                 125

Asn Phe Tyr Ser Cys Val Leu Leu Ile Met Cys Ile Ser Val Asp Arg
    130                 135                 140

Tyr Ile Ala Ile Ala Gln Ala Met Arg Ala His Thr Trp Arg Glu Lys
145                 150                 155                 160

Arg Leu Leu Tyr Ser Lys Met Val Cys Phe Thr Ile Trp Val Leu Ala
                165                 170                 175

Ala Ala Leu Cys Ile Pro Glu Ile Leu Tyr Ser Gln Ile Lys Glu Glu
            180                 185                 190

Ser Gly Ile Ala Ile Cys Thr Met Val Tyr Pro Ser Asp Glu Ser Thr
        195                 200                 205

Lys Leu Lys Ser Ala Val Leu Thr Leu Lys Val Ile Leu Gly Phe Phe
    210                 215                 220

Leu Pro Phe Val Val Met Ala Cys Cys Tyr Thr Ile Ile Ile His Thr
225                 230                 235                 240

Leu Ile Gln Ala Lys Lys Ser Ser Lys His Lys Ala Leu Lys Val Thr
                245                 250                 255

Ile Thr Val Leu Thr Val Phe Val Leu Ser Gln Phe Pro Tyr Asn Cys
            260                 265                 270

Ile Leu Leu Val Gln Thr Ile Asp Ala Tyr Ala Met Phe Ile Ser Asn
        275                 280                 285

Cys Ala Val Ser Thr Asn Ile Asp Ile Cys Phe Gln Val Thr Gln Thr
    290                 295                 300

Ile Ala Phe Phe His Ser Cys Leu Asn Pro Val Leu Tyr Val Phe Val
305                 310                 315                 320

Gly Glu Arg Phe Arg Arg Asp Leu Val Lys Thr Leu Lys Asn Leu Gly
                325                 330                 335

Cys Ile Ser Gln Ala Gln Trp Val Ser Phe Thr Arg Arg Glu Gly Ser
            340                 345                 350

Leu Lys Leu Ser Ser Met Leu Leu Glu Thr Thr Ser Gly Ala Leu Ser
        355                 360                 365

Leu

<210>  8
<211>  357
<212>  PRT
<213>  Homo sapiens

<220>
<223>  Exemplary amino acid sequence for human CCR9 precursor

<300>
<308>  NP_001243298.1
<309>  2015-03-15
<313>  (1)..(357)

<400>  8

Met Ala Asp Asp Tyr Gly Ser Glu Ser Thr Ser Ser Met Glu Asp Tyr
1               5                   10                  15

Val Asn Phe Asn Phe Thr Asp Phe Tyr Cys Glu Lys Asn Asn Val Arg
            20                  25                  30

Gln Phe Ala Ser His Phe Leu Pro Pro Leu Tyr Trp Leu Val Phe Ile
        35                  40                  45

Val Gly Ala Leu Gly Asn Ser Leu Val Ile Leu Val Tyr Trp Tyr Cys
    50                  55                  60

Thr Arg Val Lys Thr Met Thr Asp Met Phe Leu Leu Asn Leu Ala Ile
65                  70                  75                  80

Ala Asp Leu Leu Phe Leu Val Thr Leu Pro Phe Trp Ala Ile Ala Ala
                85                  90                  95

Ala Asp Gln Trp Lys Phe Gln Thr Phe Met Cys Lys Val Val Asn Ser
            100                 105                 110

Met Tyr Lys Met Asn Phe Tyr Ser Cys Val Leu Leu Ile Met Cys Ile
        115                 120                 125

Ser Val Asp Arg Tyr Ile Ala Ile Ala Gln Ala Met Arg Ala His Thr
    130                 135                 140

Trp Arg Glu Lys Arg Leu Leu Tyr Ser Lys Met Val Cys Phe Thr Ile
145                 150                 155                 160

Trp Val Leu Ala Ala Ala Leu Cys Ile Pro Glu Ile Leu Tyr Ser Gln
                165                 170                 175

Ile Lys Glu Glu Ser Gly Ile Ala Ile Cys Thr Met Val Tyr Pro Ser
            180                 185                 190

Asp Glu Ser Thr Lys Leu Lys Ser Ala Val Leu Thr Leu Lys Val Ile
        195                 200                 205

Leu Gly Phe Phe Leu Pro Phe Val Val Met Ala Cys Cys Tyr Thr Ile
    210                 215                 220

Ile Ile His Thr Leu Ile Gln Ala Lys Lys Ser Ser Lys His Lys Ala
225                 230                 235                 240

Leu Lys Val Thr Ile Thr Val Leu Thr Val Phe Val Leu Ser Gln Phe
                245                 250                 255

Pro Tyr Asn Cys Ile Leu Leu Val Gln Thr Ile Asp Ala Tyr Ala Met
            260                 265                 270

Phe Ile Ser Asn Cys Ala Val Ser Thr Asn Ile Asp Ile Cys Phe Gln
        275                 280                 285

Val Thr Gln Thr Ile Ala Phe Phe His Ser Cys Leu Asn Pro Val Leu
    290                 295                 300

Tyr Val Phe Val Gly Glu Arg Phe Arg Arg Asp Leu Val Lys Thr Leu
305                 310                 315                 320

Lys Asn Leu Gly Cys Ile Ser Gln Ala Gln Trp Val Ser Phe Thr Arg
                325                 330                 335

Arg Glu Gly Ser Leu Lys Leu Ser Ser Met Leu Leu Glu Thr Thr Ser
            340                 345                 350

Gly Ala Leu Ser Leu
        355

<210>  9
<211>  6082
<212>  DNA

<213>  Homo sapiens

<220>
<223>  Exemplary nucleic acid sequence encoding human alpha-4

<300>
<308>  NM_000885.4
<309>  2015-03-15
<313>  (1)..(6082)

<400>  9
   ataacgtctt tgtcactaaa atgttcccca ggggccttcg gcgagtcttt ttgtttggtt      60
   ttttgttttt aatctgtggc tcttgataat ttatctagtg gttgcctaca cctgaaaaac     120
   aagacacagt gtttaactat caacgaaaga actggacggc tccccgccgc agtcccactc     180
   cccgagtttg tggctggcat ttgggccacg ccgggctggg cggtcacagc gaggggcgcg     240
   cagtttgggg tcacacagct ccgcttctag gccccaacca ccgttaaaag gggaagcccg     300
   tgccccatca ggtccgctct tgctgagccc agagccatcc cgcgctctgc gggctgggag     360
   gcccgggcca ggacgcgagt cctgcgcagc cgaggttccc cagcgccccc tgcagccgcg     420
   cgtaggcaga gacggagccc ggccctgcgc ctccgcacca cgcccgggac cccacccagc     480
   ggcccgtacc cggagaagca gcgcgagcac ccgaagctcc cggctggcgg cagaaaccgg     540
   gagtggggcc gggcgagtgc gcggcatccc aggccggccc gaacgctccg cccgcggtgg     600
   gccgacttcc cctcctcttc cctctctcct tcctttagcc cgctggcgcc ggacacgctg     660
   cgcctcatct cttggggcgt tcttccccgt tggccaaccg tcgcatcccg tgcaactttg     720
   gggtagtggc cgtttagtgt tgaatgttcc ccaccgagag cgcatggctt gggaagcgag     780
   gcgcgaaccc ggcccccgaa gggccgccgt ccgggagacg gtgatgctgt tgctgtgcct     840
   gggggtcccg accggccgcc cctacaacgt ggacactgag agcgcgctgc tttaccaggg     900
   cccccacaac acgctgttcg gctactcggt cgtgctgcac agccacgggg cgaaccgatg     960
   gctcctagtg ggtgcgccca ctgccaactg gctcgccaac gcttcagtga tcaatcccgg    1020
   ggcgatttac agatgcagga tcggaaagaa tcccggccag acgtgcgaac agctccagct    1080
   gggtagccct aatggagaac cttgtggaaa gacttgtttg gaagagagag acaatcagtg    1140
   gttgggggtc acactttcca gacagccagg agaaaatgga tccatcgtga cttgtgggca    1200
   tagatggaaa aatatatttt acataaagaa tgaaaataag ctccccactg gtggttgcta    1260
   tggagtgccc cctgatttac gaacagaact gagtaaaaga atagctccgt gttatcaaga    1320
   ttatgtgaaa aaatttggag aaaattttgc atcatgtcaa gctggaatat ccagttttta    1380
   cacaaaggat ttaattgtga tgggggcccc aggatcatct tactggactg gctctctttt    1440
   tgtctacaat ataactacaa ataaatacaa ggctttttta gacaaacaaa atcaagtaaa    1500
   atttggaagt tatttaggat attcagtcgg agctggtcat tttcggagcc agcatactac    1560
   cgaagtagtc ggaggagctc ctcaacatga gcagattggt aaggcatata tattcagcat 1620
   tgatgaaaaa gaactaaata tcttacatga aatgaaaggt aaaaagcttg gatcgtactt 1680
   tggagcttct gtctgtgctg tggacctcaa tgcagatggc ttctcagatc tgctcgtggg 1740
   agcacccatg cagagcacca tcagagagga aggaagagtg tttgtgtaca tcaactctgg 1800
   ctcgggagca gtaatgaatg caatggaaac aaacctcgtt ggaagtgaca aatatgctgc 1860
   aagatttggg gaatctatag ttaatcttgg cgacattgac aatgatggct ttgaagatgt 1920
   tgctatcgga gctccacaag aagatgactt gcaaggtgct atttatattt acaatggccg 1980
   tgcagatggg atctcgtcaa ccttctcaca gagaattgaa ggacttcaga tcagcaaatc 2040
   gttaagtatg tttggacagt ctatatcagg acaaattgat gcagataata atggctatgt 2100
   agatgtagca gttggtgctt ttcggtctga ttctgctgtc ttgctaagga caagacctgt 2160
   agtaattgtt gacgcttctt taagccaccc tgagtcagta aatagaacga aatttgactg 2220
   tgttgaaaat ggatggcctt ctgtgtgcat agatctaaca ctttgtttct catataaggg 2280
   caaggaagtt ccaggttaca ttgttttgtt ttataacatg agtttggatg tgaacagaaa 2340
   ggcagagtct ccaccaagat tctatttctc ttctaatgga acttctgacg tgattacagg 2400
   aagcatacag gtgtccagca gagaagctaa ctgtagaaca catcaagcat ttatgcggaa 2460
   agatgtgcgg gacatcctca ccccaattca gattgaagct gcttaccacc ttggtcctca 2520
   tgtcatcagt aaacgaagta cagaggaatt cccaccactt cagccaattc ttcagcagaa 2580
   gaaagaaaaa gacataatga aaaaaacaat aaactttgca aggttttgtg cccatgaaaa 2640
   ttgttctgct gatttacagg tttctgcaaa gattgggttt ttgaagcccc atgaaaataa 2700
   aacatatctt gctgttggga gtatgaagac attgatgttg aatgtgtcct tgtttaatgc 2760
   tggagatgat gcatatgaaa cgactctaca tgtcaaacta cccgtgggtc tttatttcat 2820
   taagatttta gagctggaag agaagcaaat aaactgtgaa gtcacagata actctggcgt 2880
   ggtacaactt gactgcagta ttggctatat atatgtagat catctctcaa ggatagatat 2940
   tagctttctc ctggatgtga gctcactcag cagagcggaa gaggacctca gtatcacagt 3000
   gcatgctacc tgtgaaaatg aagaggaaat ggacaatcta aagcacagca gagtgactgt 3060
   agcaatacct ttaaaatatg aggttaagct gactgttcat gggtttgtaa acccaacttc 3120
   atttgtgtat ggatcaaatg atgaaaatga gcctgaaacg tgcatggtgg agaaaatgaa 3180
   cttaactttc catgttatca acactggcaa tagtatggct cccaatgtta gtgtggaaat 3240
   aatggtacca aattctttta gcccccaaac tgataagctg ttcaacattt tggatgtcca 3300
   gactactact ggagaatgcc actttgaaaa ttatcaaaga gtgtgtgcat tagagcagca 3360
   aaagagtgca atgcagacct tgaaaggcat agtccggttc ttgtccaaga ctgataagag 3420
   gctattgtac tgcataaaag ctgatccaca ttgtttaaat ttcttgtgta attttgggaa 3480
   aatggaaagt ggaaaagaag ccagtgttca tatccaactg gaaggccggc catccatttt 3540
   agaaatggat gagacttcag cactcaagtt tgaaataaga gcaacaggtt ttccagagcc 3600
   aaatccaaga gtaattgaac taaacaagga tgagaatgtt gcgcatgttc tactggaagg 3660
   actacatcat caaagaccca aacgttattt caccatagtg attatttcaa gtagcttgct 3720
   acttggactt attgtacttc tgttgatctc atatgttatg tggaaggctg gcttctttaa 3780
   aagacaatac aaatctatcc tacaagaaga aaacagaaga gacagttgga gttatatcaa 3840
   cagtaaaagc aatgatgatt aaggacttct ttcaaattga gagaatggaa aacagactca 3900
   ggttgtagta aagaaattta aaagacactg tttacaagaa aaaatgaatt ttgtttggac 3960
   ttcttttact catgatcttg tgacatatta tgtcttcatg caaggggaaa atctcagcaa 4020
   tgattactct ttgagataga agaactgcaa aggtaataat acagccaaag ataatctctc 4080
   agcttttaaa tgggtagaga aacactaaag cattcaattt attcaagaaa agtaagccct 4140
   tgaagatatc ttgaaatgaa agtataactg agttaaatta tactggagaa gtcttagact 4200
   tgaaatacta cttaccatat gtgcttgcct cagtaaaatg aaccccactg ggtgggcaga 4260
   ggttcatttc aaatacatct ttgatacttg ttcaaaatat gttctttaaa aatataattt 4320
   tttagagagc tgttcccaaa ttttctaacg agtggaccat tatcacttta aagcccttta 4380
   tttataatac atttcctacg ggctgtgttc caacaaccat tttttttcag cagactatga 4440
   atattatagt attataggcc aaactggcaa acttcagact gaacatgtac actggtttga 4500
   gcttagtgaa attacttctg gataattatt tttttataat tatggatttc accatctttc 4560
   tttctgtata tatacatgtg tttttatgta ggtatatatt taccattctt cctatctatt 4620
   cttcctataa cacaccttta tcaagcatac ccaggagtaa tcttcaaatc ttttgttata 4680
   ttctgaaaca aaagattgtg agtgttgcac tttacctgat acacgctgat ttagaaaata 4740
   cagaaaccat acctcactaa taactttaaa atcaaagctg tgcaaagact agggggccta 4800
   tacttcatat gtattatgta ctatgtaaaa tattgactat cacacaacta tttccttgga 4860
   tgtaattctt tgttaccctt tacaagtata agtgttacct tacatggaaa cgaagaaaca 4920
   aaattcataa atttaaattc ataaatttag ctgaaagata ctgattcaat ttgtatacag 4980
   tgaatataaa tgagacgaca gcaaaatttt catgaaatgt aaaatatttt tatagtttgt 5040
   tcatactata tgaggttcta ttttaaatga ctttctggat tttaaaaaat ttctttaaat 5100
   acaatcattt ttgtaatatt tattttatgc ttatgatcta gataattgca gaatatcatt 5160
   ttatctgact ctgccttcat aagagagctg tggccgaatt ttgaacatct gttataggga 5220
   gtgatcaaat tagaaggcaa tgtggaaaaa caattctggg aaagatttct ttatatgaag 5280
   tccctgccac tagccagcca tcctaattga tgaaagttat ctgttcacag gcctgcagtg 5340
   atggtgagga atgttctgag atttgcgaag gcatttgagt agtgaaatgt aagcacaaaa 5400
   cctcctgaac ccagagtgtg tatacacagg aataaacttt atgacattta tgtattttta 5460
   aaaaactttg tatcgttata aaaaggctag tcattctttc aggagaacat ctaggatcat 5520
   agatgaaaaa tcaagccccg atttagaact gtcttctcca ggatggtctc taaggaaatt 5580
   tacatttggt tctttcctac tcagaactac tcagaaacaa ctatatattt caggttatct 5640
   gagcacagtg aaagcagagt actatggttg tccaacacag gcctctcaga tacaagggga 5700
   acacaattac atattgggct agattttgcc cagttcaaaa tagtatttgt tatcaactta 5760
   ctttgttact tgtatcatga attttaaaac cctaccactt taagaagaca gggatgggtt 5820
   attctttttt ggcaggtagg ctatataact atgtgatttt gaaatttaac tgctctggat 5880
   tagggagcag tgaatcaagg cagacttatg aaatctgtat tatatttgta acagaatata 5940
   ggaaatttaa cataattgat gagctcaaat cctgaaaaat gaaagaatcc aaattatttc 6000
   agaattatct aggttaaata ttgatgtatt atgatggttg caaagttttt ttgtgtgtcc 6060
   aataaacaca ttgtaaaaaa aa 6082

<210> 10
<211> 2878
<212> DNA
<213> Homo sapiens

<220>
<223> Exemplary nucleic acid sequence encoding human beta-7

<300>
<308> NM_000889.2
<309> 2015-03-15
<313> (1)..(2878)

<400> 10
aaatcttccc caccctgggg agtgtcactt cctcctctgc cgtctcccag atcagtacac 60

aaaggctgct gctgccgcca gaggaaggac tgctctgcac gcacctatgt ggaaactaaa 120

gcccagagag aaagtctgac ttgccccaca gccagtgagt gactgcagca gcaccagaat 180

ctggtctgtt tcctgtttgg ctcttctacc actacggctt gggatctcgg gcatggtggc 240

tttgccaatg gtccttgttt tgctgctggt cctgagcaga ggtgagagtg aattggacgc 300

caagatccca tccacagggg atgccacaga atggcggaat cctcacctgt ccatgctggg 360

gtcctgccag ccagccccct cctgccagaa gtgcatcctc tcacacccca gctgtgcatg 420

gtgcaagcaa ctgaacttca ccgcgtcggg agaggcggag gcgcggcgct gcgcccgacg 480

agaggagctg ctggctcgag gctgcccgct ggaggagctg gaggagcccc gcggccagca 540

ggaggtgctg caggaccagc cgctcagcca gggcgcccgc ggagagggtg ccacccagct 600

ggcgccgcag cgggtccggg tcacgctgcg gcctggggag ccccagcagc tccaggtccg 660

cttccttcgt gctgagggat acccggtgga cctgtactac cttatggacc tgagctactc 720

catgaaggac gacctggaac gcgtgcgcca gctcgggcac gctctgctgg tccggctgca 780

ggaagtcacc cattctgtgc gcattggttt tggttccttt gtggacaaaa cggtgctgcc 840

ctttgtgagc acagtaccct ccaaactgcg ccacccctgc cccacccggc tggagcgctg 900

ccagtcacca ttcagctttc accatgtgct gtccctgacg ggggacgcac aagccttcga 960

gcgggaggtg ggcgccagag tgtgtccgg caatctggac tcgcctgaag gtggcttcga 1020

tgccattctg caggctgcac tctgccagga gcagattggc tggagaaatg tgtcccggct 1080

gctggtgttc acttcagacg acacattcca tacagctggg gacgggaagt tgggcggcat 1140

tttcatgccc agtgatgggc actgccactt ggacagcaat ggcctctaca gtcgcagcac 1200

agagtttgac tacccttctg tgggtcaggt agcccaggcc ctctctgcag caaatatcca 1260

gcccatcttt gctgtcacca gtgccgcact gcctgtctac caggagctga gtaaactgat 1320

tcctaagtct gcagttgggg agctgagtga ggactccagc aacgtggtac agctcatcat 1380

ggatgcttat aatagcctgt cttccaccgt gacccttgaa cactcttcac tccctcctgg 1440

ggtccacatt tcttacgaat cccagtgtga gggtcctgag aagagggagg gtaaggctga 1500

ggatcgagga cagtgcaacc acgtccgaat caaccagacg gtgactttct gggtttctct 1560

ccaagccacc cactgcctcc cagagcccca tctcctgagg ctccgggccc ttggcttctc 1620

agaggagctg attgtggagt tgcacacgct gtgtgactgt aattgcagtg acacccagcc 1680

ccaggctccc cactgcagtg atggccaggg acacctacaa tgtggtgtat gcagctgtgc 1740

ccctggccgc ctaggtcggc tctgtgagtg ctctgtggca gagctgtcct ccccagacct 1800

ggaatctggg tgccgggctc ccaatggcac agggcccctg tgcagtggaa agggtcactg 1860

tcaatgtgga cgctgcagct gcagtggaca gagctctggg catctgtgcg agtgtgacga 1920

tgccagctgt gagcgacatg agggcatcct ctgcggaggc tttggtcgct gccaatgtgg 1980

agtatgtcac tgtcatgcca accgcacggg cagagcatgc gaatgcagtg gggacatgga 2040

cagttgcatc agtcccgagg gagggctctg cagtgggcat ggacgctgca aatgcaaccg 2100

ctgccagtgc ttggacggct actatggtgc tctatgcgac caatgcccag gctgcaagac 2160

accatgcgag agacaccggg actgtgcaga gtgtggggcc ttcaggactg gcccactggc 2220

caccaactgc agtacagctt gtgcccatac caatgtgacc ctggccttgg cccctatctt 2280

ggatgatggc tggtgcaaag agcggaccct ggacaaccag ctgttcttct tcttggtgga 2340

ggatgacgcc agaggcacgg tcgtgctcag agtgagaccc caagaaaagg gagcagacca 2400

cacgcaggcc attgtgctgg gctgcgtagg gggcatcgtg gcagtggggc tggggctggt 2460

cctggcttac cggctctcgg tggaaatcta tgaccgccgg gaatacagtc gctttgagaa 2520

ggagcagcaa caactcaact ggaagcagga cagtaatcct ctctacaaaa gtgccatcac 2580

gaccaccatc aatcctcgct ttcaagaggc agacagtccc actctctgaa ggagggaggg 2640

acacttaccc aaggctcttc tccttggagg acagtgggaa ctggagggtg agaggaaggg 2700

tgggtctgta agaccttggt aggggactaa ttcactggcg aggtgcggcc accaccctac 2760

ttcattttca gagtgacacc caagagggct gcttcccatg cctgcaacct tgcatccatc 2820

tgggctaccc cacccaagta tacaataaag tcttacctca gaccacaaaa aaaaaaa 2878

<210> 11
<211> 1032
<212> PRT
<213> Homo sapiens

<220>
<223> Exemplary amino acid sequence for human alpha-4 precursor

<300>
<308> NP_000876.3
<309> 2015-03-15
<313> (1)..(1032)

<400> 11

<210>  12
<211>  798
<212>  PRT
<213>  Homo sapiens

<220>
<223>  Exemplary amino acid sequence for human beta-7 precursor

<300>
<308>  NP_000880.1
<309>  2015-03-15
<313>  (1)..(798)

<400>  12

Met Val Ala Leu Pro Met Val Leu Val Leu Leu Leu Val Leu Ser Arg
1               5                   10                  15

Gly Glu Ser Glu Leu Asp Ala Lys Ile Pro Ser Thr Gly Asp Ala Thr
            20                  25                  30

Glu Trp Arg Asn Pro His Leu Ser Met Leu Gly Ser Cys Gln Pro Ala
        35                  40                  45

Pro Ser Cys Gln Lys Cys Ile Leu Ser His Pro Ser Cys Ala Trp Cys

    50                  55                  60

Lys Gln Leu Asn Phe Thr Ala Ser Gly Glu Ala Glu Ala Arg Arg Cys
65                  70                  75                  80

Ala Arg Arg Glu Glu Leu Leu Ala Arg Gly Cys Pro Leu Glu Glu Leu
                85                  90                  95

Glu Glu Pro Arg Gly Gln Gln Glu Val Leu Gln Asp Gln Pro Leu Ser
            100                 105                 110

Gln Gly Ala Arg Gly Glu Gly Ala Thr Gln Leu Ala Pro Gln Arg Val
        115                 120                 125

Arg Val Thr Leu Arg Pro Gly Glu Pro Gln Gln Leu Gln Val Arg Phe
    130                 135                 140

Leu Arg Ala Glu Gly Tyr Pro Val Asp Leu Tyr Tyr Leu Met Asp Leu
145                 150                 155                 160

Ser Tyr Ser Met Lys Asp Asp Leu Glu Arg Val Arg Gln Leu Gly His
                165                 170                 175

Ala Leu Leu Val Arg Leu Gln Glu Val Thr His Ser Val Arg Ile Gly
            180                 185                 190

Phe Gly Ser Phe Val Asp Lys Thr Val Leu Pro Phe Val Ser Thr Val
        195                 200                 205

Pro Ser Lys Leu Arg His Pro Cys Pro Thr Arg Leu Glu Arg Cys Gln
    210                 215                 220

Ser Pro Phe Ser Phe His His Val Leu Ser Leu Thr Gly Asp Ala Gln
225                 230                 235                 240

Ala Phe Glu Arg Glu Val Gly Arg Gln Ser Val Ser Gly Asn Leu Asp
                245                 250                 255

Ser Pro Glu Gly Gly Phe Asp Ala Ile Leu Gln Ala Ala Leu Cys Gln
            260                 265                 270

Glu Gln Ile Gly Trp Arg Asn Val Ser Arg Leu Leu Val Phe Thr Ser
        275                 280                 285

Asp Asp Thr Phe His Thr Ala Gly Asp Gly Lys Leu Gly Gly Ile Phe
    290                 295                 300

Met Pro Ser Asp Gly His Cys His Leu Asp Ser Asn Gly Leu Tyr Ser
305                 310                 315                 320

Arg Ser Thr Glu Phe Asp Tyr Pro Ser Val Gly Gln Val Ala Gln Ala
                325                 330                 335

Leu Ser Ala Ala Asn Ile Gln Pro Ile Phe Ala Val Thr Ser Ala Ala
            340                 345                 350

Leu Pro Val Tyr Gln Glu Leu Ser Lys Leu Ile Pro Lys Ser Ala Val
        355                 360                 365

Gly Glu Leu Ser Glu Asp Ser Ser Asn Val Val Gln Leu Ile Met Asp
    370                 375                 380

Ala Tyr Asn Ser Leu Ser Ser Thr Val Thr Leu Glu His Ser Ser Leu
385                 390                 395                 400

Pro Pro Gly Val His Ile Ser Tyr Glu Ser Gln Cys Glu Gly Pro Glu
                405                 410                 415

Lys Arg Glu Gly Lys Ala Glu Asp Arg Gly Gln Cys Asn His Val Arg
            420                 425                 430

Ile Asn Gln Thr Val Thr Phe Trp Val Ser Leu Gln Ala Thr His Cys
        435                 440                 445

Leu Pro Glu Pro His Leu Leu Arg Leu Arg Ala Leu Gly Phe Ser Glu
    450                 455                 460

Glu Leu Ile Val Glu Leu His Thr Leu Cys Asp Cys Asn Cys Ser Asp
465                 470                 475                 480

Thr Gln Pro Gln Ala Pro His Cys Ser Asp Gly Gln Gly His Leu Gln
                485                 490                 495

Cys Gly Val Cys Ser Cys Ala Pro Gly Arg Leu Gly Arg Leu Cys Glu
            500                 505                 510

Cys Ser Val Ala Glu Leu Ser Ser Pro Asp Leu Glu Ser Gly Cys Arg
        515                 520                 525

Ala Pro Asn Gly Thr Gly Pro Leu Cys Ser Gly Lys Gly His Cys Gln
    530                 535                 540

Cys Gly Arg Cys Ser Cys Ser Gly Gln Ser Ser Gly His Leu Cys Glu
545                 550                 555                 560

Cys Asp Asp Ala Ser Cys Glu Arg His Glu Gly Ile Leu Cys Gly Gly
                565                 570                 575

Phe Gly Arg Cys Gln Cys Gly Val Cys His Cys His Ala Asn Arg Thr
            580                 585                 590

Gly Arg Ala Cys Glu Cys Ser Gly Asp Met Asp Ser Cys Ile Ser Pro
        595                 600                 605

Glu Gly Gly Leu Cys Ser Gly His Gly Arg Cys Lys Cys Asn Arg Cys
    610                 615                 620

Gln Cys Leu Asp Gly Tyr Tyr Gly Ala Leu Cys Asp Gln Cys Pro Gly
625                 630                 635                 640

Cys Lys Thr Pro Cys Glu Arg His Arg Asp Cys Ala Glu Cys Gly Ala
                645                 650                 655

Phe Arg Thr Gly Pro Leu Ala Thr Asn Cys Ser Thr Ala Cys Ala His
            660                 665                 670

Thr Asn Val Thr Leu Ala Leu Ala Pro Ile Leu Asp Asp Gly Trp Cys
        675                 680                 685

Lys Glu Arg Thr Leu Asp Asn Gln Leu Phe Phe Phe Leu Val Glu Asp
    690                 695                 700

Asp Ala Arg Gly Thr Val Val Leu Arg Val Arg Pro Gln Glu Lys Gly
705                 710                 715                 720

Ala Asp His Thr Gln Ala Ile Val Leu Gly Cys Val Gly Gly Ile Val
                725                 730                 735

Ala Val Gly Leu Gly Leu Val Leu Ala Tyr Arg Leu Ser Val Glu Ile
            740                 745                 750

Tyr Asp Arg Arg Glu Tyr Ser Arg Phe Glu Lys Glu Gln Gln Gln Leu
        755                 760                 765

Asn Trp Lys Gln Asp Ser Asn Pro Leu Tyr Lys Ser Ala Ile Thr Thr
    770                 775                 780

Thr Ile Asn Pro Arg Phe Gln Glu Ala Asp Ser Pro Thr Leu
785                 790                 795

<210>  13
<211>  1244

<212>  DNA
<213>  Homo sapiens

<220>
<223>  Exemplary nucleic acid sequence encoding human CCR10

<300>
<308>  NM_016602.2
<309>  2015-03-15
<313>  (1)..(1244)

<400>  13
agagatgggg acggaggcca cagagcaggt ttcctggggc cattactctg gggatgaaga      60

ggacgcatac tcggctgagc cactgccgga gctttgctac aaggccgatg tccaggcctt     120

cagccgggcc ttccaaccca gtgtctccct gaccgtggct gcgctgggtc tggccggcaa     180

tggcctggtc ctggccaccc acctggcagc ccgacgcgca gcgcgctcgc ccacctctgc     240

ccacctgctc cagctggccc tggccgacct cttgctggcc ctgactctgc ccttcgcggc     300

agcaggggct cttcagggct ggagtctggg aagtgccacc tgccgcacca tctctggcct     360

ctactcggcc tccttccacg ccggcttcct cttcctggcc tgtatcagcg ccgaccgcta     420

cgtggccatc gcgcgagcgc tcccagccgg gccgcggccc tccactcccg gccgcgcaca     480

cttggtctcc gtcatcgtgt ggctgctgtc actgctcctg gcgctgcctg cgctgctctt     540

cagccaggat gggcagcggg aaggccaacg acgctgtcgc ctcatcttcc ccgagggcct     600

cacgcagacg gtgaaggggg cgagcgccgt ggcgcaggtg gccctgggct tcgcgctgcc     660

gctgggcgtc atggtagcct gctacgcgct tctgggccgc acgctgctgg ccgccagggg     720

gcccgagcgc cggcgtgcgc tgcgcgtcgt ggtggctctg gtggcggcct tcgtggtgct     780

gcagctgccc tacagcctcg ccctgctgct ggatactgcc gatctactgg ctgcgcgcga     840

gcggagctgc cctgccagca aacgcaagga tgtcgcactg ctggtgacca gcggcttggc     900

cctcgcccgc tgtggcctca atcccgttct ctacgccttc ctgggcctgc gcttccgcca     960

ggacctgcgg aggctgctac ggggtgggag ctgcccctca gggcctcaac cccgccgcgg    1020

ctgcccccgc cggccccgcc tttcttcctg ctcagctccc acggagaccc acagtctctc    1080

ctgggacaac tagggctgcg aatctagagg agggggcagg ctgagggtcg tgggaaaggg    1140

gagtaggtgg gggaacactg agaaagaggc agggacctaa agggactacc tctgtgcctt    1200

gccacattaa attgataaca tggaaatgag atgcaaccca acaa                      1244


<210>  14
<211>  1244
<212>  DNA
<213>  Homo sapiens

<220>
<223>  Exemplary nucleic acid sequence encoding human CCR10

<300>
<308>  AF215981.1
<309>  2000-04-13
<313>  (1)..(1244)

<400>  14
agagatgggg acggaggcca cagagcaggt ttcctggggc cattactctg gggatgaaga      60

ggacgcatac tcggctgagc cactgccgga gctttgctac aaggccgatg tccaggcctt     120

cagccgggcc ttccaaccca gtgtctccct gaccgtggct gcgctgggtc tggccggcaa     180

tggcctggtc ctggccaccc acctggcagc ccgacgcgca gcgcgctcgc ccacctctgc     240

ccacctgctc cagctggccc tggccgacct cttgctggcc ctgactctgc ccttcgcggc     300

agcaggggct cttcagggct ggagtctggg aagtgccacc tgccgcacca tctctggcct     360

ctactcggcc tccttccacg ccggcttcct cttcctggcc tgtatcagcg ccgaccgcta     420

cgtggccatc gcgcgagcgc tcccagccgg gccgcggccc tccactcccg gccgcgcaca     480

cttggtctcc gtcatcgtgt ggctgctgtc actgctcctg gcgctgcctg cgctgctctt     540

cagccaggat gggcagcggg aaggccaacg acgctgtcgc ctcatcttcc ccgagggcct     600

cacgcagacg gtgaaggggg cgagcgccgt ggcgcaggtg gccctgggct tcgcgctgcc     660

gctgggcgtc atggtagcct gctacgcgct tctgggccgc acgctgctgg ccgccagggg     720

gcccgagcgc cggcgtgcgc tgcgcgtcgt ggtggctctg gtggcggcct tcgtggtgct     780

gcagctgccc tacagcctcg ccctgctgct ggatactgcc gatctactgg ctgcgcgcga     840

gcggagctgc cctgccagca aacgcaagga tgtcgcactg ctggtgacca gcggcttggc     900

cctcgcccgc tgtggcctca atcccgttct ctacgccttc ctgggcctgc gcttccgcca     960

ggacctgcgg aggctgctac ggggtgggag ctcgccctca gggcctcaac cccgccgcgg    1020

ctgcccccgc cggccccgcc tttcttcctg ctcagctccc acggagaccc acagtctctc    1080

ctgggacaac tagggctgcg aatctagagg agggggcagg ctgagggtcg tgggaaaggg    1140

gagtaggtgg gggaacactg agaaagaggc agggacctaa agggactacc tctgtgcctt    1200

gccacattaa attgataaca tggaaatgaa aaaaaaaaaa aaaa                    1244

<210>  15
<211>  362
<212>  PRT
<213>  Homo sapiens

<220>
<223>  Exemplary amino acid sequence for human CCR10 precursor

<300>
<308>  NP_057686.2
<309>  2015-03-15
<313>  (1)..(362)

<400>  15

Met Gly Thr Glu Ala Thr Glu Gln Val Ser Trp Gly His Tyr Ser Gly
1               5                   10                  15

Asp Glu Glu Asp Ala Tyr Ser Ala Glu Pro Leu Pro Glu Leu Cys Tyr
            20                  25                  30

Lys Ala Asp Val Gln Ala Phe Ser Arg Ala Phe Gln Pro Ser Val Ser
        35                  40                  45

Leu Thr Val Ala Ala Leu Gly Leu Ala Gly Asn Gly Leu Val Leu Ala
    50                  55                  60

Thr His Leu Ala Ala Arg Arg Ala Ala Arg Ser Pro Thr Ser Ala His
65                  70                  75                  80

Leu Leu Gln Leu Ala Leu Ala Asp Leu Leu Leu Ala Leu Thr Leu Pro
                85                  90                  95

Phe Ala Ala Ala Gly Ala Leu Gln Gly Trp Ser Leu Gly Ser Ala Thr
            100                 105                 110

Cys Arg Thr Ile Ser Gly Leu Tyr Ser Ala Ser Phe His Ala Gly Phe
        115                 120                 125

Leu Phe Leu Ala Cys Ile Ser Ala Asp Arg Tyr Val Ala Ile Ala Arg
    130                 135                 140

Ala Leu Pro Ala Gly Pro Arg Pro Ser Thr Pro Gly Arg Ala His Leu
145                 150                 155                 160

Val Ser Val Ile Val Trp Leu Leu Ser Leu Leu Leu Ala Leu Pro Ala
                165                 170                 175

Leu Leu Phe Ser Gln Asp Gly Gln Arg Glu Gly Gln Arg Arg Cys Arg
            180                 185                 190

Leu Ile Phe Pro Glu Gly Leu Thr Gln Thr Val Lys Gly Ala Ser Ala
        195                 200                 205

Val Ala Gln Val Ala Leu Gly Phe Ala Leu Pro Leu Gly Val Met Val
    210                 215                 220

Ala Cys Tyr Ala Leu Leu Gly Arg Thr Leu Leu Ala Ala Arg Gly Pro
225                 230                 235                 240

Glu Arg Arg Arg Ala Leu Arg Val Val Val Ala Leu Val Ala Ala Phe
                245                 250                 255

Val Val Leu Gln Leu Pro Tyr Ser Leu Ala Leu Leu Leu Asp Thr Ala
            260                 265                 270

Asp Leu Leu Ala Ala Arg Glu Arg Ser Cys Pro Ala Ser Lys Arg Lys
        275                 280                 285

Asp Val Ala Leu Leu Val Thr Ser Gly Leu Ala Leu Ala Arg Cys Gly
    290                 295                 300

Leu Asn Pro Val Leu Tyr Ala Phe Leu Gly Leu Arg Phe Arg Gln Asp
305                 310                 315                 320

Leu Arg Arg Leu Leu Arg Gly Gly Ser Cys Pro Ser Gly Pro Gln Pro
                325                 330                 335

Arg Arg Gly Cys Pro Arg Arg Pro Arg Leu Ser Ser Cys Ser Ala Pro
            340                 345                 350

Thr Glu Thr His Ser Leu Ser Trp Asp Asn
        355                 360

<210>  16
<211>  362
<212>  PRT
<213>  Homo sapiens

<220>
<223>  Exemplary amino acid sequence for human CCR10 precursor

<300>
<308>  P46092.3
<309>  2015-07-22
<313>  (1)..(362)

<400>  16

Met Gly Thr Glu Ala Thr Glu Gln Val Ser Trp Gly His Tyr Ser Gly
1               5                   10                  15

Asp Glu Glu Asp Ala Tyr Ser Ala Glu Pro Leu Pro Glu Leu Cys Tyr
            20                  25                  30

Lys Ala Asp Val Gln Ala Phe Ser Arg Ala Phe Gln Pro Ser Val Ser
        35                  40                  45

Leu Thr Val Ala Ala Leu Gly Leu Ala Gly Asn Gly Leu Val Leu Ala
    50                  55                  60

Thr His Leu Ala Ala Arg Arg Ala Ala Arg Ser Pro Thr Ser Ala His
65                  70                  75                  80

Leu Leu Gln Leu Ala Leu Ala Asp Leu Leu Leu Ala Leu Thr Leu Pro
                85                  90                  95

Phe Ala Ala Ala Gly Ala Leu Gln Gly Trp Ser Leu Gly Ser Ala Thr
            100                 105                 110

Cys Arg Thr Ile Ser Gly Leu Tyr Ser Ala Ser Phe His Ala Gly Phe
        115                 120                 125

Leu Phe Leu Ala Cys Ile Ser Ala Asp Arg Tyr Val Ala Ile Ala Arg
    130                 135                 140

Ala Leu Pro Ala Gly Pro Arg Pro Ser Thr Pro Gly Arg Ala His Leu
145                 150                 155                 160

Val Ser Val Ile Val Trp Leu Leu Ser Leu Leu Leu Ala Leu Pro Ala
                165                 170                 175

Leu Leu Phe Ser Gln Asp Gly Gln Arg Glu Gly Gln Arg Arg Cys Arg
            180                 185                 190

Leu Ile Phe Pro Glu Gly Leu Thr Gln Thr Val Lys Gly Ala Ser Ala
        195                 200                 205

Val Ala Gln Val Ala Leu Gly Phe Ala Leu Pro Leu Gly Val Met Val
    210                 215                 220

Ala Cys Tyr Ala Leu Leu Gly Arg Thr Leu Leu Ala Ala Arg Gly Pro
225                 230                 235                 240

Glu Arg Arg Arg Ala Leu Arg Val Val Val Ala Leu Val Ala Ala Phe
                245                 250                 255

Val Val Leu Gln Leu Pro Tyr Ser Leu Ala Leu Leu Leu Asp Thr Ala
            260                 265                 270

Asp Leu Leu Ala Ala Arg Glu Arg Ser Cys Pro Ala Ser Lys Arg Lys
        275                 280                 285

Asp Val Ala Leu Leu Val Thr Ser Gly Leu Ala Leu Ala Arg Cys Gly
    290                 295                 300

Leu Asn Pro Val Leu Tyr Ala Phe Leu Gly Leu Arg Phe Arg Gln Asp
305                 310                 315                 320

Leu Arg Arg Leu Leu Arg Gly Gly Ser Cys Pro Ser Gly Pro Gln Pro

325                 330                 335

Arg Arg Gly Cys Pro Arg Arg Pro Arg Leu Ser Ser Cys Ser Ala Pro
            340                 345                 350

Thr Glu Thr His Ser Leu Ser Trp Asp Asn
        355                 360

<210>  17
<211>  1487
<212>  DNA
<213>  Homo sapiens

<220>
<223>  Exemplary nucleic acid sequence encoding human CCR8

<300>
<308>  NM_005201.3
<309>  2015-03-15
<313>  (1)..(1487)

<400>  17
tttgtagtgg gaggatacct ccagagaggc tgctgctcat tgagctgcac tcacatgagg      60

atacagactt tgtgaagaag gaattggcaa cactgaaacc tccagaacaa aggctgtcac     120

taaggtcccg ctgccttgat ggattataca cttgacctca gtgtgacaac agtgaccgac     180

tactactacc ctgatatctt ctcaagcccc tgtgatgcgg aacttattca gacaaatggc     240

aagttgctcc ttgctgtctt ttattgcctc ctgtttgtat tcagtcttct gggaaacagc     300

ctggtcatcc tggtccttgt ggtctgcaag aagctgagga gcatcacaga tgtatacctc     360

ttgaacctgg ccctgtctga cctgcttttt gtcttctcct tcccctttca gacctactat     420

ctgctggacc agtgggtgtt tgggactgta atgtgcaaag tggtgtctgg cttttattac     480

attggcttct acagcagcat gtttttcatc accctcatga gtgtggacag gtacctggct     540

gttgtccatg ccgtgtatgc cctaaaggtg aggacgatca ggatgggcac aacgctgtgc     600

ctggcagtat ggctaaccgc cattatggct accatcccat tgctagtgtt ttaccaagtg     660

gcctctgaag atggtgttct acagtgttat tcattttaca atcaacagac tttgaagtgg     720

aagatcttca ccaacttcaa aatgaacatt ttaggcttgt tgatcccatt caccatcttt     780

atgttctgct acattaaaat cctgcaccag ctgaagaggt gtcaaaacca caacaagacc     840

aaggccatca ggttggtgct cattgtggtc attgcatctt tacttttctg ggtcccattc     900

aacgtggttc ttttcctcac ttccttgcac agtatgcaca tcttggatgg atgtagcata     960

agccaacagc tgacttatgc cacccatgtc acagaaatca tttcctttac tcactgctgt    1020

gtgaaccctg ttatctatgc ttttgttggg gagaagttca agaaacacct ctcagaaata    1080

tttcagaaaa gttgcagcca aatcttcaac tacctaggaa gacaaatgcc tagggagagc    1140

tgtgaaaagt catcatcctg ccagcagcac tcctcccgtt cctccagcgt agactacatt 1200

ttgtgaggat caatgaagac taaatataaa aaacattttc ttgaatggca tgctagtagc 1260

agtgagcaaa ggtgtgggtg tgaaaggttt ccaaaaaaag ttcagcatga aggatgccat 1320

atatgttgtt gccaacactt ggaacacaat gactaaagac atagttgtgc atgcctggca 1380

caacatcaag cctgtgattg tgtttattga tgatgttgaa caagtggtaa ctttaaagga 1440

ttctgtatgc caagtgaaaa aaaaagatgt ctgacctcct tacatat 1487

<210> 18
<211> 1420
<212> DNA
<213> Homo sapiens

<220>
<223> Exemplary nucleic acid sequence encoding human CCR8

<300>
<308> BC107159.1
<309> 2006-10-04
<313> (1)..(1420)

<400> 18
ctttgtgaag aaggaattgg caacactgaa acctccagaa caaaggctgt cactaaggtc 60

ccgctgcctt gatggattat acacttgacc tcagtgtgac aacagtgacc gactactact 120

accctgatat cttctcaagc ccctgtgatg cggaacttat tcagacaaat ggcaagttgc 180

tccttgctgt cttttattgc ctcctgtttg tattcagtct tctgggaaac agcctggtca 240

tcctggtcct tgtggtctgc aagaagctga ggagcatcac agatgtatac ctcttgaacc 300

tggccctgtc tgacctgctt tttgtcttct ccttcccctt tcagacctac tatctgctgg 360

accagtgggt gtttgggact gtaatgtgca aagtggtgtc tggcttttat tacattggct 420

tctacagcag catgtttttc atcaccctca tgagtgtgga caggtacctg gctgttgtcc 480

atgccgtgta tgccctaaag gtgaggacga tcaggatggg cacaacgctg tgcctggcag 540

tatggctaac cgccattatg gctaccatcc cattgctagt gttttaccaa gtggcctctg 600

aagatggtgt tctacagtgt tattcatttt acaatcaaca gactttgaag tggaagatct 660

tcaccaactt caaaatgaac attttaggct tgttgatccc attcaccatc tttatgttct 720

gctacattaa aatcctgcac cagctgaaga ggtgtcaaaa ccacaacaag accaaggcca 780

tcaggttggt gctcattgtg gtcattgcat ctttactttt ctgggtccca ttcaacgtgg 840

ttcttttcct cacttccttg cacagtatgc acatcttgga tggatgtagc ataagccaac 900

agctgactta tgccacccat gtcacagaaa tcatttcctt tactcactgc tgtgtgaacc 960

ctgttatcta tgcttttgtt ggggagaagt tcaagaaaca cctctcagaa atatttcaga 1020

aaagttgcag ccaaatcttc aactacctag gaagacaaat gcctagggag agctgtgaaa 1080

agtcatcatc ctgccagcag cactcctccc gttcctccag cgtagactac attttgtgag     1140

gatcaatgaa gactaaatat aaaaaacatt ttcttgaatg gcatgctagt agcagtgagc     1200

aaaggtgtgg gtgtgaaagg tttccaaaaa aagttcagca tgaaggatgc cgtgtgtgtt     1260

gttgccaaca cttggaacac gatgactggg gacgtggttg tgcatgcctg gcacaacatc     1320

aagcctgtga ttgtgtttat tgatgatgtt gaacaagtgg tggctttgga ggattctgta     1380

tgccaagtga aaggggagat gtctgacctc cttcatatag                           1420

<210>  19
<211>  355
<212>  PRT
<213>  Homo sapiens

<220>
<223>  Exemplary amino acid sequence for human CCR8 precursor

<300>
<308>  NP_005192.1
<309>  2015-03-15
<313>  (1)..(355)

<400>  19

Met Asp Tyr Thr Leu Asp Leu Ser Val Thr Thr Val Thr Asp Tyr Tyr
1               5                   10                  15

Tyr Pro Asp Ile Phe Ser Ser Pro Cys Asp Ala Glu Leu Ile Gln Thr
            20                  25                  30

Asn Gly Lys Leu Leu Leu Ala Val Phe Tyr Cys Leu Leu Phe Val Phe
        35                  40                  45

Ser Leu Leu Gly Asn Ser Leu Val Ile Leu Val Leu Val Val Cys Lys
    50                  55                  60

Lys Leu Arg Ser Ile Thr Asp Val Tyr Leu Leu Asn Leu Ala Leu Ser
65                  70                  75                  80

Asp Leu Leu Phe Val Phe Ser Phe Pro Phe Gln Thr Tyr Tyr Leu Leu
                85                  90                  95

Asp Gln Trp Val Phe Gly Thr Val Met Cys Lys Val Val Ser Gly Phe
            100                 105                 110

Tyr Tyr Ile Gly Phe Tyr Ser Ser Met Phe Phe Ile Thr Leu Met Ser
        115                 120                 125

Val Asp Arg Tyr Leu Ala Val Val His Ala Val Tyr Ala Leu Lys Val
    130                 135                 140

Arg Thr Ile Arg Met Gly Thr Thr Leu Cys Leu Ala Val Trp Leu Thr
145                 150                 155                 160

Ala Ile Met Ala Thr Ile Pro Leu Leu Val Phe Tyr Gln Val Ala Ser
                165                 170                 175

Glu Asp Gly Val Leu Gln Cys Tyr Ser Phe Tyr Asn Gln Gln Thr Leu
            180                 185                 190

Lys Trp Lys Ile Phe Thr Asn Phe Lys Met Asn Ile Leu Gly Leu Leu
        195                 200                 205

Ile Pro Phe Thr Ile Phe Met Phe Cys Tyr Ile Lys Ile Leu His Gln
    210                 215                 220

Leu Lys Arg Cys Gln Asn His Asn Lys Thr Lys Ala Ile Arg Leu Val
225                 230                 235                 240

Leu Ile Val Val Ile Ala Ser Leu Leu Phe Trp Val Pro Phe Asn Val
                245                 250                 255

Val Leu Phe Leu Thr Ser Leu His Ser Met His Ile Leu Asp Gly Cys
            260                 265                 270

Ser Ile Ser Gln Gln Leu Thr Tyr Ala Thr His Val Thr Glu Ile Ile
        275                 280                 285

Ser Phe Thr His Cys Cys Val Asn Pro Val Ile Tyr Ala Phe Val Gly
    290                 295                 300

Glu Lys Phe Lys Lys His Leu Ser Glu Ile Phe Gln Lys Ser Cys Ser
305                 310                 315                 320

Gln Ile Phe Asn Tyr Leu Gly Arg Gln Met Pro Arg Glu Ser Cys Glu
                325                 330                 335

Lys Ser Ser Ser Cys Gln Gln His Ser Ser Arg Ser Ser Ser Val Asp
            340                 345                 350

Tyr Ile Leu
        355

<210>  20
<211>  355
<212>  PRT
<213>  Homo sapiens

<220>

<223>  Exemplary amino acid sequence for human CCR8 precursor

<300>
<308>  AAI07160.1
<309>  2006-10-04
<313>  (1)..(355)

<400>  20

Met Asp Tyr Thr Leu Asp Leu Ser Val Thr Thr Val Thr Asp Tyr Tyr
1               5                   10                  15

Tyr Pro Asp Ile Phe Ser Ser Pro Cys Asp Ala Glu Leu Ile Gln Thr
            20                  25                  30

Asn Gly Lys Leu Leu Leu Ala Val Phe Tyr Cys Leu Leu Phe Val Phe
        35                  40                  45

Ser Leu Leu Gly Asn Ser Leu Val Ile Leu Val Leu Val Val Cys Lys
    50                  55                  60

Lys Leu Arg Ser Ile Thr Asp Val Tyr Leu Leu Asn Leu Ala Leu Ser
65                  70                  75                  80

Asp Leu Leu Phe Val Phe Ser Phe Pro Phe Gln Thr Tyr Tyr Leu Leu
                85                  90                  95

Asp Gln Trp Val Phe Gly Thr Val Met Cys Lys Val Val Ser Gly Phe
            100                 105                 110

Tyr Tyr Ile Gly Phe Tyr Ser Ser Met Phe Phe Ile Thr Leu Met Ser
        115                 120                 125

Val Asp Arg Tyr Leu Ala Val Val His Ala Val Tyr Ala Leu Lys Val
    130                 135                 140

Arg Thr Ile Arg Met Gly Thr Thr Leu Cys Leu Ala Val Trp Leu Thr
145                 150                 155                 160

Ala Ile Met Ala Thr Ile Pro Leu Leu Val Phe Tyr Gln Val Ala Ser
                165                 170                 175

Glu Asp Gly Val Leu Gln Cys Tyr Ser Phe Tyr Asn Gln Gln Thr Leu
            180                 185                 190

Lys Trp Lys Ile Phe Thr Asn Phe Lys Met Asn Ile Leu Gly Leu Leu
        195                 200                 205

Ile Pro Phe Thr Ile Phe Met Phe Cys Tyr Ile Lys Ile Leu His Gln
    210                 215                 220

Leu Lys Arg Cys Gln Asn His Asn Lys Thr Lys Ala Ile Arg Leu Val
225                 230                 235                 240

Leu Ile Val Val Ile Ala Ser Leu Leu Phe Trp Val Pro Phe Asn Val
                245                 250                 255

Val Leu Phe Leu Thr Ser Leu His Ser Met His Ile Leu Asp Gly Cys
            260                 265                 270

Ser Ile Ser Gln Gln Leu Thr Tyr Ala Thr His Val Thr Glu Ile Ile
        275                 280                 285

Ser Phe Thr His Cys Cys Val Asn Pro Val Ile Tyr Ala Phe Val Gly
    290                 295                 300

Glu Lys Phe Lys Lys His Leu Ser Glu Ile Phe Gln Lys Ser Cys Ser
305                 310                 315                 320

Gln Ile Phe Asn Tyr Leu Gly Arg Gln Met Pro Arg Glu Ser Cys Glu
                325                 330                 335

Lys Ser Ser Ser Cys Gln Gln His Ser Ser Arg Ser Ser Ser Val Asp
            340                 345                 350

Tyr Ile Leu
        355

<210>  21
<211>  1657
<212>  DNA
<213>  Homo sapiens

<220>
<223>  Exemplary nucleic acid sequence encoding human CCR4

<300>
<308>  NM_005508.4
<309>  2015-07-19
<313>  (1)..(1657)

<400>  21
   gctcacagga agccacgcac ccttgaaagg caccgggtcc ttcttagcat cgtgcttcct      60
   gagcaagcct ggcattgcct cacagacctt cctcagagcc gctttcagaa aagcaagctg     120
   cttctggttg ggcccagacc tgccttgagg agcctgtaga gttaaaaaat gaaccccacg     180
   gatatagcag acaccaccct cgatgaaagc atatacagca attactatct gtatgaaagt     240
   atccccaagc cttgcaccaa agaaggcatc aaggcatttg gggagctctt cctgccccca     300
   ctgtattcct tggtttttgt atttggtctg cttggaaatt ctgtggtggt tctggtcctg     360
   ttcaaataca agcggctcag gtccatgact gatgtgtacc tgctcaacct tgccatctcg 420
   gatctgctct tcgtgttttc cctccctttt tggggctact atgcagcaga ccagtgggtt 480
   tttgggctag gtctgtgcaa gatgatttcc tggatgtact tggtgggctt ttacagtggc 540
   atattctttg tcatgctcat gagcattgat agatacctgg caattgtgca cgcggtgttt 600
   tccttgaggg caaggacctt gacttatggg gtcatcacca gtttggctac atggtcagtg 660
   gctgtgttcg cctcccttcc tggctttctg ttcagcactt gttatactga gcgcaaccat 720
   acctactgca aaaccaagta ctctctcaac tccacgacgt ggaaggttct cagctccctg 780
   gaaatcaaca ttctcggatt ggtgatcccc ttagggatca tgctgttttg ctactccatg 840
   atcatcagga ccttgcagca ttgtaaaaat gagaagaaga acaaggcggt gaagatgatc 900
   tttgccgtgg tggtcctctt ccttgggttc tggacacctt acaacatagt gctcttccta 960
   gagaccctgg tggagctaga agtccttcag gactgcacct ttgaaagata cttggactat 1020
   gccatccagg ccacagaaac tctggctttt gttcactgct gccttaatcc catcatctac 1080
   ttttttctgg gggagaaatt tcgcaagtac atcctacagc tcttcaaaac ctgcaggggc 1140
   ctttttgtgc tctgccaata ctgtgggctc ctccaaattt actctgctga cacccccagc 1200
   tcatcttaca cgcagtccac catggatcat gatctccatg atgctctgta gaaaaatgaa 1260
   atggtgaaat gcagagtcaa tgaactttcc acattcagag cttacttaaa attgtatttt 1320
   agtaagagat tcctgagcca gtgtcaggag gaaggcttac acccacagtg gaaagacagc 1380
   ttctcatcct gcaggcagct tttctctccc cactagacaa gtccagcctg gcaagggttc 1440
   acctgggctg aggcatcctt cctcacacca ggcttgcctg caggcatgag tcagtctgat 1500
   gagaactctg agcagtgctt gaatgaagtt gtaggtaata ttgcaaggca aagactattc 1560
   ccttctaacc tgaactgatg ggtttctcca gagggaattg cagagtactg gctgatggag 1620
   taaatcgcta ccttttgctg tggcaaatgg gccctct 1657

<210> 22
<211> 360
<212> PRT
<213> Homo sapiens

<220>
<223> Exemplary amino acid sequence for human CCR4 precursor

<300>
<308> P51679.1
<309> 2015-07-22
<313> (1)..(360)

<400> 22

Met Asn Pro Thr Asp Ile Ala Asp Thr Thr Leu Asp Glu Ser Ile Tyr
1               5                   10                  15

Ser Asn Tyr Tyr Leu Tyr Glu Ser Ile Pro Lys Pro Cys Thr Lys Glu
            20                  25                  30

Gly Ile Lys Ala Phe Gly Glu Leu Phe Leu Pro Pro Leu Tyr Ser Leu
        35                  40                  45

Val Phe Val Phe Gly Leu Leu Gly Asn Ser Val Val Val Leu Val Leu
    50                  55                  60

Phe Lys Tyr Lys Arg Leu Arg Ser Met Thr Asp Val Tyr Leu Leu Asn
65                  70                  75                  80

Leu Ala Ile Ser Asp Leu Leu Phe Val Phe Ser Leu Pro Phe Trp Gly
                85                  90                  95

Tyr Tyr Ala Ala Asp Gln Trp Val Phe Gly Leu Gly Leu Cys Lys Met
            100                 105                 110

Ile Ser Trp Met Tyr Leu Val Gly Phe Tyr Ser Gly Ile Phe Phe Val
        115                 120                 125

Met Leu Met Ser Ile Asp Arg Tyr Leu Ala Ile Val His Ala Val Phe
    130                 135                 140

Ser Leu Arg Ala Arg Thr Leu Thr Tyr Gly Val Ile Thr Ser Leu Ala
145                 150                 155                 160

Thr Trp Ser Val Ala Val Phe Ala Ser Leu Pro Gly Phe Leu Phe Ser
                165                 170                 175

Thr Cys Tyr Thr Glu Arg Asn His Thr Tyr Cys Lys Thr Lys Tyr Ser
            180                 185                 190

Leu Asn Ser Thr Thr Trp Lys Val Leu Ser Ser Leu Glu Ile Asn Ile
        195                 200                 205

Leu Gly Leu Val Ile Pro Leu Gly Ile Met Leu Phe Cys Tyr Ser Met
    210                 215                 220

Ile Ile Arg Thr Leu Gln His Cys Lys Asn Glu Lys Lys Asn Lys Ala
225                 230                 235                 240

Val Lys Met Ile Phe Ala Val Val Val Leu Phe Leu Gly Phe Trp Thr
                245                 250                 255

Pro Tyr Asn Ile Val Leu Phe Leu Glu Thr Leu Val Glu Leu Glu Val

260                 265                 270

Leu Gln Asp Cys Thr Phe Glu Arg Tyr Leu Asp Tyr Ala Ile Gln Ala
        275                 280                 285

Thr Glu Thr Leu Ala Phe Val His Cys Cys Leu Asn Pro Ile Ile Tyr
    290                 295                 300

Phe Phe Leu Gly Glu Lys Phe Arg Lys Tyr Ile Leu Gln Leu Phe Lys
305                 310                 315                 320

Thr Cys Arg Gly Leu Phe Val Leu Cys Gln Tyr Cys Gly Leu Leu Gln
                325                 330                 335

Ile Tyr Ser Ala Asp Thr Pro Ser Ser Ser Tyr Thr Gln Ser Thr Met
            340                 345                 350

Asp His Asp Leu His Asp Ala Leu
        355                 360

<210>  23
<211>  2638
<212>  DNA
<213>  Homo sapiens

<220>
<223>  Exemplary nucleic acid sequence encoding human CLA

<300>
<308>  NM_001206609.1
<309>  2015-03-15
<313>  (1)..(2638)

<400>  23
aatcatccga gaaccttgga gggtggacag tgcccctttt acagatgaga aaactgaggc      60

ttgaagggga gaagcagctg cctctggcgg catggcttct ggctgcagga tgcccatgga     120

gttcgtggtg accctaggcc tgtgtctcgg cttcctttgc tgaacttgaa caggaagatg     180

gcagtggggg ccagtggtct agaaggagat aagatggctg gtgccatgcc tctgcaactc     240

ctcctgttgc tgatcctact gggccctggc aacagcttgc agctgtggga cacctgggca     300

gatgaagccg agaaagcctt gggtcccctg cttgcccggg accggagaca ggccaccgaa     360

tatgagtacc tagattatga tttcctgcca gaaacggagc ctccagaaat gctgaggaac     420

agcactgaca ccactcctct gactgggcct ggaacccctg agtctaccac tgtggagcct     480

gctgcaaggc gttctactgg cctggatgca ggaggggcag tcacagagct gaccacggag     540

ctggccaaca tggggaacct gtccacggat tcagcagcta tggagataca gaccactcaa     600

ccagcagcca cggaggcaca gaccactcaa ccagtgccca cggaggcaca gaccactcca     660

ctggcagcca cagaggcaca gacaactcga ctgacggcca cggaggcaca gaccactcca 720

ctggcagcca cagaggcaca gaccactcca ccagcagcca cggaagcaca gaccactcaa 780

cccacaggcc tggaggcaca gaccactgca ccagcagcca tggaggcaca gaccactgca 840

ccagcagcca tggaagcaca gaccactcca ccagcagcca tggaggcaca gaccactcaa 900

accacagcca tggaggcaca gaccactgca ccagaagcca cggaggcaca gaccactcaa 960

cccacagcca cggaggcaca gaccactcca ctggcagcca tggaggccct gtccacagaa 1020

cccagtgcca cagaggccct gtccatggaa cctactacca aaagaggtct gttcataccc 1080

ttttctgtgt cctctgttac tcacaagggc attcccatgg cagccagcaa tttgtccgtc 1140

aactacccag tgggggcccc agaccacatc tctgtgaagc agtgcctgct ggccatccta 1200

atcttggcgc tggtggccac tatcttcttc gtgtgcactg tggtgctggc ggtccgcctc 1260

tcccgcaagg gccacatgta ccccgtgcgt aattactccc ccaccgagat ggtctgcatc 1320

tcatccctgt tgcctgatgg gggtgagggg ccctctgcca cagccaatgg gggcctgtcc 1380

aaggccaaga gcccgggcct gacgccagag cccagggagg accgtgaggg ggatgacctc 1440

accctgcaca gcttcctccc ttagctcact ctgccatctg ttttggcaag accccacctc 1500

cacgggctct cctgggccac ccctgagtgc ccagacccca ttccacagct ctgggcttcc 1560

tcggagaccc ctggggatgg ggatcttcag ggaaggaact ctggccaccc aaacaggaca 1620

agagcagcct ggggccaagc agacgggcaa gtggagccac ctctttcctc cctccgcgga 1680

tgaagcccag ccacatttca gccgaggtcc aaggcaggag gccatttact tgagacagat 1740

tctctccttt ttcctgtccc ccatcttctc tgggtccctc taacatctcc catggctctc 1800

cccgcttctc ctggtcactg gagtctcctc cccatgtacc caaggaagat ggagctcccc 1860

catcccacac gcactgcact gccattgtct tttggttgcc atggtcacca aacaggaagt 1920

ggacattcta agggaggagt actgaagagt gacggacttc tgaggctgtt tcctgctgct 1980

cctctgactt ggggcagctt gggtcttctt gggcacctct ctgggaaaac ccagggtgag 2040

gttcagcctg tgagggctgg gatgggtttc gtgggcccaa gggcagacct ttctttggga 2100

ctgtgtggac caaggagctt ccatctagtg acaagtgacc cccagctatc gcctcttgcc 2160

ttcccctgtg gccactttcc agggtggact ctgtcttgtt cactgcagta tcccaactgc 2220

aggtccagtg caggcaataa atatgtgatg gacaaacgat agcggaatcc ttcaaggttt 2280

caaggctgtc tccttcaggc agccttcccg gaattctcca tccctcagtg caggatgggg 2340

gctggtcctc agctgtctgc cctcagcccc tggcccccca ggaagcctct ttcatgggct 2400

gttaggttga cttcagtttt gcctcttgga caacaggggg tcttgtacat ccttgggtga 2460

ccaggaaaag ttcaggctat ggggggccaa agggagggct gccccttccc caccagtgac 2520

cactttattc cacttcctcc attacccagt tttggcccac agagtttggt cccccccaaa 2580

cctcggacca atatccctct aaacatcaat ctatcctcct gttaaagaaa aaaaaaaa     2638

<210>  24
<211>  2573
<212>  DNA
<213>  Homo sapiens

<220>
<223>  Exemplary nucleic acid sequence encoding human CLA

<300>
<308>  NM_003006.4
<309>  2015-03-15
<313>  (1)..(2573)

<400>  24
   acacacagcc attgggggtt gctcggatcc gggactgccg cagggggtgc cacagcagtg      60
   cctggcagcg tgggctggga ccttgtcact aaagcagaga agccacttct tctgggccca     120
   cgaggcagct gtcccatgct ctgctgagca cggtggtgcc atgcctctgc aactcctcct     180
   gttgctgatc ctactgggcc ctggcaacag cttgcagctg tgggacacct gggcagatga     240
   agccgagaaa gccttgggtc ccctgcttgc ccgggaccgg agacaggcca ccgaatatga     300
   gtacctagat tatgatttcc tgccagaaac ggagcctcca gaaatgctga ggaacagcac     360
   tgacaccact cctctgactg ggcctggaac ccctgagtct accactgtgg agcctgctgc     420
   aaggcgttct actggcctgg atgcaggagg ggcagtcaca gagctgacca cggagctggc     480
   caacatgggg aacctgtcca cggattcagc agctatggag atacagacca ctcaaccagc     540
   agccacggag gcacagacca ctcaaccagt gcccacggag gcacagacca ctccactggc     600
   agccacagag gcacagacaa ctcgactgac ggccacggag gcacagacca ctccactggc     660
   agccacagag gcacagacca ctccaccagc agccacggaa gcacagacca ctcaacccac     720
   aggcctggag gcacagacca ctgcaccagc agccatggag gcacagacca ctgcaccagc     780
   agccatggaa gcacagacca ctccaccagc agccatggag gcacagacca ctcaaaccac     840
   agccatggag gcacagacca ctgcaccaga agccacggag gcacagacca ctcaacccac     900
   agccacggag gcacagacca ctccactggc agccatggag gccctgtcca cagaacccag     960
   tgccacagag gccctgtcca tggaacctac taccaaaaga ggtctgttca tacccttttc    1020
   tgtgtcctct gttactcaca agggcattcc catggcagcc agcaatttgt ccgtcaacta    1080
   cccagtgggg gccccagacc acatctctgt gaagcagtgc ctgctggcca tcctaatctt    1140
   ggcgctggtg gccactatct tcttcgtgtg cactgtggtg ctggcggtcc gcctctcccg    1200
   caagggccac atgtaccccg tgcgtaatta ctcccccacc gagatggtct gcatctcatc    1260
   cctgttgcct gatgggggtg aggggccctc tgccacagcc aatgggggcc tgtccaaggc    1320
   caagagcccg ggcctgacgc cagagcccag ggaggaccgt gagggggatg acctcaccct    1380
   gcacagcttc ctcccttagc tcactctgcc atctgttttg gcaagacccc acctccacgg 1440
   gctctcctgg gccacccctg agtgcccaga ccccattcca cagctctggg cttcctcgga 1500
   gacccctggg gatggggatc ttcagggaag gaactctggc cacccaaaca ggacaagagc 1560
   agcctggggc caagcagacg ggcaagtgga gccacctctt tcctccctcc gcggatgaag 1620
   cccagccaca tttcagccga ggtccaaggc aggaggccat ttacttgaga cagattctct 1680
   cctttttcct gtcccccatc ttctctgggt ccctctaaca tctcccatgg ctctccccgc 1740
   ttctcctggt cactggagtc tcctccccat gtacccaagg aagatggagc tcccccatcc 1800
   cacacgcact gcactgccat tgtcttttgg ttgccatggt caccaaacag gaagtggaca 1860
   ttctaaggga ggagtactga agagtgacgg acttctgagg ctgtttcctg ctgctcctct 1920
   gacttggggc agcttgggtc ttcttgggca cctctctggg aaaacccagg gtgaggttca 1980
   gcctgtgagg gctgggatgg gtttcgtggg cccaagggca gacctttctt tgggactgtg 2040
   tggaccaagg agcttccatc tagtgacaag tgacccccag ctatcgcctc ttgccttccc 2100
   ctgtggccac tttccagggt ggactctgtc ttgttcactg cagtatccca actgcaggtc 2160
   cagtgcaggc aataaatatg tgatggacaa acgatagcgg aatccttcaa ggtttcaagg 2220
   ctgtctcctt caggcagcct tcccggaatt ctccatccct cagtgcagga tgggggctgg 2280
   tcctcagctg tctgccctca gcccctggcc ccccaggaag cctctttcat gggctgttag 2340
   gttgacttca gttttgcctc ttggacaaca gggggtcttg tacatccttg ggtgaccagg 2400
   aaaagttcag gctatggggg gccaaaggga gggctgcccc ttccccacca gtgaccactt 2460
   tattccactt cctccattac ccagttttgg cccacagagt ttggtccccc ccaaacctcg 2520
   gaccaatatc cctctaaaca tcaatctatc ctcctgttaa agaaaaaaaa aaa 2573

<210> 25
<211> 428
<212> PRT
<213> Homo sapiens

<220>
<223> Exemplary amino acid sequence for human CLA precursor

<300>
<308> NP_001193538.1
<309> 2015-03-15
<313> (1)..(428)

<400> 25

Met Ala Val Gly Ala Ser Gly Leu Glu Gly Asp Lys Met Ala Gly Ala
1               5                   10                  15

Met Pro Leu Gln Leu Leu Leu Leu Leu Ile Leu Leu Gly Pro Gly Asn
            20                  25                  30

Ser Leu Gln Leu Trp Asp Thr Trp Ala Asp Glu Ala Glu Lys Ala Leu
        35                  40                  45

Gly Pro Leu Leu Ala Arg Asp Arg Arg Gln Ala Thr Glu Tyr Glu Tyr
    50                  55                  60

Leu Asp Tyr Asp Phe Leu Pro Glu Thr Glu Pro Pro Glu Met Leu Arg
65                  70                  75                  80

Asn Ser Thr Asp Thr Thr Pro Leu Thr Gly Pro Gly Thr Pro Glu Ser
                85                  90                  95

Thr Thr Val Glu Pro Ala Ala Arg Arg Ser Thr Gly Leu Asp Ala Gly
            100                 105                 110

Gly Ala Val Thr Glu Leu Thr Thr Glu Leu Ala Asn Met Gly Asn Leu
        115                 120                 125

Ser Thr Asp Ser Ala Ala Met Glu Ile Gln Thr Thr Gln Pro Ala Ala
    130                 135                 140

Thr Glu Ala Gln Thr Thr Gln Pro Val Pro Thr Glu Ala Gln Thr Thr
145                 150                 155                 160

Pro Leu Ala Ala Thr Glu Ala Gln Thr Thr Arg Leu Thr Ala Thr Glu
                165                 170                 175

Ala Gln Thr Thr Pro Leu Ala Ala Thr Glu Ala Gln Thr Thr Pro Pro
            180                 185                 190

Ala Ala Thr Glu Ala Gln Thr Thr Gln Pro Thr Gly Leu Glu Ala Gln
        195                 200                 205

Thr Thr Ala Pro Ala Ala Met Glu Ala Gln Thr Thr Ala Pro Ala Ala
    210                 215                 220

Met Glu Ala Gln Thr Thr Pro Pro Ala Ala Met Glu Ala Gln Thr Thr
225                 230                 235                 240

Gln Thr Thr Ala Met Glu Ala Gln Thr Thr Ala Pro Glu Ala Thr Glu
                245                 250                 255

Ala Gln Thr Thr Gln Pro Thr Ala Thr Glu Ala Gln Thr Thr Pro Leu
            260                 265                 270

Ala Ala Met Glu Ala Leu Ser Thr Glu Pro Ser Ala Thr Glu Ala Leu

275                 280                 285

Ser Met Glu Pro Thr Thr Lys Arg Gly Leu Phe Ile Pro Phe Ser Val
    290                 295                 300

Ser Ser Val Thr His Lys Gly Ile Pro Met Ala Ala Ser Asn Leu Ser
305                 310                 315                 320

Val Asn Tyr Pro Val Gly Ala Pro Asp His Ile Ser Val Lys Gln Cys
                325                 330                 335

Leu Leu Ala Ile Leu Ile Leu Ala Leu Val Ala Thr Ile Phe Phe Val
            340                 345                 350

Cys Thr Val Val Leu Ala Val Arg Leu Ser Arg Lys Gly His Met Tyr
        355                 360                 365

Pro Val Arg Asn Tyr Ser Pro Thr Glu Met Val Cys Ile Ser Ser Leu
    370                 375                 380

Leu Pro Asp Gly Gly Glu Gly Pro Ser Ala Thr Ala Asn Gly Gly Leu
385                 390                 395                 400

Ser Lys Ala Lys Ser Pro Gly Leu Thr Pro Glu Pro Arg Glu Asp Arg
                405                 410                 415

Glu Gly Asp Asp Leu Thr Leu His Ser Phe Leu Pro
            420                 425

<210>  26
<211>  412
<212>  PRT
<213>  Homo sapiens

<220>
<223>  Exemplary amino acid sequence for human CLA precursor

<300>
<308>  NP_002997.2
<309>  2015-03-15
<313>  (1)..(412)

<400>  26

Met Pro Leu Gln Leu Leu Leu Leu Leu Ile Leu Leu Gly Pro Gly Asn
1               5                   10                  15

Ser Leu Gln Leu Trp Asp Thr Trp Ala Asp Glu Ala Glu Lys Ala Leu
            20                  25                  30

Gly Pro Leu Leu Ala Arg Asp Arg Arg Gln Ala Thr Glu Tyr Glu Tyr

        35                  40                  45

Leu Asp Tyr Asp Phe Leu Pro Glu Thr Glu Pro Pro Glu Met Leu Arg
    50                  55                  60

Asn Ser Thr Asp Thr Thr Pro Leu Thr Gly Pro Gly Thr Pro Glu Ser
65                  70                  75                  80

Thr Thr Val Glu Pro Ala Ala Arg Arg Ser Thr Gly Leu Asp Ala Gly
                85                  90                  95

Gly Ala Val Thr Glu Leu Thr Thr Glu Leu Ala Asn Met Gly Asn Leu
            100                 105                 110

Ser Thr Asp Ser Ala Ala Met Glu Ile Gln Thr Thr Gln Pro Ala Ala
        115                 120                 125

Thr Glu Ala Gln Thr Thr Gln Pro Val Pro Thr Glu Ala Gln Thr Thr
    130                 135                 140

Pro Leu Ala Ala Thr Glu Ala Gln Thr Thr Arg Leu Thr Ala Thr Glu
145                 150                 155                 160

Ala Gln Thr Thr Pro Leu Ala Ala Thr Glu Ala Gln Thr Thr Pro Pro
                165                 170                 175

Ala Ala Thr Glu Ala Gln Thr Thr Gln Pro Thr Gly Leu Glu Ala Gln
            180                 185                 190

Thr Thr Ala Pro Ala Ala Met Glu Ala Gln Thr Thr Ala Pro Ala Ala
        195                 200                 205

Met Glu Ala Gln Thr Thr Pro Pro Ala Ala Met Glu Ala Gln Thr Thr
    210                 215                 220

Gln Thr Thr Ala Met Glu Ala Gln Thr Thr Ala Pro Glu Ala Thr Glu
225                 230                 235                 240

Ala Gln Thr Thr Gln Pro Thr Ala Thr Glu Ala Gln Thr Thr Pro Leu
                245                 250                 255

Ala Ala Met Glu Ala Leu Ser Thr Glu Pro Ser Ala Thr Glu Ala Leu
            260                 265                 270

Ser Met Glu Pro Thr Thr Lys Arg Gly Leu Phe Ile Pro Phe Ser Val
        275                 280                 285

Ser Ser Val Thr His Lys Gly Ile Pro Met Ala Ala Ser Asn Leu Ser
    290                 295                 300

Val Asn Tyr Pro Val Gly Ala Pro Asp His Ile Ser Val Lys Gln Cys
305                 310                 315                 320

Leu Leu Ala Ile Leu Ile Leu Ala Leu Val Ala Thr Ile Phe Phe Val
                325                 330                 335

Cys Thr Val Val Leu Ala Val Arg Leu Ser Arg Lys Gly His Met Tyr
            340                 345                 350

Pro Val Arg Asn Tyr Ser Pro Thr Glu Met Val Cys Ile Ser Ser Leu
        355                 360                 365

Leu Pro Asp Gly Gly Glu Gly Pro Ser Ala Thr Ala Asn Gly Gly Leu
    370                 375                 380

Ser Lys Ala Lys Ser Pro Gly Leu Thr Pro Glu Pro Arg Glu Asp Arg
385                 390                 395                 400

Glu Gly Asp Asp Leu Thr Leu His Ser Phe Leu Pro
                405                 410